(19) Europäisches Patentamt
European Patent Office
Office européen des brevets

(11) EP 2 010 531 B1

(12) EUROPÄISCHE PATENTSCHRIFT

(45) Veröffentlichungstag und Bekanntmachung des Hinweises auf die Patenterteilung:
**17.08.2011 Patentblatt 2011/33**

(21) Anmeldenummer: **07724560.3**

(22) Anmeldetag: **25.04.2007**

(51) Int Cl.:
***C07D 471/10*** (2006.01) ***C07D 491/10*** (2006.01)
***C07D 493/10*** (2006.01) ***C07D 495/10*** (2006.01)
***A61K 31/407*** (2006.01) ***A61P 25/00*** (2006.01)
***A61P 25/04*** (2006.01)

(86) Internationale Anmeldenummer:
**PCT/EP2007/003630**

(87) Internationale Veröffentlichungsnummer:
**WO 2007/124903 (08.11.2007 Gazette 2007/45)**

(54) **SPIROCYCLISCHE CYCLOHEXANDERIVATE MIT ANALGESISCHER WIRKUNG**

SPIROCYCLIC CYCLOHEXANE DERIVATIVES WITH ANALGESIC PROPERTIES

DERIVES DE CYCLOHEXANE SPIROCYCLIQUES A ACTION ANALGESIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **27.04.2006 DE 102006019597**

(43) Veröffentlichungstag der Anmeldung:
**07.01.2009 Patentblatt 2009/02**

(73) Patentinhaber: **Grünenthal GmbH**
**52078 Aachen (DE)**

(72) Erfinder:
- **MERLA, Beatrix**
  **52078 Aachen (DE)**
- **OBERBÖRSCH, Stefan**
  **52074 Aachen (DE)**
- **SUNDERMANN, Bernd**
  **61381 Friedrichsdorf (DE)**
- **ENGLBERGER, Werner**
  **52223 Stolberg (DE)**
- **HENNIES, Hagen-Heinrich**
  **52152 Simmerath (DE)**
- **KÖGEL, Babette-Yvonne**
  **52379 Langerwehe-Hamich (DE)**
- **SCHUNK, Stephan**
  **52070 Aachen (DE)**
- **GRAUBAUM, Heinz**
  **12557 Berlin (DE)**

(74) Vertreter: **Bülle, Jan et al**
**Kutzenberger & Wolff**
**Patentanwaltssozietät**
**Theodor-Heuss-Ring 23**
**50668 Köln (DE)**

(56) Entgegenhaltungen:
**WO-A-2004/043967** **WO-A-2005/063769**
**WO-A-2005/066183** **WO-A-2006/018184**
**WO-A1-2004/043967** **WO-A1-2004/043967**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft spirocyclische Cyclohexan-Derivate, Verfahren zu deren Herstellung, Arzneimittel enthaltend diese Verbindungen und die Verwendung von substituierten spirocyclischen Cyclohexan-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

WO 04 043967 offenbart ebenfalls spirocyclische Cyclohexan-Derivate, die jedoch in 1-Position des Cyclohexanrings eine Aminofunktion anstelle einer Aminomethylgruppe tragen.

WO 2005/063769, WO 2005/066183 und WO 2006/018184 offenbaren ebenfalls spirocyclische Cyclohexan-Derivate unterschiedlicher Struktur.

Eine der Erfindung zugrundeliegende Aufgabe bestand darin, neue analgetisch wirksame Substanzen zur Verfügung zu stellen, die sich zur Schmerztherapie - insbesondere auch chronischer und neuropathischer Schmerzen - eignen. Gegenstand der Erfindung sind daher spirocyclische Cyclohexan-Derivate der allgemeinen Formel I,

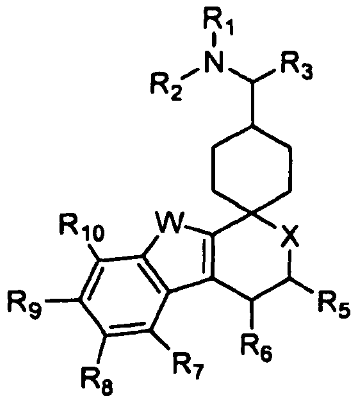

I

worin

$R^1$ und $R^2$ unabhängig voneinander H; $C_{1-5}$-Alkyl, jeweils gesättigt oder ungesättigt, verzweigt oder unverzweigt, bedeutet, wobei $R^1$ und $R^2$ nicht gleichzeitig H bedeuten,

oder die Reste $R^1$ und $R^2$ zusammen $CH_2CH_2OCH_2CH_2$- $CH_2CH_2NR^{11}CH_2CH_2$ oder $(CH_2)_{3-6}$ bedeuten,

wobei $R^{11}$ H; $C_{1-5}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, oder Heteroaryl, jeweils einfach oder mehrfach substituiert oder unsubstituiert; oder über $C_{1-3}$-Alkyl-Gruppe gebundenes Aryl oder $C_{3-8}$-Cycloalkyl oder Heteroaryl, jeweils unsubstituiert oder einfach oder mehrfach substituiert; bedeutet;

$R^3$ Phenyl oder

Thienyl, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl; einen über eine $C_{1-3}$-Alkylkette gebundenen Phenylrest, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-8}$-Alkyl, $CF_3$, $C_{1-6}$-Alkyl, bedeutet ;

W für $NR^4$, O oder S steht und

$R^4$ für H; $C_{1-5}$-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine $C_{1-3}$-Alkyl-Gruppe gebundenes Aryl, Heteroaryl oder Cycloalkyl, jeweils einfach oder mehrfach substituiert oder unsubstituiert; $COR^{12}$; $CSR^{12}$; $SO_2R^{12}$ steht, wobei $R^{12}$ H; $C_{1-8}$-Alkyl, jeweils gesättigt oder ungesättigt, verzweigt oder unverzweigt, einfach oder mehrfach substituiert

oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; $(CHR^a)_qOR^{13}$, q = 0, 1 oder 2 und $R^a$ = H, Methyl oder Ethyl; $NR^{14}R^{15}$ bedeutet;

$R^5$ für H; $C_{1-5}$-Alkyl, gesättigt oder ungesättigt, verzweigt oder unverzweigt, unsubstituiert oder einfach oder mehrfach substituiert; $COOR^{13}$, $CONR^{13}$, $OR^{13}$; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

$R^6$ für H; $OR^{13}$, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Phenyl; oder über $C_{1-3}$-Alkyl gebundenes Aryl, oder Heteroaryl, steht;

$R^7$, $R^8$, $R^9$ und $R^{10}$ unabhängig voneinander für H, F, Cl, Br, $NO_2$, $CF_3$, $OR^{13}$, CN, $COOR^{13}$, $NR^{14}R^{15}$; $C_{1-5}$-Alkyl, Phenyl oder Benzyl, stehen;

wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeutet;

$R^{14}$ und $R^{15}$ unabhängig voneinander H oder $C_{1-5}$-Alkyl bedeuten oder $R^{14}$ und $R^{15}$ zusammen $CH_2CH_2OCH_2CH_2$, $CH_2CH_2NR_{16}CH_2CH_2$ oder $(CH_2)_{3-6}$ bilden,

wobei $R^{16}$ H; $C_{1-5}$-Alkyl, gesättigt oder ungesättigt, bedeutet;

X für O, S, SO, $SO_2$ oder $NR^{17}$ steht;

wobei $R_{17}$ für H; $C_{1-5}$-Alkyl, gesättigt oder ungesättigt, verzweigt oder unverzweigt, jeweils einfach oder mehrfach substituiert oder unsubstituiert; $COR^{12}$ oder $SO_2R^{12}$ bedeutet,

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Wenn ein Substituent, wie z. B. $R^{13}$, innerhalb einer Verbindung in verschiedenen Positionen mehrfach erscheint, wie z. B. als Reste $R^7$ -$R^{10}$ in der Bedeutung $OR^{13}$, kann der Substituent, hier $R^{13}$, für zwei oder mehrere Reste innerhalb einer Substanz unterschiedliche Bedeutungen annehmen.

Die Verbindungen weisen eine Affinität zum μ-Opioid-Rezeptor auf.

Die Ausdrücke "$C_{1-3}$-Alkyl", "$C_{1-5}$-Alkyl"oder "$C_{1-8}$-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 3 C-Atomen bzw. 1 bis 5 C-Atomen bzw. 1 bis 5 C-Atomen, d.h. $C_{1-3}$-Alkanyle, $C_{2-3}$-Alkenyle und $C_{2-3}$-Alkinyle bzw. $C_{1-5}$-Alkanyle, $C_{2-5}$-Alkenyle und $C_{2-5}$-Alkinyle bzw. $C_{1-8}$-Alkanyle, $C_{2-8}$-Alkenyle und $C_{2-8}$-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, Ethylenyl (Vinyl), Ethinyl, Propenyl ($-CH_2CH=CH_2$, $-CH=CH-CH_3$, $-C(=CH_2)-CH_3$), Propinyl ($-CH-C\equiv CH$, $-C\equiv C-CH_3$), Butenyl, Butinyl, Pentenyl, Pentinyl, Hexyl, Hexenyl, Hexinyl, Heptyl, Octyl und 1-Ethyl-pentyl umfasst. Besonders vorteihaft sind Methyl, Ethyl und Propyl.

Der Ausdruck "Aryl" bedeutet im Sinne dieser Erfindung aromatische Kohlenwasserstoffe, u.a. Phenyle und Naphthyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält. Besonders vorteilhaft ist der Phenyl-Rest.

Der Ausdruch "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxadiazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann. Besonders bevorzugt ist der Thienylrest.

Der Ausdruck "über $C_{1-3}$-Alkyl gebundenes Aryl oder Heteroaryl" bedeuten für die Zwecke der vorliegenden Erfindung, daß $C_{1-3}$-Alkyl und Aryl bzw. Heteroaryl die oben definierten Bedeutungen haben und der Aryl- bzw. Heteroaryl-

Rest über eine $C_{1-3}$-Alkyl-Gruppe an die Verbindung der allgemeinen Struktur I gebunden ist. Besonders vorteilhaft im Sinne dieser Erfindung ist Benzyl und Phenethyl.

Im Zusammenhang mit "Alkyl" versteht man unter dem Begriff "substituiert" im Sinne dieser Erfindung die Substitution eines Wasserstoffrestes durch F, Cl, Br, I, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O) $C_{1-6}$-Alkyl, $CO_2H$, $CO_2$-$C_{1-6}$-Alkyl oder Benzyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von $CF_3$ oder -$CH_2CF_3$ oder an verschiedenen Stellen wie im Falle von -CH(OH)-CH=CH-$CHCl_2$. Die Mehrfachsubstitution kann mit dem gleichen oder mit verschiedenen Substituenten erfolgen.

In Bezug auf "Aryl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, $NH_2$, NH-$C_{1-6}$-Alkyl, NH- $C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, $CO_2H$, $CO_2$-$C_{1-6}$-Alkyl, $CF_3$, $C_{1-6}$-Alkyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" und "Heteroaryl" sind dabei bevorzugte Substituenten -F und -Cl.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind:

Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Hippursäure, Phosphorsäure und/oder Asparaginsäure. Besonders bevorzugt sind die Zitronensäure und die Salzsäure.

Unter dem Begriff $(CH_2)_{3-6}$ bzw. $(CH_2)_{4-5}$ ist -$CH_2$-$CH_2$-$CH_2$-, -$CH_2$-$CH_2$-$CH_2$-$CH_2$-, - $CH_2$-$CH_2$-$CH_2$-$CH_2$-$CH_2$- und $CH_2$-$CH_2$-$CH_2$-$CH_2$-$CH_2$-$CH_2$- bzw -$CH_2$-$CH_2$-$CH_2$-$CH_2$-, -$CH_2$-$CH_2$-$CH_2$-$CH_2$-$CH_2$ zu verstehen.

Bevorzugt sind substituierte Cyclohexanderivate der allgemeinen Formel I, worin $R^1$ und $R^2$ unabhängig voneinander H oder $C_{1-6}$-Alkyl bedeuten, wobei $R^1$ und $R^2$ nicht gleichzeitig H bedeuten, oder $R^1$ und $R^2$ zusammen $CH_2CH_2OCH_2CH_2$, oder $(CH_2)_{3-5}$, insbesondere $(CH_2)_3$, bedeuten.

Besonders bevorzugt sind substituierte Cyclohexanderivate, worin $R^1$ und $R^2$ $CH_3$ bedeuten.

Bei den erfindungsgemäßen Cyclohexanderivaten bedeutet

$R^3$ Phenyl oder Thienyl, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl; einen über eine $C_{1-3}$-Alkylkette gebundenen Phenylrest, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl,

insbesondere

$R^3$ Phenyl, unsubstituiert oder einfach oder mehrfach substituiert mit F, Cl, OH, $OCH_3$, $CF_3$ oder $CH_3$; Thienyl; oder einen über eine $C_{1-3}$-Alkylkette gebundenen Phenylrest, unsubstituiert oder einfach oder mehrfach substituiert mit F, Cl, CN, OH, $OCH_3$, $CF_3$ oder $CH_3$

Besonders bevorzugt sind Cyclohexan-Derivate, worin $R^3$ Phenyl, unsubstituiert oder einfach substituiert mit Cl oder F, Phenethyl oder Thienyl bedeutet.

Weiterhin bevorzugt sind substituierte Cyclohexanderivate, worin der Rest $R^5$ für H, $CH_3$, COOH, $COOCH_3$ oder $CH_2OH$ steht

insbesondere

$R^5$ H oder $CH_3$ bedeutet.

Außerdem bevorzugt sind substituierte Cyclohexanderivate, worin der Rest $R^6$ für H steht.

Bevorzugt sind außerdem substituierte Cyclohexanderivate der allgemeinen Formel I, worin $R^7$ $R^8$, $R^9$ und $R^{10}$ unabhängig voneinander H; $C_{1-5}$-Alkyl, verzweigt oder unverzweigt, unsubstituiert oder ein- oder mehrfach substituiert; F, Cl, Br, I, $CF_3$, OH, $OCH_3$, $NH_2$, COOH, $COOCH_3$, $NHCH_3$, $N(CH_3)_2$, $NO_2$ oder Phenyl bedeuten vorzugsweise die Reste $R^8$ und $R^9$ unabhängig voneinander für H; $C_{1-5}$-Alkyl, F, Cl, Br, I, OH, $OCH_3$, COOH, $COOCH_3$, $NH_2$, $NHCH_3$ , $N(CH_3)_2$ oder $NO_2$ stehen, während die Reste $R^{10}$ und $R^7$ H sind.

Besonders bevorzugt sind substituierte Cyclohexanderivate der allgemeinen Formel I, worin $R^8$ für H, F oder $CH_3$ steht, während die Reste $R^7$, $R^9$ und $R^{10}$ H bedeuten.

Weiterhin besonders bevorzugt sind substituierte Cyclohexanderivate, bei denen X O oder $NR^{17}$ bedeutet, wobei $R^{17}$ für H, $CH_3$, $C_2H_5$, Acetyl, Phenyl, Benzyl oder $COR^{12}$ steht

insbesondere

$R^{17}$ für H oder $COR^{12}$ steht.

Es ist bevorzugt, dass W für NH steht.

Weiterhin ist bevorzugt, dass $R^{12}$ für Phenyl, Phenethyl, Phenethenyl, 1-Methyl-phenethenyl, oder Benzyl, unsubstituiert oder einfach oder mehrfach substituiert mit F, Cl, Br, I, CN, $NH_2$, NH-$C_{1-6}$-Alkyl, NH- $C_{1-6}$-Alkyl-OH, N ($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, $CO_2H$, $CO_2$-$C_{1-6}$-Alkyl, $CF_3$, $C_{1-6}$-Alkyl; Thienyl oder Benzothienyl, gegebenenfalls verbrückt über eine $CH_2$- oder eine $CH_2CH_2$-Kette, unsubstituiert oder einfach oder mehrfach substituiert mit F, Cl, Br, I, CN, $NH_2$, NH-$C_{1-6}$-Alkyl, NH-$C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O) $C_{1-6}$-Alkyl, $CO_2H$, $CO_2$-$C_{1-6}$-Alkyl, $CF_3$, $C_{1-6}$-Alkyl; oder Cyclopropyl, Cyclobutyl, Cyclopentyl oder Cyclohexyl, gegebenenfalls verbrückt über eine $CH_2$-oder eine $CH_2CH_2$-Kette, jeweils unsubstituiert oder einfach oder mehrfach substituiert mit F, Cl, Br, -CN, $NH_2$, NH-$C_{1-6}$-Alkyl, NH-$C_{1-6}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alkyl, S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl oder Benzyl; $(CHR^a)qOR^{13}$, q = 1 oder 2 und $R^a$ = H oder Methyl; steht;

insbesondere

$R^{12}$ für Phenyl, Cyclohexyl, Methyl-*tert*-butyl, 3,4-Dimethoxybenzyl, 1-Methyl-phenethenyl, 4-Chlorphenoxy-methyl, 2-Chlor-phenethenyl, 2-Benzothienyl, Methyl, Phenethenyl, Methoxymethyl, Thienylmethyl, Benzyloxymethyl, Ethylcyclopentyl, Cyclopropyl, 2-Ethyl-pentyl, 1-Methyl-ethyl, Ethyl, 4-Fluorbenzyl, 3-Chlor-2-Benzothienyl, Cyclopentyl, 3-Methoxybenzyl, 2-Phenoxyethyl, 5-Methyl-2-benzothienyl, 2-Methyl-propyl, Phenethyl oder 1-Ethyl-propyl steht.

Besonders bevorzugt sind spirocyclische Cyclohexan-Derivate aus der Gruppe

(17) 1,1-[3-(Dimethylamino-(4-fluorophenyl)methyl)-pentamethylen]-3,4-dihydro-1H-2,9-diazafluoren
(18) 1,1-[3-(Dimethylamino-(3-fluorophenyl)methyl)-pentamethylen]-3,4-dihydro-1H-2,9-diazafluoren
(19) 1,1-[3-(Dimethylamino-phenylmethyl)-pentamethylen]-3,4-dihydro-1H-2,9-diazafluoren
(20) 1,1-[3-(Dimethylamino-(phenylethyl)-methyl)-pentamethylen]-3,4-dihydro-1H-2,9-diazafluoren Citrat
(21) 1,1-[3-(Dimethylamino-(thiophen-2-yl)-methyl)-pentamethylen]-3,4-dihydro-1H-2,9-diazafluoren
(22) 1,1-[3-(4-Chlorphenyl-(dimethylamino)-methyl)-pentamethylen]-3,4-dihydro-1H-2,9-diazafluoren
(23) Cyclohexyl{1,1-[3-(Dimethylamino-(4-fluorophenyl)-methyl)-pentamethylen]-3,4-dihydro-1H-2,9-diazafluoren-2-yl}methanon
(24) Phenyl{1,1-[3-(Dimethylamino-(4-fluorophenyl)-methyl)-pentamethylen]-3,4-dihydro-1H-2,9-diazafluoren-2-yl} methanon
(25) 1,1-[3-(Dimethylamino-(4-fluorphenyl)-methyl)-pentamethylen]-1,3,4,9-tetrahydro-pyrano[3,4-b]indol
(26) 1,1-[3-(Dimethylamino-(3-fluorphenyl)-methyl)-pentamethylen]-1,3,4,9-tetrahydro-pyrano[3,4-b]indol
(27) 1,1-[3-(Dimethylamino-(phenyl)-methyl)-pentamethylen]-1,3,4,9-tetrahydropyrano[3,4-b]indol Citrat
(28) 1,1-[3-(Dimethylamino-(phenylethyl)-methyl)-pentamethylen]-1,3,4,9-tetrahydropyrano[3,4-b]indol
(29) 1,1-[3-(Dimethylamino-(thiophen-2-yl)-methyl)-pentamethylen]-1,3,4,9-tetrahydro-pyrano[3,4-b]indol
(30) 1,1-[3-(Dimethylamino-(4-chlorphenyl)-methyl)-pentamethylen]-1,3,4,9-tetrahydro-pyrano[3,4-b]indol
(35) 6-Fluor-1,1-[3-(dimethylamino-(4-fluorphenyl)-methyl)-pentamethylen]-1,3,4,9-tetrahydropyrano[3,4-b]indol
(36) 6-Fluor-1,1-[3-(dimethylamino-(3-fluorphenyl)-methyl)-pentamethylen]-1,3,4,9-tetrahydropyrano[3,4-b]indol
(37) 6-Fluor-1,1-[3-(dimethylamino-(phenyl)-methyl)-pentamethylen]-1,3,4,9-tetrahydro-pyrano[3,4-b]indol
(38) 6-Fluor-1,1-[3-(dimethylamino-(4-chlorphenyl)-methyl)-pentamethylen]-1,3,4,9-tetrahydropyrano[3,4-b]indol
(40) 3,6-Dimethyl-1,1-[3-(dimethylamino-(4-fluorphenyl)-methyl)-pentamethylen]-1,3,4,9-tetrahydropyrano[3,4-b] indol
(41) 3,6-Dimethyl-1,1-[3-(dimethylamino-(3-fluorphenyl)-methyl)-pentamethylen]-1,3,4,9-tetrahydropyrano[3,4-b] indol
(42) 3,6-Dimethyl-1,1-[3-(dimethylamino-(phenyl)-methyl)-pentamethylen]-1,3,4,9-tetrahydropyrano[3,4-b]indol Citrat
(43) 3,6-Dimethyl-1,1-[3-(dimethylamino-(phenylethyl)-methyl)-pentamethylen]-1,3,4,9-tetrahydropyrano[3,4-b]indol
(44) 3,6-Dimethyl-1,1-[3-(dimethylamino-(4-chlorphenyl)-methyl)-pentamethylen]-1,3,4,9-tetrahydropyrano[3,4-b] indol
(51) 6-Fluor-3-methyl-1,1-[3-(dimethylamino-(4-fluorphenyl)-methyl)-pentamethylen]-1,3,4,9-tetrahydropyrano[3,4-b]indol
(52) 6-Fluor-3-methyl-1,1-[3-(dimethylamino-(3-fluorphenyl)-methyl)-pentamethylen]-1,3,4,9-tetrahydropyrano[3,4-b]indol
(53) 6-Fluor-3-methyl-1,1-[3-(dimethylamino-(phenyl)-methyl)-pentamethylen]-1,3,4,9-tetrahydropyrano[3,4-b]indol
(54) 6-Fluor-3-methyl-1,1-[3-(dimethylamino-(phenylethyl)-methyl)-pentamethylen]-1,3,4,9-tetrahydropyrano[3,4-b]indol

(55) 6-Fluor-3-methyl-1,1-[3-(dimethylamino-(4-chlorphenyl)-methyl)-pentamethylen]-1,3,4,9-tetrahydropyrano [3,4-b]indol

(56) 1-{1,1-[3-(Dimethylamino-phenylmethyl)-pentamethylen]-3,4-dihydro-1H-2,9-diazafluoren-2-yl}-3,3-dimethylbutan-1-on

(57) 2-[(3,4-Dimethoxyphenyl)acetyl]-{1,1-[3-(dimethylamino-phenylmethyl)-pentamethylen]-3,4-dihydro-1H-2,9-diazafluoren}

(58) 1-{1,1-[3-(Dimethylamino-phenylmethyl)-pentamethylen]-3,4-dihydro-1H-2,9-diazafluoren-2-yl}-2-methyl-3-phenylprop-2-en-1-on

(59) 2-(Cyclohexylcarbonyl)-{1,1-[3-(dimethylamino-phenylmethyl)-pentamethylen]-3,4-dihydro-1H-2,9-diazafluoren}

(60) 2-[(4-Chlorophenoxy)acetyl]-{1,1-[3-(dimethylamino-phenylmethyl)-pentamethylen]-3,4-dihydro-1H-2,9-diazafluoren}

(61) 1-{1,1-[3-(Dimethylamino-phenylmethyl)-pentamethylen]-3,4-dihydro-1H-2,9-diazafluoren-2-yl}-3-(2-chlorphenyl)prop-2-en-1-on

(62) 1-{1,1-[3-(Dimethylamino-(4-fluorphenyl)methyl)-pentamethylen]-3,4-dihydro-1H-2,9-diazafluoren-2-yl}-3,3-dimethylbutan-1-on

(63) 2-[(3,4-Dimethoxyphenyl)acetyl]-{1,1-[3-(dimethylamino-(4-fluorophenyl)methyl)-pentamethylen]-3,4-dihydro-1H-2,9-diazafluoren}

(64) 1-{1,1-[3-(Dimethylamino-(4-fluorophenyl)methyl)-pentamethylen]-3,4-dihydro-1H-2,9-diazafluoren-2-yl}-2-methyl-3-phenylprop-2-en-1-on

(65) 2-[(4-Chlorophenoxy)acetyl]-{1,1-[3-(dimethylamino-(4-fluorophenyl)methyl)-pentamethylen]-3,4-dihydro-1H-2,9-diazafluoren}

(66) 1-{1,1-[3-(Dimethylamino-(3-fluorphenyl)methyl)-pentamethylen]-3,4-dihydro-1H-2,9-diazafluoren-2-yl}-3,3-dimethylbutan-1-on

(67) 2-[(3,4-Dimethoxyphenyl)acetyl]-{1,1-[3-(dimethylamino-(3-fluorophenyl)methyl)-pentamethylen]-3,4-dihydro-1H-2,9-diazafluoren}

(68) 1-{1,1-[3-(Dimethylamino-(3-fluorophenyl)methyl)-pentamethylen]-3,4-dihydro-1H-2,9-diazafluoren-2-yl}-2-methyl-3-phenylprop-2-en-1-on

(69) 2-(Cyclohexylcarbonyl)-{1,1-[3-(dimethylamino-(3-fluorophenyl)methyl)-pentamethylen]-3,4-dihydro-1H-2,9-diazafluoren}

(70) 2-[(4-Chlorophenoxy)acetyl]-{1,1-[3-(dimethylamino-(3-fluorophenyl)methyl)-pentamethylen]-3,4-dihydro-1H-2,9-diazafluoren}

(71) 2-(1-Benzothien-2-ylcarbonyl)-{1,1-[3-(dimethylamino-(3-fluorophenyl)methyl)-pentamethylen]-3,4-dihydro-1H-2,9-diazafluoren}

(72) 1-{1,1-[3-(Dimethylamino-(3-fluorphenyl)methyl)-pentamethylen]-3,4-dihydro-1H-2,9-diazafluoren-2-yl}-3-(2-chlorphenyl)prop-2-en-1-on

(73) 1-{1,1-[3-(Dimethylamino-(4-chlorphenyl)methyl)-pentamethylen]-3,4-dihydro-1H-2,9-diazafluoren-2-yl}-3,3-dimethylbutan-1-on

(74) 2-[(3,4-Dimethoxyphenyl)acetyl]-{1,1-[3-(dimethylamino-(4-chlorophenyl)methyl)-pentamethylen]-3,4-dihydro-1H-2,9-diazafluoren}

(75) 1-{1,1-[3-(Dimethylamino-(4-chlorphenyl)methyl)-pentamethylen]-3,4-dihydro-1H-2,9-diazafluoren-2-yl}-2-methyl-3-phenylprop-2-en-1-on

(76) 2-(Cyclohexylcarbonyl)-{1,1-[3-(dimethylamino-(4-chlorophenyl)methyl)-pentamethylen]-3,4-dihydro-1H-2,9-diazafluoren}

(77) 2-[(4-Chlorophenoxy)acetyl]-{1,1-[3-(dimethylamino-(4-chlorophenyl)methyl)-pentamethylen]-3,4-dihydro-1H-2,9-diazafluoren}

(78) 2-(1-Benzothien-2-ylcarbonyl)-{1,1-[3-(dimethylamino-(4-chlorophenyl)methyl)-pentamethylen]-3,4-dihydro-1H-2,9-diazafluoren}

(79) 2-Acetyl-{1,1-[3-(dimethylamino-phenylmethyl)-pentamethylen]-3,4-dihydro-1H-2,9-diazafluoren}

(80) 1-{1,1-[3-(Dimethylamino-phenylmethyl)-pentamethylen]-3,4-dihydro-1H-2,9-diazafluoren-2-yl}-3-phenylprop-2-en-1-on

(81) 2-(Methoxyacetyl)-{1,1-[3-(dimethylamino-phenylmethyl)-pentamethylen]-3,4-dihydro-1H-2,9-diazafluoren}

(82) 2-(2-Thienylacetyl)-{1,1-[3-(dimethylamino-phenylmethyl)-pentamethylen]-3,4-dihydro-1H-2,9-diazafluoren}

(83) 2-(Benzyloxyacetyl)-{1,1-[3-(dimethylamino-phenylmethyl)-pentamethylen]-3,4-dihydro-1H-2,9-diazafluoren}

(84) Acetyl-{1,1-[3-(dimethylamino-(4-fluorophenyl)methyl)-pentamethylen]-3,4-dihydro-1H-2,9-diazafluoren}

(85) 2-(3-Cyclopentylpropanoyl)-{1,1-[3-(dimethylamino-(4-fluorophenyl)methyl)-pentamethylen]-3,4-dihydro-1H-2,9-diazafluoren}

(86) 1-{1,1-[3-(Dimethylamino-(4-fluorphenyl)methyl)-pentamethylen]-3,4-dihydro-1H-2,9-diazafluoren-2-yl}-3-phenylprop-2-en-1-on
(87) 2-(2-Thienylacetyl)-{1,1-[3-(dimethylamino-(4-fluorophenyl)methyl)-pentamethylen]-3,4-dihydro-1H-2,9-diazafluoren}
(88) 2-(Benzyloxyacetyl)-{1,1-[3-(dimethylamino-(4-fluorophenyl)methyl)-pentamethylen]-3,4-dihydro-1H-2,9-diazafluoren}
(89) Acetyl-{1,1-[3-(dimethylamino-(3-fluorophenyl)methyl)-pentamethylen]-3,4-dihydro-1H-2,9-diazafluoren}
(90) 2-(3-Cyclopentylpropanoyl)-{1,1-[3-(dimethylamino-(3-fluorophenyl)methyl)-pentamethylen]-3,4-dihydro-1H-2,9-diazafluoren}
(91) 2-(Cyclopropylcarbonyl)-{1,1-[3-(dimethylamino-(3-fluorophenyl)methyl)-pentamethylen]-3,4-dihydro-1H-2,9-diazafluoren}
(92) 2-(Methoxyacetyl)-{1,1-[3-(dimethylamino-(3-fluorophenyl)methyl)-pentamethylen]-3,4-dihydro-1H-2,9-diazafluoren}
(93) 2-(2-Thienylacetyl)-{1,1-[3-(dimethylamino-(3-fluorophenyl)methyl)-pentamethylen]-3,4-dihydro-1H-2,9-diazafluoren}
(94) 2-(Benzyloxyacetyl)-{1,1-[3-(dimethylamino-(3-fluorophenyl)methyl)-pentamethylen]-3,4-dihydro-1H-2,9-diazafluoren}
(95) 2-(3-Cyclopentylpropanoyl)-{1,1-[3-(dimethylamino-(4-chlorophenyl)methyl)-pentamethylen]-3,4-dihydro-1H-2,9-diazafluoren}
(96) 2-(Cyclopropylcarbonyl)-{1,1-[3-(dimethylamino-(4-chlorophenyl)methyl)-pentamethylen]-3,4-dihydro-1H-2,9-diazafluoren}
(97) 2-(Methoxyacetyl)-{1,1-[3-(dimethylamino-(4-chlorophenyl)methyl)-pentamethylen]-3,4-dihydro-1H-2,9-diazafluoren}
(98) 2-(2-Thienylacetyl)-{1,1-[3-(dimethylamino-(4-chlorophenyl)methyl)-pentamethylen]-3,4-dihydro-1H-2,9-diazafluoren}
(99) 2-(Benzyloxyacetyl)-{1,1-[3-(dimethylamino-(4-chlorophenyl)methyl)-pentamethylen]-3,4-dihydro-1H-2,9-diazafluoren}
(100) 2-(2-Ethylhexanoyl)-{1,1-[3-(dimethylamino-phenylmethyl)-pentamethylen]-3,4-dihydro-1H-2,9-diazafluoren}
(101) 2-Isobutyryl-{1,1-[3-(dimethylamino-phenylmethyl)-pentamethylen]-3,4-dihydro-1H-2,9-diazafluoren}
(102) 2-Propionyl-{1,1-[3-(dimethylamino-phenylmethyl)-pentamethylen]-3,4-dihydro-1H-2,9-diazafluoren}
(103) 2-(1-Benzothien-3-ylcarbonyl)-{1,1-[3-(dimethylamino-phenylmethyl)-pentamethylen]-3,4-dihydro-1H-2,9-diazafluoren}
(104) 2-[(4-Fluorophenyl)acetyl]-{1,1-[3-(dimethylamino-phenylmethyl)-pentamethylen]-3,4-dihydro-1H-2,9-diazafluoren}
(105) 2-(3-Chloro-1-benzothien-2-ylcarbonyl)-{1,1-[3-(dimethylamino-phenylmethyl)-pentamethylen]-3,4-dihydro-1H-2,9-diazafluoren}
(106) 2-(2-Ethylhexanoyl)-{1,1-[3-(dimethylamino-(4-fluorophenyl)methyl)-pentamethylen]-3,4-dihydro-1H-2,9-diazafluoren}
(107) 2-Isobutyryl-{1,1-[3-(dimethylamino-(4-fluorophenyl)methyl)-pentamethylen]-3,4-dihydro-1H-2,9-diazafluoren}
(108) 2-Propionyl-{1,1-[3-(dimethylamino-(4-fluorophenyl)methyl)-pentamethylen]-3,4-dihydro-1H-2,9-diazafluoren}
(109) 2-Cyclopentylcarbonyl-{1,1-[3-(dimethylamino-(4-fluorophenyl)methyl)-pentamethylen]-3,4-dihydro-1H-2,9-diazafluoren}
(110) 2-[(4-Fluorophenyl)acetyl]-{1,1-[3-(dimethylamino-(4-fluorophenyl)methyl)-pentamethylen]-3,4-dihydro-1H-2,9-diazafluoren}
(111) 2-(2-Ethylhexanoyl)-{1,1-[3-(dimethylamino-(3-fluorophenyl)methyl)-pentamethylen]-3,4-dihydro-1H-2,9-diazafluoren}
(112) 2-Isobutyryl-{1,1-[3-(dimethylamino-(3-fluorophenyl)methyl)-pentamethylen]-3,4-dihydro-1H-2,9-diazafluoren}
(113) 2-Propionyl-{1,1-[3-(dimethylamino-(3-fluorophenyl)methyl)-pentamethylen]-3,4-dihydro-1H-2,9-diazafluoren}
(114) 2-(1-Benzothien-3-ylcarbonyl)-{1,1-[3-(dimethylamino-(3-fluorophenyl)methyl)-pentamethylen]-3,4-dihydro-1H-2,9-diazafluoren}
(115) 2-Cyclopentylcarbonyl-{1,1-[3-(dimethylamino-(3-fluorophenyl)methyl)-pentamethylen]-3,4-dihydro-1H-2,9-diazafluoren}
(116) 2-[(4-Fluorophenyl)acetyl]-{1,1-[3-(dimethylamino-(3-fluorophenyl)methyl)-pentamethylen]-3,4-dihydro-1H-2,9-diazafluoren}
(117) 2-(3-Chloro-1-benzothien-2-ylcarbonyl)-{1,1-[3-(dimethylamino-(3-fluorophenyl)methyl)-pentamethylen]-3,4-

dihydro-1H-2,9-diazafluoren}

(118) 2-(2-Ethylhexanoyl)-{1,1-[3-(dimethylamino-(4-chlorophenyl)methyl)-pentamethylen]-3,4-dihydro-1H-2,9-diazafluoren}

(119) 2-Isobutyryl-{1,1-[3-(dimethylamino-(4-chlorophenyl)methyl)-pentamethylen]-3,4-dihydro-1H-2,9-diazafluoren}

(120) 2-Propionyl-{1,1-[3-(dimethylamino-(4-chlorophenyl)methyl)-pentamethylen]-3,4-dihydro-1H-2,9-diazafluoren}

(121) 2-(1-Benzothien-3-ylcarbonyl)-{1,1-[3-(dimethylamino-(4-chlorophenyl)methyl)-pentamethylen]-3,4-dihydro-1H-2,9-diazafluoren}

(122) 2-Cyclopentylcarbonyl-{1,1-[3-(dimethylamino-(4-chlorophenyl)methyl)-pentamethylen]-3,4-dihydro-1H-2,9-diazafluoren}

(123) 2-[(4-Fluorophenyl)acetyl]-{1,1-[3-(dimethylamino-(4-chlorophenyl)methyl)-pentamethylen]-3,4-dihydro-1H-2,9-diazafluoren}

(124) 2-(3-Chloro-1-benzothien-2-ylcarbonyl)--{1,1-[3-(dimethylamino-(4-chlorophenyl)methyl)-pentamethylen]-3,4-dihydro-1H-2,9-diazafluoren}

(125) 2-Cyclopentylcarbonyl-{1,1-[3-(dimethylamino-phenylmethyl)-pentamethylen]-3,4-dihydro-1H-2,9-diazafluoren}

(126) 2-[(3-Methoxyphenyl)acetyl]-{1,1-[3-(dimethylamino-(3-fluorophenyl)methyl)-pentamethylen]-3,4-dihydro-1H-2,9-diazafluoren}

(127) 2-(2-Phenoxypropanoyl)-{1,1-[3-(dimethylamino-(3-fluorophenyl)methyl)-pentamethylen]-3,4-dihydro-1H-2,9-diazafluoren}

(128) 2-[5-Methylbenzothien-2-ylcarbonyl]-{1,1-[3-(dimethylamino-(3-fluorophenyl)methyl)-pentamethylen]-3,4-dihydro-1H-2,9-diazafluoren}

(129) 1-{1,1-[3-(Dimethylamino-(3-fluorphenyl)methyl)-pentamethylen]-3,4-dihydro-1H-2,9-diazafluoren-2-yl}-3-methylbutan-1-on

(130) 1-{1,1-[3-(Dimethylamino-(3-fluorphenyl)methyl)-pentamethylen]-3,4-dihydro-1H-2,9-diazafluoren-2-yl}-3-phenylpropan-1-on

(131) 2-(3-phenylpropanoyl)-{1,1-[3-(dimethylamino-(4-chlorophenyl)methyl)-pentamethylen]-3,4-dihydro-1H-2,9-diazafluoren}

(132) 2-[(3-Bromphenyl)acetyl]-{1,1-[3-(dimethylamino-(4-chlorophenyl)methyl)-pentamethylen]-3,4-dihydro-1H-2,9-diazafluoren}

(133) 2-(2-Phenoxypropanoyl)-{1,1-[3-(dimethylamino-(4-chlorophenyl)methyl)-pentamethylen]-3,4-dihydro-1H-2,9-diazafluoren}

(134) 1-{1,1-[3-(Dimethylamino-(4-chlorophenyl)methyl)-pentamethylen]-3,4-dihydro-1H-2,9-diazafluoren-2-yl}-3-methylbutan-1-on

(135) (3-Phenylpropanoyl)-1-{1,1-[3-(dimethylamino-(4-chlorophenyl)methyl)-pentamethylen]-3,4-dihydro-1H-2,9-diazafluoren}

(136) 2-[(3-Methoxyphenyl)acetyl]-{1,1-[3-(dimethylamino-phenylmethyl)-pentamethylen]-3,4-dihydro-1H-2,9-diazafluoren}

(137) 2-(2-Phenoxypropanoyl)-{1,1-[3-(dimethylamino-phenylmethyl)-pentamethylen]-3,4-dihydro-1H-2,9-diazafluoren}

(138) 1-{1,1-[3-(Dimethylamino-phenylmethyl)-pentamethylen]-3,4-dihydro-1H-2,9-diazafluoren-2-yl}-3-methylbutan-1-on

(139) 1-{1,1-[3-(Dimethylamino-(4-fluorophenyl)methyl)-pentamethylen]-3,4-dihydro-1H-2,9-diazafluoren-2-yl}-2-ethylbutan-1-on

(140) 2-[(3-Methoxyphenyl)acetyl]-{1,1-[3-(dimethylamino-(4-fluorophenyl)methyl)-pentamethylen]-3,4-dihydro-1H-2,9-diazafluoren}

(141) 2-(2-Phenoxypropanoyl)-{1,1-[3-(dimethylamino-(4-fluorophenyl)methyl)-pentamethylen]-3,4-dihydro-1H-2,9-diazafluoren}

(142) 1-{1,1-[3-(Dimethylamino-(4-fluorphenyl)methyl)-pentamethylen]-3,4-dihydro-1H-2,9-diazafluoren-2-yl}-3-phenylpropan-1-on

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Ein weiterer Gegenstand der Erfindung ist ein Verfahren zur Herstellung eines erfindungsgemäßen spirocyclischen Cyclohexan-Derivates. Die erfindungsgemäßen Substanzen können nach folgendem Syntheseschema hergestellt werden:

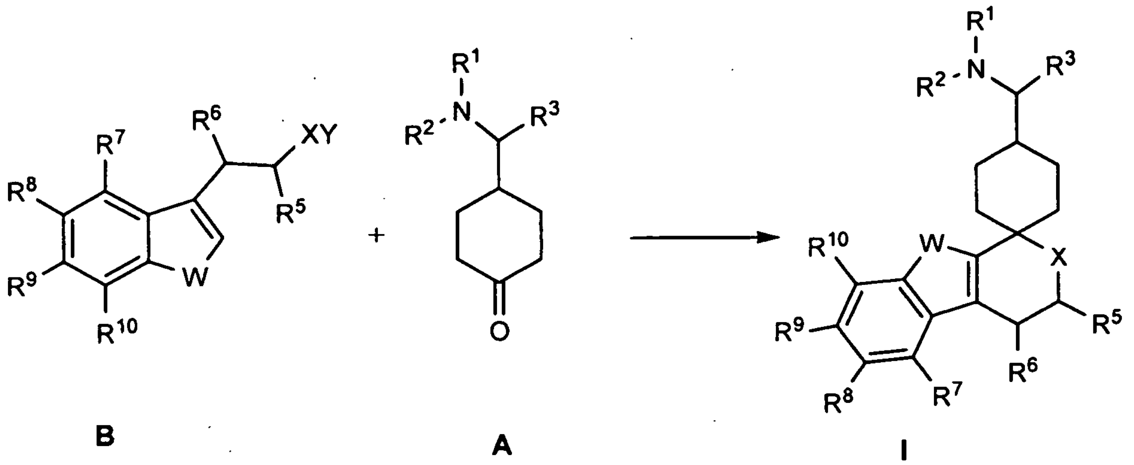

B A I

wobei Y H oder Trimethylsilyl bedeutet.

Zur Herstellung der Verbindungen der allgemeinen Formel I werden Ketone der allgemeinen Formel **A** mit Heteroaromaten der allgemeinen Formel **B** unter Zugabe von Säure oder deren Trimethylsilylester, beispielsweise Trifluormethansulfonsäuretrimethylsilylester, Essigsäure, Phosphorsäure, Methansulfonsäure oder Trifluoressigsäure in einem geeigneten Lösungsmittel, beispielsweise Dichlorethan, Dichlormethan, Chloroform, Acetonitril, Diethylether oder Nitromethan, umgesetzt.

Für die Herstellung der Ketone der allgemeinen Formel **A** schützt man die Ketofunktion von 4-Oxo-cyclohexancarbonsäureester,

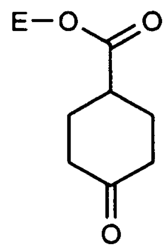

wobei E für einen $C_{1-6}$-Alkylrest, vorzugsweise Ethyl, steht, nach dem Fachmann bekannten Methoden,

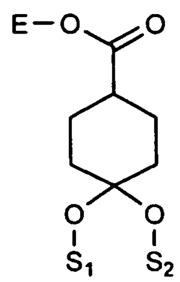

**C**

wobei $S^1$ und $S^2$ jeweils für eine Schutzgruppe stehen, vorzugsweise einen Ring bilden und zusammen für $-CH_2-CH_2-$ stehen. Der Ester **C** wird mit einem Reduktionsmittel, beispielsweise Diisobutylaluminiumhydrid zum Aldehyd **D**

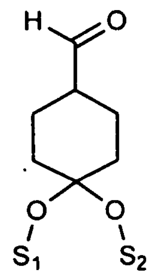

**D**

reduziert. Durch Zugabe eines Amins der allgemeinen Formel $R^3R^4NH$ und eines Cyanids, beispielsweise KCN oder NaCN, wird der Aldehyd **D** unter Zugabe einer Säure, beispielsweise Salzsäure, in einem organischen Lösungsmittel, beispielsweise Methanol oder Ethanol, zum Nitril **E** umgesetzt.

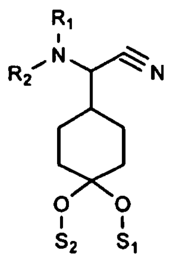

**E**

Das Nitril E wird mit einem Grignard-Reagenz der allgemeinen Formel $R^2MgHal$, wobei Hal für Br, Cl oder I steht, oder einer metallorganischen Verbindung der allgemeinen Formel $R^2Li$ in einem organischen Lösungsmittel, beispielsweise Diethylether, Dioxan oder Tetrahydrofuran, zu einer Verbindung der allgemeinen Formel **F** umgesetzt.

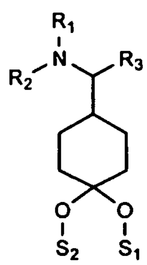

**F**

Die Schutzgruppen werden nach den üblichen Methoden abgespalten, wobei man das Keton **A** erhält.

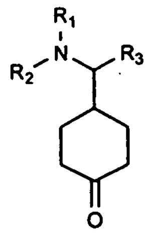

**A**

Die Verbindungen der allgemeinen Formel **B** sind entweder kommerziell erhältlich, ihre Herstellung ist aus dem Stand der Technik bekannt öder in für den Fachmann offensichtlicher Weise aus dem Stand der Technik abgeleitet worden. Insbesondere relevant sind hierfür die folgenden Zitate: Jirkovsky et al., J. Heterocycl. Chem., 12, 1975, 937-940; Campaigne et al., J. Heterocycl. Chem., 2, 1965, 231-235; Efange et al., J. Med. Chem., 41, 1998, 4486 - 4491; Ellingboe et al., J. Med. Chem., 35, 1992, 1176-1183; Pearson et al., Aust. J. Chem., 44, 1991, 907-917; Yokohama et al., Chem. Pharm. Bull., 40, 1992, 2391-2398; Beck et al., J. Chem. Soc. Perkin 1, 1992, 813-822; Shinada et al., Tetrahedron Lett., 39, 1996, 7099-7102; Garden et al., Tetrahedron, 58, 2002, 8399-8412.

Die gegebenenfalls bei den Synthesen anfallenden Diastereomeren können nach dem Fachmann bekannten Methoden zur Trennung von Diastereomeren getrennt werden, z. B. durch Chromatographie, insbesondere an Kieselgel, Normalphase oder Umkehrphase. Besonders geeignet zur Trennung der Diastereomeren ist RP-HPLC (mobile Phase Acetonitril/Wasser oder Methanol/Wasser).

Spirocyclische Cyclohexanderivate der allgemeinen Formel I, bei denen X $NR^{17}$ und $R^{17}$ $COR^{12}$ oder $SO_2$ $R^{12}$ bedeutet, können durch die Umsetzung von spirocyclischen Cyclohexanderivaten der allgemeinen Formel I, bei denen X NH bedeutet, durch Umsetzung mit einem Anhydrid oder einem Säurechlorid unter Zugabe einer Base, beispielsweise Triethylamin, erhalten werden. Vorzugsweise findet diese Reaktion unter Mikrowelleneinstrahlung statt.

Spirocyclische Cyclohexanderivate der allgemeinen Formel I, bei denen X SO oder $SO_2$ bedeuten, können durch Umsetzung von spirocyclischen Cyclohexanderivaten der allgemeinen Formel I, bei denen X S bedeutet, mit einem Oxidationsmittel, beispielsweise $H_2O_2$, erhalten werden.

Es hat sich gezeigt, dass die erfindungsgemäßen Substanzen nicht nur an den $\mu$-Opioid-Rezeptor binden, sondern auch die Serotonin- und Noradrenalin-Wiederaufnahme hemmen. Noradrenalin- und Serotonin-Wiederaufnahmehemmer haben eine antidepressive und anxiolytische Wirkung, sind jedoch auch geeignet zur Behandlung von Schmerz (Analgesics - from Chemistry and Pharmacology to Clinical Application, Wiley 2002, S. 265-284)

Die erfindungsgemäßen Substanzen eignen sich als pharmazeutische Wirkstoffe in Arzneimitteln. Ein weiterer Gegenstand der Erfindung sind daher Arzneimittel enthaltend wenigstens ein erfindungsgemäßes substituiertes spirocyclisches Cyclohexan-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten spirocyclischen Cyclohexan-Derivat gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Cyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen spirocyclischen Cyclohexan-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 20 mg/kg, bevorzugt 0,05 bis 5 mg/kg wenigstens eines erfindungsgemäßen spirocyclisches Cyclohexan-Derivats appliziert.

Das Arzneimittel kann ein erfindungsgemäßes spirocyclisches Cyclohexan-Derivat als reines Diastereomer und/oder Enantiomer, als Razemat oder als nicht-äquimolare oder äquimolare Mischung der Diastereomere und/oder Enantiomere enthalten.

Ein weiterer Gegenstand der Erfindung ist die Verwendung eines erfindungsgemäßen spirocyclischen Cyclohexan-Derivats zur Herstellung eines Arzneimittels zur Behandlung von Schmerz, insbesondere von akutem, neuropathischem oder chronischem Schmerz.

Ein weiterer Gegenstand der Erfindung ist die Verwendung eines erfindungsgemäßen spirocyclischen Cyclohexand-Derivats zur Herstellung eines Arzneimittels zur Behandlung von Depressionen und/oder zur Anxiolyse.

Die substituierten spirocyclischen Cyclohexan-Derivate der allgemeinen Formel I eignen sich auch zur Behandlung von Harninkontinenz, Diarrhöe, Pruritus, Alkohol-und Drogenmißbrauch, Medikamentenabhängigkeit und Antriebslosigkeit.

Gegenstand der Erfindung ist daher auch die Verwendung eines substituierten spirocyclischen Cyclohexan-Derivates der allgemeinen Formel I zur Herstellung eines Arzneimittels zur Behandlung von von Harninkontinenz, Diarrhöe, Pruritus, Alkohol- und Drogenmißbrauch, Medikamentenabhängigkeit und Antriebslosigkeit.

**Beispiele:**

**Synthese der Ketone 11 - 16**

Die Ketone **11 - 16** wurden in einer fünfstufigen Synthese aus 4-Oxocyclohexancarbonsäureethylester hergestellt.

1,4-Dioxa-spiro[4.5]decan-8-carbonsäure-ethylester **2** 4-Oxo-cyclohexancarbonsäure-ethylester 1 (52,8 g, 0,31 mol, Merck, Bestell-Nr. 814249), Ethylenglykol (67,4 g, 1,08 mol) und p-Toluolsulfonsäure (0,7 g) in Toluol (160 ml) wurden 20 h bei RT gerührt, die Reaktionslösung in Diethylether (300 ml) gegossen und mit Wasser, Natriumhydrogencarbonat-Lösung und Natriumchlorid-Lösung gewaschen. Die Lösung wurde getrocknet ($Na_2SO_4$), i. Vak. eingeengt und die verbliebene farblose Flüssigkeit ohne Reinigung weiter verarbeitet.

Ausbeute: 66,5 g (100 %)

$^1$H-NMR ($CDCl_3$): 1,24 (t, 3 H); 1,53 (m, 2 H); 1,76 (m, 4 H); 1,92 (m, 2 H); 2,31 (m, 1 H); 3,91 (s, 4 H); 4,11 (q, 2 H).

$^{13}$C-NMR ($CDCl_3$): 14,28 (q); 26,32 (t); 33,76 (t); 41,59 (d); 60,14 (t); 64,21 (t); 107,90 (d); 174,77 (s).

1,4-Dioxa-spiro[4.5]decan-8-carbaldehyd **3**

Eine Lösung aus 1,4-Dioxa-spiro[4.5]decan-8-carbonsäureethylester **2** (32,13 g, 150 mmol) in absol.Toluol (160 ml) wurde bei -70 bis -65 °C unter Argon tropfenweise mit Diisobutylaluminiumhydrid (1,5 M Lösung in Toluol, 102 ml, 153 mmol) versetzt und 30 min gerührt. Anschließend wurde der Ansatz bei -70 bis -60 °C durch Zugabe von Methanol (80 ml) gequencht. Die Reaktionslösung wurde auf RT erwärmt, mit gesättigter Natriumchlorid-Lösung (100 ml) versetzt und die Reaktionslösung über Kieselgur abgesaugt. Das Kiesegur wurde zweimal mit Essigester gewaschen, die wäßrige Lösung abgetrennt und zweimal mit Essigester extrahiert. Die vereinigten organischen Extrakte wurden mit gesättigter Natriumchlorid-Lösung gewaschen, über Natriumsulfat getrocknet und i. Vak. eingeengt.

Ausbeute: 24,01 g (94 %), gelbes Öl

$^1$H-NMR ($CDCl_3$): 1,54 (m, 2 H); 1,74 (m, 4 H); 1,91 (m, 2 H); 2,21 (m, 1 H); 3,91 (s, 4 H); 9,60 (s, 1 H).

$^{13}$C-NMR ($CDCl_3$): 23,35 (t); 33,37 (t); 48,18 (d); 64,30 (t); 107,89 (d); 203,51 (s).

Dimethylamino-(1,4-dioxa-spiro[4.5]dec-8-yl)-acetonitril **4**

Zu einem Gemisch aus 4N Salzsäure (37 ml) und Methanol (22 ml) wurde unter Eiskühlung 40-proz. wässrige Dimethylaminlösung (85 ml, 0,67 mol), 1,4-Dioxa-spiro-[4.5]decan-8-carbaldehyd 3 (240 g, 0,141 mol) und Kaliumcyanid (22,05 g, 0,338 mol) addiert. Die Mischung wurde **4** d bei Raumtemperatur gerührt und anschließend nach Zugabe von Wasser (80 ml) mit Diethylether (4 x 100 ml) extrahiert. Die organische Phase wurde über Natriumsulfat getrocknet, i. Vak. eingeengt und das Produkt als weißer Feststoff erhalten.

Ausbeute: 25,2 g (81 %)

Schmelzpunkt: 48-51 °C.

$^1$H-NMR ($CDCl_3$): 1,23 - 2,03 (m, 9 H); 2,28 (s, 6 H); 3,16 (d, 1 H); 3,93 (m, 4 H). $^{13}$C-NMR ($CDCl_3$): 26,67 (t); 27,93 (t); 33,87 (t); 36,94 (d); 41,90 (q); 64,30 (t); 64,36 (t); 108,33 (d); 115,94 (s).

[(1,4-Dioxa-spiro[4.5]dec-8-yl)-4-fluorphenyl-methyl]-dimethylamin **5** ($R^3$ = 4-Fluorphenyl)

Eine 1 M Lösung von 4-Fluorphenylmagnesiumbromid in THF (220 ml, 220 mmol) wurde unter Argon und Eiskühlung tropfenweise mit einer Lösung des Aminonitrils **4** (19,89 g, 88 mmol) in absol. THF (160 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet ($Na_2SO_4$) und eingeengt. Ausbeute: 31 g (>100 %)

$^{13}$C-NMR ($CDCl_3$): 26,68 (t); 28,11 (t); 34,43 (t); 34,55 (t); 37,37 (d); 41,68 (q); 64,12 (t); 73,65 (d); 108,88 (d); 114,23 (d); 114,44 (d); 130,27; 130,35; 132,43; 160,36 (s); 162,78 (s).

[(1,4-Dioxa-spiro[4.5]dec-8-yl)-3-fluorphenyl-methyl]-dimethyl-amin **6** ($R^3$ = 3-Fluorphenyl)

Eine 1 M Lösung von 3-Fluorphenylmagnesiumbromid in THF (208 ml, 208 mmol) wurde unter Argon und Eiskühlung tropfenweise mit einer Lösung des Aminonitrils **4** (23,45 g, 104 mmol) in absol. THF (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 30,33 g (99 %).

$^{1}$H-NMR ($CDCl_3$): 1,12 (m, 1 H); 1,26 (m, 1 H); 1,46 - 1,81 (m, 7 H); 2,10 (s, 6 H); 3,10 (d, 1 H); 3,90 (m, 4 H); 6,85 (m, 3 H); 7,27 (m, 1 H).

$^{13}$C-NMR ($CDCl_3$): 26,80 (t); 28,08 (t); 34,48 (t); 34,45 (t); 34,59 (t); 37,26 (d); 41,71 (q); 64,19 (t); 74,04 (t); 108,91 (d); 113,51 (d); 113,71 (d); 115,52 (d); 115,72 (d); 124,83 (d); 128,82 (d); 128,90 (d); 139,66 (s); 161,15 (s); 163,58 (s).

[(1,4-Dioxa-spiro[4.5]dec-8-yl)-phenyl-methyl]-dimethyl-amin 7 ($R^3$ = Phenyl)

Eine 25-proz. Lösung von Phenylmagnesiumchlorid (144 ml, 262.5 mmol) in THF wurde unter Argon und Eiskühlung tropfenweise mit einer Lösung des Aminonitrils **4** (23,56 g, 105 mmol) in absol. THF (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) addiert und mit Diethylether (3 x 100 ml) extrahiert. Die organische Phase wurde mit Wasser und gesättigter Natriumchlorid-Lösung gewaschen, getrocknet ($Na_2SO_4$) und eingeengt.

Ausbeute: 28,9 g (100 %).

$^{13}$C-NMR ($CDCl_3$): 27,05; 28,13; 34,48, 34,57; 36,94 (s); 41,64 (q); 64,15 (d); 74,33 (d); 109,02 (s); 126,70 (s); 127,49 (s); 129,12 (s); 136,57 (s).

[1-(1,4-Dioxa-spiro[4.5]dec-8-yl)-3-phenyl-propyl]-dimethylamin **8** ($R^3$ = Phenethyl)

Eine 1 M Lösung von Phenylethylmagnesiumchlorid in THF (242 ml, 242 mmol) wurde unter Argon und Eiskühlung tropfenweise mit einer Lösung des Aminonitrils **4** (21,93 g, 97 mmol) in absol. THF (180 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 34 g (>100 %).

$^{13}$C-NMR ($CDCl_3$): 27,43 (t); 28,95 (t); 29,42 (t); 34,82 (t); 35,40 (t); 38,76 (d); 41,16 (q); 64,17 (t); 67,41 (d); 108,86 (s); 125,41 (d); 127,66 (d); 128,11 (d); 142,69 (s).

[(1,4-Dioxa-spiro[4.5]dec-8-yl)-thiophen-2-yl-methyl]-dimethylamin **9** ($R^3$ = 2-Thiophen)

Eine 1 M Lösung von Thiophen-2-yl-magnesiumbromid in THF (20 ml, 20 mmol) wurde unter Argon und Eiskühlung tropfenweise mit einer Lösung des Aminonitrils **4** (2,24 g, 10 mmol) in absol. THF (10 ml) versetzt und 20 h bei RT gerührt. Zur Aufarbeitung des Reaktionsgemisches wurde unter Eiskühlung gesättigte Ammoniumchlorid-Lösung (10 ml) und Wasser (10 ml) gegeben und mit Diethylether (3 x 10 ml) extrahiert. Die organische Phase wurde mit Wasser und gesättigter Natriumchlorid-Lösung gewaschen, getrocknet und eingeengt.

Ausbeute: 2,8 g (100 %)

$^{13}$C-NMR ($CDCl_3$): 27,72; 27,88; 34,27; 39,28; 41,10; 64,11; 68,89; 108,88; 123,55; 125,88; 127,53; 139,50.

[(4-Chlorphenyl)-(1,4-dioxa-spiro[4.5]dec-8-yl)-methyl]-dimethyl-amin **10** ($R^3$ = 4-Chlorphenyl)

Eine 1 M Lösung von 4-Chlorphenylmagnesiumbromid in Ether (200 ml, 200 mmol) wurde unter Argon und Eiskühlung tropfenweise mit einer Lösung des Aminonitrils **4** (22,43 g, 100 mmol) in absol. Ether (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 30,9 g (100 %)

$^{13}$C-NMR ($CDCl_3$): 26,65; 28,11; 34,46; 34,60; 37,28; 41,76; 64,17; 73,80; 108,88; 127,72; 129,53; 132,39; 135,33.

4-[Dimethylamino-(4-fluorphenyl)-methyl]-cyclohexanon **11** ($R^3$ = 4-Fluorphenyl)

Das Rohprodukt des Ketals **5** (26 g, 88 mmol) wurde in Wasser (40 ml) gelöst, mit konz. Salzsäure (59 ml) versetzt und 20 h bei RT gerührt. Das Reaktionsgemisch wurde mit Diethylether (2 x 100 ml) extrahiert, die wässrige Phase unter Eiskühlung mit 5N NaOH alkalisch gestellt, mit Dichlormethan (3 x 100 ml) extrahiert, getrocknet und eingeengt.

Ausbeute: 21,36 g (98 %)

$^{13}$C-NMR ($CDCl_3$): 28,90 (t); 30,48 (t); 37,00 (t); 40,49 (t); 40,72 (t); 41,79 (q); 72,98 (d); 114,42 (d); 114,62 (d); 130,20 (d); 130,28 (d); 131,88 (s); 160,50 (s); 162,93 (s); 211,44 (s).

4-[Dimethylamino-(3-fluorphenyl)-methyl]-cyclohexanon **12** ($R^3$ = 3-Fluorphenyl) Das Ketal 6 (30,3 g, 103 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloser Feststoff isoliert.

Ausbeute: 22,4 g (87 %)

Schmelzpunkt: 72-75 °C.

$^{13}$C-NMR ($CDCl_3$): 28,97 (t); 30,44 (t); 36,90 (t); 40,52 (t); 40,75 (t); 41,82 (q); 73,37 (d); 113,84; 114,06; 115,42; 115,62; 124,71; 129,03; 129,11; 139,00; 139,06; 161,16; 163,60; 211,40 (s).

4-(Dimethylamino-phenyl-methyl)-cyclohexanon **13** ($R^3$ = Phenyl)

Das Ketal **7** (28,9 g, 0,105 mol) wurde in Wasser (44 ml) gelöst, mit konz. Salzsäure (64 ml) versetzt und 20 h bei RT gerührt. Das Reaktionsgemisch wurde mit

Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloses Öl isoliert.

Ausbeute: 18,2 g (75 % )

$^1$H-NMR ($CDCl_3$): 1,20 (1 H, m); 1,33 (1 H, m); 1,74 (1 H, m); 2,17 (6 H, s, $N(CH_3)_2$); 2,70 (6 H, m); 3,10 (1 H, d, $C_8$-H); 7,07 (2 H, m, $C_{arom}$-H); 7,23 (3 H, m, $C_{arom}$-H). $^{13}$C-NMR ($CDCl_3$): 29,13 (t); 30,56 (t); 36,90 (d); 40,61 (t); 40,82 (t); 41,89 (q); 73,79 (d); 127,05 (d); 127,67 (d); 129,00 (d); 136,13 (s); 211,79 (s).

4-(1-Dimethylamino-3-phenyl-propyl)-cyclohexanon **14** ($R^3$ = Phenethyl)

Das Rohprodukt des Ketals **8** (29,6 g, 97 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloses Öl isoliert.

Ausbeute: 16,9 g (58 %)

$^{13}$C-NMR ($CDCl_3$): 29,40 (t); 30,02 (t); 30,97 (t); 35,34 (t); 38,71 (t); 40,79 (t); 41,01 (t); 41,23 (q); 66,65 (d); 125,66 (d); 128,12 (d); 128,19 (d); 142,27 (s); 211,70 (s).

4-(Dimethylamino-thiophen-2-yl-methyl)-cyclohexanon **15** ($R^3$= 2-Thiophen)

Das Ketal **9** (2,80 g, 10 mmol) wurde in Wasser (4,4 ml) gelöst, mit konz. Salzsäure (6,4 ml) versetzt und 20 h bei RT gerührt. Das Reaktionsgemisch wurde mit Diethylether (2x10 ml) ausgeschüttelt, die wässrige Phase unter Eiskühlung mit 5N NaOH alkalisch gestellt, mit Dichlormethan (3x10 ml) extrahiert, getrocknet und eingeengt. Das Keton wurde als Öl isoliert.

Ausbeute: 1,79 g (75 %)

$^{13}$C-NMR ($CDCl_3$): 30,02; 30,18; 38,84; 40,29; 39,28; 41,17; 68,24; 123,88; 126,01; 126,34; 138,77; 211,49.

4-[(4-Chlor-phenyl)-dimethylamino-methyl]-cyclohexanon **16** ($R^3$ = 4-Chlorphenyl)

Das Ketal **10** (30,98 g, 100 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit

5N NaOH alkalisch gestellt, mit Dichlormethan (3 x 100 ml) extrahiert, getrocknet und eingeengt. Das Keton wurde als Öl isoliert.

Ausbeute: 21,9 g (82 %)

$^{13}$C-NMR ($CDCl_3$): 28,88; 30,45; 36,89; 40,49; 40,74; 41,83; 73,12; 127,87; 130,16; 132,75; 134,70; 211,35.

Synthese der 1,1-[3-(Dimethylaminoarylmethyl)-pentamethylen]3,4-dihydro-1H-2,9-diazafluoren-Derivate

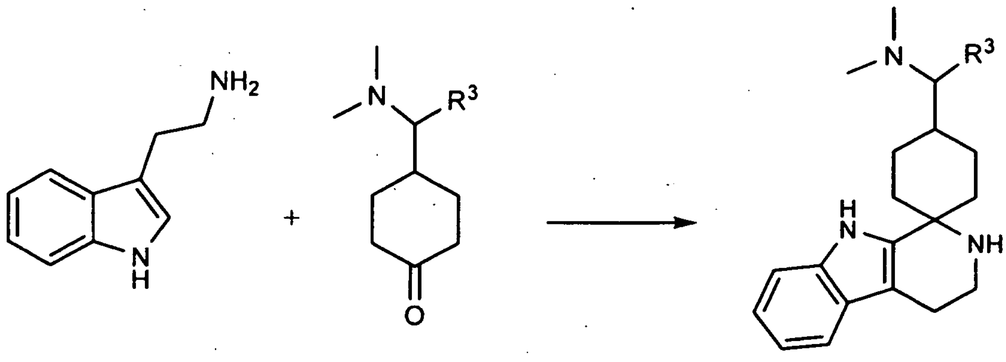

**11 - 16** **17 - 22**

1,1-[3-(Dimethylamino-(4-fluorophenyl)methyl)-pentamethylen]-3,4-dihydro-1H-2,9-diazafluoren ($R^3$ = 4-Fluorphenyl) **17**

Das Keton **11** (250 mg, 1,0 mmol) und Tryptamin (161 mg, 1,0 mmol, Acros, Bestell-Nr. 18612) wurden in MeOH (10 ml) gelöst und über Nacht bei RT gerührt. Die Reaktionslösung wurde i. Vak. eingeengt, mit Dichlorethan (10 ml) aufgenommen, TFA (1 ml) addiert und 3 h bei RT gerührt. Nach Zugabe von 1 N NaOH (5 ml) und $CH_2Cl_2$ (20 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und i. Vak. eingeengt. Das verbliebene Öl wurde in EtOH (5 ml) gelöst, wobei die Spiroverbindung über Nacht im Kühlschrank auskristallisierte.

Ausbeute: 260 mg (62 %)

Schmelzpunkt: 120-130 °C

$^{13}$C-NMR ($CDCl_3$): 23,19; 24,69; 25,86; 36,50; 36,79; 38,39; 39,26; 41,58; 52,24; 74,30; 108,33; 110,49; 114,34; 114,55; 118,04; 119,20; 121,34; 127,44; 130,40; 132,34; 135,26; 140,59; 160,45; 162,87.

1,1-[3-(Dimethylamino-(3-fluorophenyl)methyl)-pentamethylen]-3,4-dihydro-1H-2,9-diazafluoren ($R^3$ = 3-Fluorphenyl) **18**

Das Keton **12** (249 mg, 1 mmol) und Tryptamin (160 mg, 1 mmol, Acros, Bestell-Nr. 18612) wurden in MeOH (10 ml) gelöst und über Nacht bei RT gerührt. Die Reaktionslösung wurde i. Vak. eingeengt, mit Dichlorethan (10 ml) aufgenommen, TFA (1 ml) addiert und 2 h bei RT gerührt. Nach Zugabe von 1 N NaOH (5 ml) und $CH_2Cl_2$ (20 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und i. Vak. eingeengt. Der verbliebene Rückstand wurde aus EE/Hexan umkristallisiert.

Ausbeute: 170 mg (43 %)

Schmelzpunkt: 226-229 °C

$^{13}$C-NMR (DMSO-$d_6$): 23,77; 24,23; 32,97; 33,19; 35,89; 38,14; 40,78; 57,02; 72,79; 104,87; 111,12; 113,74; 115,15; 115,35; 117,87; 118,70; 121,52; 124,95; 125,46; 129,45; 134,77; 135,72; 138,35; 158,02; 160,49; 162,89.

1,1-[3-(Dimethylamino-phenylmethyl)-pentamethylen]-3,4-dihydro-1H-2,9-diazafluoren ($R^3$ = Phenyl) **19**

Das Keton **13** (1,15 g, 5 mmol) und Tryptamin (0,80 g, 5 mmol, Acros, Bestell-Nr. 18612) wurden in MeOH (50 ml) gelöst und über Nacht bei RT gerührt. Die Reaktionslösung wurde i. Vak. eingeengt, mit Dichlorethan (20 ml) aufgenommen, TFA (5 ml) addiert und 2 h bei RT gerührt. Nach Zugabe von 1 N NaOH (5 ml) und $CH_2Cl_2$ (20 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und i. Vak. eingeengt. Der verbliebene Rückstand wurde aus EE/Hexan umkristallisiert und anschließend durch Flashchromatographie mit MeCN/MeOH/0,5M $NH_4Cl$ (9: 1: 1) gereinigt.

Ausbeute: 0,84 g (45 %), 1 Diastereomer

Schmelzpunkt: 182-184°C

$^{13}C$-NMR ($CDCl_3$): 23,17; 24,92; 25,84; 36,48; 36,76; 38,14; 39,24; 41,53; 52,24; 74,96; 108,24; 110,48; 118,00; 119,14; 121,27; 126,75; 127,43; 127,55; 129,20; 135,24; 136,44; 140,68.

1,1-[3-(Dimethylamino-(phenylethyl)-methyl)-pentamethylen]-3,4-dihydro-1H-2,9-diazafluoren Citrat ($R^3$ = Phenethyl) **20**

Das Keton **14** (259 mg, 1,0 mmol) und Tryptamin (161 mg, 1,0 mmol, Acros, Bestell-Nr. 18612) wurden in MeOH (10 ml) gelöst und über Nacht bei RT gerührt. Die Reaktionslösung wurde i. Vak. eingeengt, mit Dichlorethan (10 ml) aufgenommen, TFA (1 ml) addiert und 3 h bei RT gerührt. Nach Zugabe von 1 N NaOH (5 ml) und $CH_2Cl_2$ (20 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und i. Vak. eingeengt. Das verbliebene Öl wurde in EtOH (5 ml) gelöst, 1,5M Zitronensäure in EtOH (1 ml) addiert und i. Vak. eingeengt. Die Spiroverbindung kristallisierte beim Durcharbeiten mit Acetonitril.
Ausbeute: 458 mg (77 %)
Schmelzpunkt: 130-135 °C
$^{13}C$-NMR ($CDCl_3$): 23,25; 25,22; 26,31; 29,41; 35,38; 35,46; 36,40; 36,96; 39,32; 39,84; 40,84; 41,06; 41,22; 52,09; 68,11; 108,26; 110,52; 111,03; 118,00; 119,15; 121,29; 125,57; 127,44; 128,17; 128,23; 128,43; 128,87; 135,26; 140,69; 142,73.

1,1-[3-(Dimethylamino-(thiophen-2-yl)-methyl)-pentamethylen]-3,4-dihydro-1H-2,9-diazafluoren ($R^3$ = 2-Thiophen) **21**

Das Keton **15** (474 mg, 2 mmol) und Tryptamin (320 mg, 2 mmol, Acros, Bestell-Nr. 18612) wurden in MeOH (20 ml) gelöst und über Nacht bei RT gerührt. Die Reaktionslösung wurde i. Vak. eingeengt, mit Dichlorethan (10 ml) aufgenommen, TFA (2 ml) addiert und 4 h bei RT gerührt. Nach Zugabe von 1 N NaOH (5 ml) und $CH_2Cl_2$ (20 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und i. Vak. eingeengt. Das verbliebene Öl wurde in EtOH (5 ml) gelöst, wobei die Spiroverbindung über Nacht im Kühlschrank auskristallisierte.
Ausbeute: 258 mg (27 %)
Schmelzpunkt: 167-168 °C
$^{13}C$-NMR ($CDCl_3$): 23,19; 25,60; 25,71; 36,43; 36,62; 39,26; 40,56; 41,08; 52,20; 69,78; 108,24; 110,50; 118,00; 119,15; 121,30; 123,73; 126,00; 126,46; 127,40; 135,25; 139,51; 140,61.

1,1-[3-(4-Chlorphenyl-(dimethylamino)-methyl)-pentamethylen]-3,4-dihydro-1H-2,9-diazafluoren ($R^3$ = 4-Chlorphenyl) **22**

Das Keton **16** (264 mg, 1 mmol) und Tryptamin (161 mg, 1 mmol, Acros, Bestell-Nr. 18612) wurden in MeOH (10 ml) gelöst und über Nacht bei RT gerührt. Die Reaktionslösung wurde i. Vak. eingeengt, mit Dichlorethan (10 ml) aufgenommen, TFA (1 ml) addiert und 3 h bei RT gerührt. Nach Zugabe von 1 N NaOH (5 ml) und $CH_2Cl_2$ (20 ml) wurde noch 30 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und i. Vak. eingeengt. Das verbliebene Öl wurde in EtOH (5 ml) gelöst, wobei die Spiroverbindung über Nacht im Kühlschrank auskristallisierte.
Ausbeute: 191 mg (47%)
Schmelzpunkt: 102-104 °C
$^{13}C$-NMR ($CDCl_3$): 23,18; 24,62; 25,79; 36,46; 36,76; 38,26; 39,25; 41,59; 52,22; 74,39; 108,32; 110,48; 118,02; 119,18; 121,33; 127,42; 127,78; 130,36; 132,45; 135,15; 135,25; 140,55.

**Synthese der {1,1-[3-(Dimethylamino-(4-fluorophenyl)-methyl)-pentamethylen]-3,4-dihydro-1H-2,9-diazafluoren-2-yl}methanone**

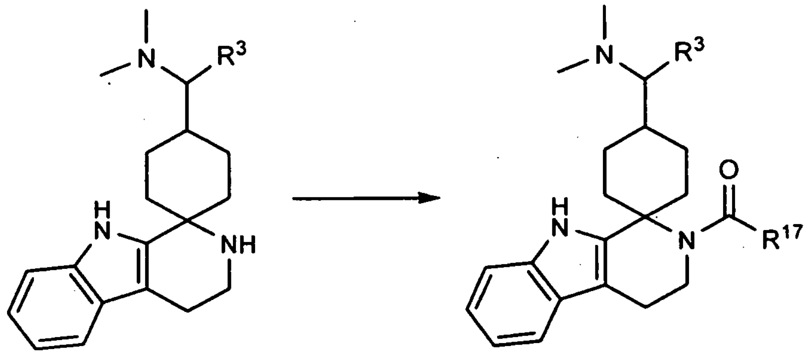

17 → 23, 24

Cyclohexyl{1,1-[3-(Dimethylamino-(4-fluorophenyl)-methyl)-pentamethylen]-3,4-dihydro-1H-2,9-diazafluoren-2-yl}methanon ($R^3$ = 4-Fluorphenyl, $R^{17}$ = Cyclohexyl) **23** 1,1-[3-(Dimethylamino-(4-fluorophenyl)methyl)-pentamethylen]-3,4-dihydro-1H-2,9-diazafluoren **17** (75 mg, 0,19 mmol) gelöst in Acetonitril (2 ml) wurde im Mikrowellenglas vorgelegt und mit Cyclohexancarbonsäurechlorid (35 mg, 0,24 mmol), N-Ethyl-diisopropylamin (31 mg, 0,24 mmol) und mit DMAP (2mg, 0,02 mmol) für 10 min. bei 120°C in der Mikrowelle bestrahlt.

Die entstandene Suspension wurde mit ges. Natriumhydrogencarbonat-Lsg. gequencht und mit Essigester extrahiert. Die wässrige Phase wurde noch zweimal mit DCM extrahiert. Die vereinigten organischen Phasen wurden über Magnesiumsulfat getrocknet, abgesaugt und eingeengt. Die Aufreinigung erfolgte durch Flashchromatographie mit Essigester/Hexan (2 : 1 mit 5% Triethylamin). Ausbeute: 73 %

1H NMR (600 MHz, DMSO-$d_6$) 0,88 - 1,18 (m, 5 H) 1,19- 1,34 (m, 2 H) 1,35- 1,45 (m, 2H) 1,46 - 1,55 (m, 1H) 1,55 - 1,78 (m, 5 H) 1,77 - 1,90 (m, 1 H) 2,07 (s, 7 H) 2,55 - 2,68 (m, 3 H) 2,70 - 2,84 (m, 1 H) 2,87 - 3,06 (m, 2H) 3,64 - 3,89 (m, 2 H) 6,92 - 6,93 (m, 1 H) 6,96 - 7,05 (m, 1 H) 7,08 - 7,17 (m, 2 H) 7,17 - 7,24 (m, 2 H) 7,24 - 7,33 (m, 2 H) 10,69 (s, 1 H)

Phenyl{1,1-[3-(Dimethylamino-(4-fluorophenyl)-methyl)-pentamethylen]-3,4-dihydro-1H-2,9-diazafluoren-2-yl}methanon ($R^3$ = 4-Fluorphenyl, $R^{17}$ = Phenyl) **24** 1,1-[3-(Dimethylamino-(4-fluorophenyl)methyl)-pentamethylen]-3,4-dihydro-1H-2,9-diazafluoren **17** (75 mg, 0,19 mmol) gelöst in Acetonitril (2 ml) wurde im Mikrowellenglas vorgelegt und mit Benzoylchlorid (33 mg, 0,24 mmol), N-Ethyl-diisopropylamin (31 mg, 0,24 mmol) und mit DMAP (2mg, 0,02 mmol) für 10 min. bei 120°C in der Mikrowelle bestrahlt.

Die entstandene Suspension wurde mit ges. Natriumhydrogencarbonat-Lsg. gequencht und mit Essigester extrahiert. Die wässrige Phase wurde noch zweimal mit DCM extrahiert. Die vereinigten organischen Phasen wurden über Magnesiumsulfat getrocknet, abgesaugt und eingeengt. Die Aufreinigung erfolgte durch Flashchromatographie mit Essigester/Hexan (2 : 1 mit 5% Triethylamin). Ausbeute: 98 %

1H NMR (600 MHz, DMSO-$d_6$) 1,09 - 1,29 (m, 3 H) 1,45 - 1,57 (m, 1H) 1,77 - 1,91 (m, 2 H) 1,91 - 2,00 (m, 1H) 2,05 - 2,20 (m, 7 H) 2,78 - 3,03 (m, 3H) 3,03 - 3,18 (m, 1H) 3,44 - 3,66 (m, 2H) 6,93 (t, *J*=7,18 Hz, 1 H) 7,03 (t, *J*=7,55 Hz, 1H) 7,09 - 7,20 (m, 4 H) 7,26 (dd, *J*=7,93, 5,67 Hz, 2H) 7,30 (dd, *J*=17,37, 8,31 Hz, 2H) 7,39 (t, *J*=7,55 Hz, 3 H) 7,48 (t, *J*=7,55 Hz, 1H) 10,80 (s, 1 H)

**Synthese der 1,1-[3-(Dimethylamino-(aryl)-methyl)-pentamethylen]-1,3,4,9-tetrahydropyrano[3,4-b]indol-Derivate**

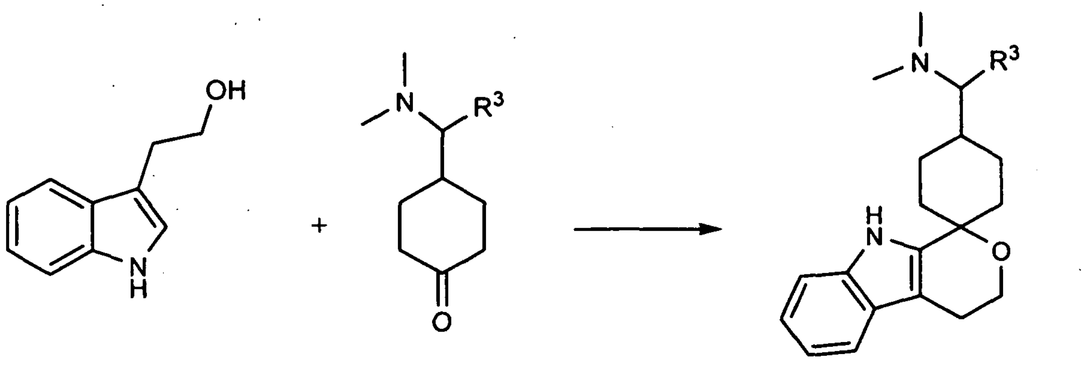

16 - 22

25-30

1,1-[3-(Dimethylamino-(4-fluorphenyl)-methyl)-pentamethylen]-1,3,4,9-tetrahydro-pyrano[3,4-b]indol ($R^3$ = 4-Fluorphenyl) **25**

Keton **11** (250 mg, 1,0 mmol) und das Tryptophol (161 mg, 1 mmol, Acros, Bestell-Nr. 14060) wurden unter Argon in Methylenchlorid (5 ml) gelöst und mit Methansulfonsäure (106 mg, 1,1 mmol) versetzt. Der Ansatz wurde über Nacht bei RT gerührt, 1N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (2:1) gereinigt.

Ausbeute: 113 mg (29 %)

$^{13}$C-NMR ($CDCl_3$): 22,87; 24,75; 26,21; 36,02; 38,45; 41,08; 60,02; 72,62; 74,75; 107,44; 110,98; 114,65; 114,85; 118,36; 119,76; 121,83; 127,35; 130,70; 132,87; 135,78; 139,24; 160,77; 163,19.

1,1-[3-(Dimethylamino-(3-fluorphenyl)-methyl)-pentamethylen]-1,3,4,9-tetrahydro-pyrano[3,4-b]indol Citrat ($R^3$ = 3-Fluorphenyl) **26**

Keton **12** (498 mg, 2 mmol) und das Tryptophol (322 mg, 2 mmol, Acros, Bestell-Nr. 14060) wurden unter Argon in Methylenchlorid (20 ml) gelöst und mit Trifluormethansulfonsäure (0,18 ml, 2,028 mmol) versetzt. Der Ansatz wurde über Nacht bei RT gerührt, der dabei ausgefallene Niederschlag wurde abgesaugt, in 2N NaOH (5 ml) und Methylenchlorid (10 ml) aufgenommen und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Ausbeute: 460 mg (58 %), poröser Feststoff, 2 Diastereomere

$^{13}$C-NMR (DMSO-$d_6$): 20,95; 22,21; 22,40; 29,64; 29,75; 31,00; 34,07; 41,10; 43,12; 58,86; 65,03; 71,66; 71,88; 72,05; 73,29; 104,93; 110,74; 114,03; 114,23; 115,69; 115,85; 117,52; 117,79; 118,20; 120,37; 120,49; 125,53; 126,26; 129,61; 129,68; 135,22; 135,33; 137,29; 139,26; 139,66; 160,57; 162,99; 170,94; 174,85.

1,1-[3-(Dimethylamino-(phenyl)-methyl)-pentamethylen]-1,3,4,9-tetrahydropyrano[3,4-b]indol Citrat ($R^3$ = Phenyl) **27**

Keton **13** (462 mg, 2 mmol) und das Tryptophol (322 mg, 2 mmol, Acros, Bestell-Nr. 14060) wurden unter Argon in Methylenchlorid (100 ml) gelöst und mit Trifluormethansulfonsäure (0,18 ml, 2,03 mmol) versetzt. Der Ansatz wurde 6 h bei RT gerührt, 2N NaOH (5 ml) zugegeben und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt.

Ausbeute: 740 mg (99 %), poröser Feststoff, 2 Diastereomere

Der Feststoff wurde in Ethanol (5 ml) gelöst und mit Citronensäure (2 mmol) versetzt. Im Kühlschrank setzte sich ein Öl ab, die Lösung wurde dekantiert und der Rückstand mit Ether versetzt. Dabei fiel ein farbloser Niederschlag aus, der abgesaugt und getrocknet wurde.

Ausbeute: 431 mg (38 %), 2 Diastereomere

Schmelzpunkt: 162-165 °C

$^{13}$C-NMR ($CDCl_3$): 22,55; 22,93; 24,77; 25,59; 25,85; 34,68; 35,08; 37,89; 41,65; 42,72; 59,67; 59,82; 72,34; 72,58; 73,20; 75,05; 107,03; 110,65; 117,98; 118,09; 119,39; 121,46; 126,36; 126,72; 127,02; 127,16; 127,53; 127,78; 129,18; 129,43; 135,19; 135,44; 136,60; 137,57; 138,45; 139,03.

1,1-[3-(Dimethylamino-(phenylethyl)-methyl)-pentamethylen]-1,3,4,9-tetrahydropyrano[3,4-b]indol ($R^3$ = Phenethyl) **28**

Keton **14** (259 mg, 1,0 mmol) und Tryptophol (161 mg, 1 mmol, Acros, Bestell-Nr. 14060) wurden unter Argon in Methylenchlorid (5 ml) gelöst und mit Methansulfonsäure (106 mg, 1,1 mmol) versetzt. Der Ansatz wurde über Nacht bei RT gerührt, 1N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (2:1) gereinigt.

Ausbeute: 187 mg (46 %), gelbes Öl

$^{13}C$-NMR ($CDCl_3$): 22,59; 25,04; 26,42; 29,36; 35,36; 35,77; 35,84; 39,19; 41,23; 59,73; 68,08; 72,20; 107,00; 110,68; 118,07; 119,37; 121,45; 125,54; 127,01; 128,15; 128,22; 135,45; 138,98; 142,75.

1,1-[3-(Dimethylamino-(thiophen-2-yl)-methyl)-pentamethylen]-1,3,4,9-tetrahydro-pyrano[3,4-b]indol ($R^3$ = 2-Thiophen) **29**

Keton **15** (237 mg, 1 mmol) und das Tryptophol (161 mg, 1 mmol, Acros, Bestell-Nr. 14060) wurden unter Argon in Methylenchlorid (5 ml) gelöst und mit Methansulfonsäure (106 mg, 1,1 mmol) versetzt. Der Ansatz wurde über Nacht bei RT gerührt, 1N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 241 mg (63 %), poröser Feststoff

$^{13}C$-NMR ($CDCl_3$): 22,61; 23,60; 24,32; 33,37; 33,46; 36,41; 41,81; 59,82; 60,43; 65,77; 72,34; 72,84; 107,13; 110,72; 118,07; 119,40; 121,50; 123,88; 126,23; 126,42; 126,63; 135,37; 138,77; 140,10.

1,1-[3-(Dimethylamino-(4-chlorphenyl)-methyl)-pentamethylen]-1,3,4,9-tetrahydro-pyrano[3,4-b]indol ($R^3$ = 4-Chlorphenyl) **30**

Keton **16** (265 mg, 1 mmol) und das Tryptophol (161 mg, 1 mmol, Acros, Bestell-Nr. 14060) wurden unter Argon in Methylenchlorid (5 ml) gelöst und mit Methansulfonsäure (106 mg, 1,1 mmol) versetzt. Der Ansatz wurde über Nacht bei RT gerührt, 1N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Ausbeute: 404 mg (99 %), poröser Feststoff

$^{13}C$-NMR ($CDCl_3$): 22,54; 23,10; 24,38; 25,80; 33,95; 34,16; 34,70; 35,65; 37,95; 41,74; 42,23; 59,68; 59,80; 71,10; 72,29; 74,48; 107,06; 110,66; 118,10; 121,49; 121,62; 127,00; 127,75; 127,96; 130,33; 132,40; 135,32; 135,45; 138,87.

Synthese der 6-Fluor-1,1-[3-(dimethylamino-(aryl)-methyl)-pentamethylen]-1,3,4,9-tetrahydro-pyrano[3,4-b]indol-Derivate

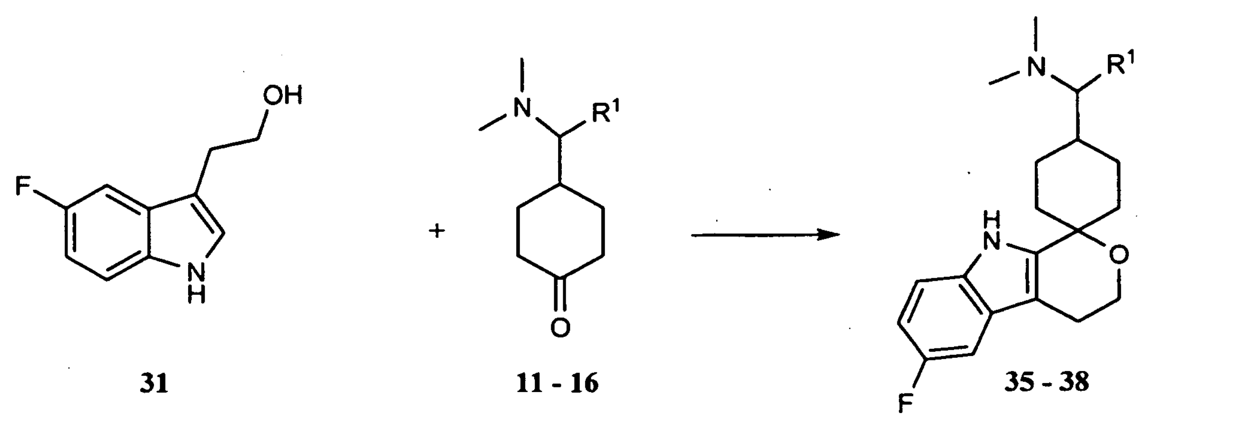

**31** + **11 - 16** → **35 - 38**

(5-Fluor-3-hydroxy-2-oxo-2,3-dihydro-1*H*-indol-3-yl)essigsäure-ethylester **34**

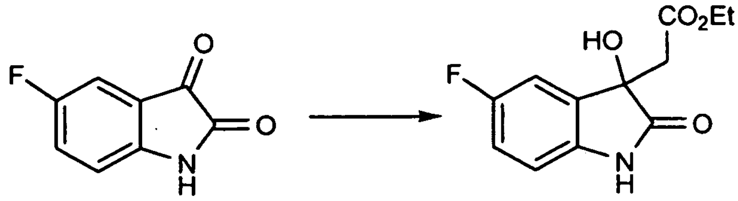

**32** **34**

5-Fluorisatin **32** (10 mmol, Lancaster, Bestell-Nr. 14553) wurde in einem Gemisch aus Ethanol/Pyridin/Essigsäure (50 ml, 15 : 5 : 2) gelöst, mit Ethyl-kaliummalonat **33** (1,87 g, 11 mmol) versetzt und 14h unter Rückfluß erhitzt. Der Verlauf der Reaktion wurde mittels DC (Eluent: Ethylacetat/Hexan 1 : 1) kontrolliert. Zur Aufarbeitung wurde das Lösungsmittelgemisch im Vakuum abdestilliert. Der Rückstand wurde in Ethylacetat (50 ml) aufgenommen und mit Wasser (50 ml) ausgeschüttelt. Nach Phasentrennung wurde die wäßrige Phase zweimal mit Ethylacetat (je 30 ml) extrahiert. Die kombinierten organischen Phasen wurden mit 2N HCl (50 ml) gewaschen, über $Na_2SO_4$ getrocknet und im Vakuum auf 20 ml eingeengt. Zu der Lösung wurde solange Hexan zugegeben, bis Kristallisation des Produktes einsetzte.

Zur Vervollständigung der Kristallisation wurde der Ansatz 12 h auf 10 °C gekühlt. Der Feststoff wurde abgesaugt und im Vakuum getrocknet.

Ausbeute: 89 %

Schmelzpunkt: 133-135°C

2-(5-Fluor-1*H*-indol-3-yl)ethanol **31**

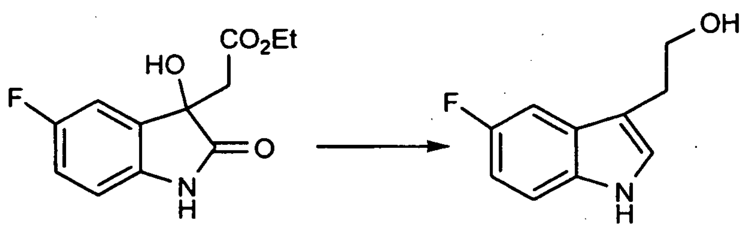

**34** **31**

Das Aldolprodukt **34** (10 mmol) wurde unter Ar-Atmosphäre in absolutem THF (20 ml) gelöst. Der Ansatz wurde anschließend unter Wasserbadkühlung mit $BH_3 \times THF$ (40 ml, 1 M Lösung, 40 mmol) versetzt und 14 h bei Raumtemperatur gerührt. Der Reaktionsverlauf wurde mittels DC überwacht. Nach Beendigung der Reaktion wurde die Reaktionslösung zu einer Mischung aus Ethylacetat (50 ml) und $H_2O$ (50 ml) gegeben. Nach Phasentrennung wurde die wäßrige Phase zweimal mit Ethylacetat (je 30 ml) extrahiert. Die kombinierten organischen Phasen wurden über $Na_2SO_4$ getrocknet und im Vakuum eingeengt. Der Rückstand wurde über Kieselgel mit Ethylacetat filtriert. Das nach Entfernen des Lösungsmittels erhaltene Produkt als ausreichend reines Öl vor und kristallisierte spontan.

Die $^1$H-NMR-Daten entsprachen der Literatur: T. V. RajanBabu, B. L. Chendard, M. A. Petti, J. Org. Chem. 1986, 51, 1704 - 1712.

6-Fluor-1,1-[3-(dimethylamino-(4-fluorphenyl)-methyl)-pentamethylen]-1,3,4, 9-tetrahydropyrano[3,4-b]indol ($R^3$ = 4-Fluorphenyl) **35**

Keton **11** (249 mg, 1,0 mmol) und das Tryptophol-Derivat **31** (179 mg, 1,0 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1N NaOH (10 ml) und $CH_2Cl_2$ (10 ml) addiert und 30 min nachgerührt. Nach dem Trennen der Phasen

wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 214 mg (52 %), poröser Feststoff

$^{13}$C-NM R ($CDCl_3$): 22,37; 22,97; 23,07; 25,04; 33,51; 34,11; 34,78; 42,02; 59,68; 71,13; 72,99; 103,10; 103,33; 107,62; 107,66; 109,66; 109,92; 111,19; 111,29; 114,63; 114,84; 127,00; 130,79; 131,88; 133,04; 140,67; 156,52; 159,05; 160,78; 163,22.

6-Fluor-1,1-[3-(dimethylamino-(3-fluorphenyl)-methyl)-pentamethylen]-1, 3,4,9-tetrahydropyrano[3,4-b]indol ($R^3$ = 3-Fluorphenyl) **36**

Keton **12** (249 mg, 1,0 mmol) und das Tryptophol-Derivat **31** (179 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 213 mg (52 %), poröser Feststoff

$^{13}$C-NMR ($CDCl_3$): 22,37; 23,10; 24,71; 33,68; 33,81; 34,48; 42,01; 59,68; 71,05; 72,91; 103,11; 103,34; 107,57; 107,61; 109,59; 109,86; 111,25; 111,35; 113,97; 114,19; 115,97; 116,17; 125,10; 127,04; 127,13; 129,26; 129,34; 131,90; 140,01; 140,07; 140,75; 156,72; 159,05; 161,47; 163,92.

6-fluor-1,1-[3-(dimethylamino-(phenyl)-methyl)-pentamethylen]-1,3,4,9-tetrahydro-pyrano[3,4-b]indol ($R^3$ = Phenyl) **37**

Keton **13** (231 mg, 1 mmol) und das Tryptophol-Derivat **31** (179 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 215 mg (55 %), poröser Feststoff

$^{13}$C-NMR ($CDCl_3$): 22,40; 22,74; 23,07; 25,47; 34,31; 34,51; 34,95; 42,68; 59,69; 72,63; 73,15; 103,03; 103,25; 107,56; 107,61; 109,50; 109,76; 111,27; 111,36; 126,86; 126,96; 127,28; 127,92; 129,60; 131,82; 137,78; 140,59; 156,67; 159,00. 6-fluor-1,1-[3-(dimethylamino-(4-chlorphenyl)-methyl)-pentamethylen]-1,3,4,9-tetrahydropyrano[3,4-b] indol ($R^3$ = 4-Chlorphenyl) **38**

Keton **16** (265 mg, 1,0 mmol) und das Tryptophol-Derivat 31 (179 mg, 1,0 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 160 mg (37 %), poröser Feststoff

$^{13}$C-NMR ($CDCl_3$): 22,38; 22,91; 25,00; 26,91; 33,89; 34,10; 34,65; 42,02; 42,23; 59,69; 71,27; 72,97; 103,09; 103,32; 107,64; 107,69; 109,68; 109,95; 111,27; 111,37; 126,98; 127,08; 128,10; 130,75; 131,89; 132,98; 135,88; 140,60; 156,73; 159,06.

**Synthese der 3,6-Dimethyl-1,1-[3-(dimethylamino-(aryl)-methyl)-pentamethylen]-1,3,4,9-tetrahydropyrano [3,4-b]indol-Derivate**

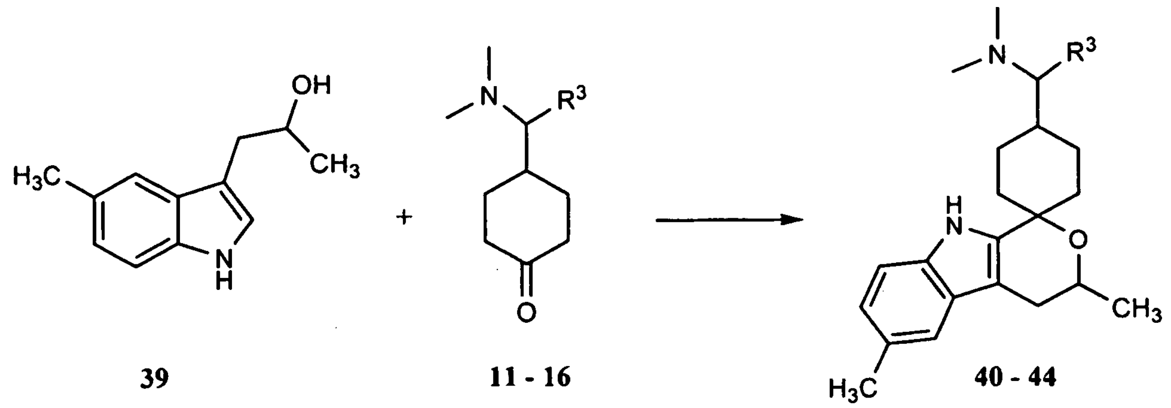

39 11 - 16 40 - 44

Die Synthese des 1-(5-Methyl-1H-indol-3yl)propan-2-ols **39** erfolgte analog der bei S. J. Garden, R. B. da Silva and A. C. Pinto in Tetrahedron 2002, 58, 8399-8412 Methode.

3-Hydroxy-5-methyl-3-(2-oxo-propyl)-1,3-dihydro-indol-2-on **47**

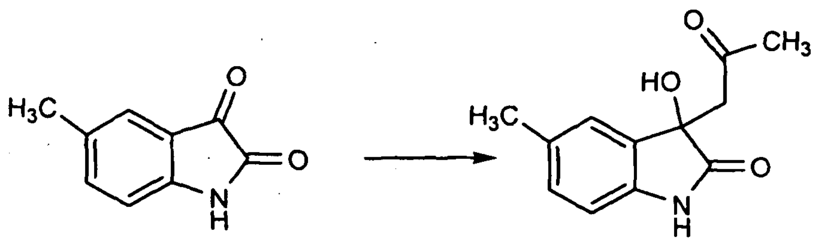

45 47

5-Methylisatin **45** (10 mmol, Lancaster, Bestell-Nr. 8009) wurde in Aceton **46** (50 ml) gelöst, mit $K_2CO_3$ (138 mg, 1 mmol) versetzt und 24 h bei Raumtemperatur gerührt. Der Verlauf der Reaktion wurde mittels DC (Eluent: Ethylacetat) kontrolliert. Zur Aufarbeitung wurde die Reaktionslösung im Vakuum auf 40 ml eingeengt, wobei das Produkt aus der resultierenden Lösung auszufallen begann. Zur Vervollständigung der Fällung wurde die Lösung über Nacht im Kühlschrank auf ca. 10°C gekühlt. Das ausgefallene Produkt wurde abgesaugt und im Vakuum getrocknet. Weiteres
Reaktionsprodukt konnte isoliert werden, indem das Aceton vollständig entfernt und der verbleibende Rückstand in Ethylacetat gelöst wurde. Der bei anschließender Fällung mit Hexan gebildete Feststoff wurde abgesaugt und im Vakuum getrocknet.
Ausbeute: 74 %
Schmelzpunkt: 158°C

1-(5-Methyl-1*H*-indol-3-yl)propan-2-ol **39**

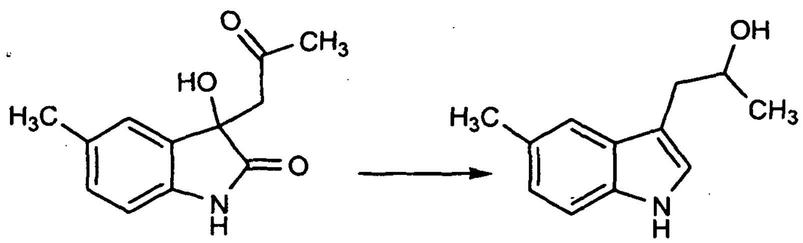

47 39

Das Aldolprodukt **47** (10 mmol) wurde unter Ar-Atmosphäre in absolutem THF (20 ml) gelöst. Der Ansatz wurde anschließend unter Wasserbadkühlung mit $BH_3 \times THF$ (30 ml, 1 M Lösung, 30 mmol) versetzt und 15 h bei Raumtemperatur gerührt. Der Reaktionsverlauf wurde durch DC überwacht. Nach Beendigung der Reaktion wurde die Reaktionslösung zu einer Mischung aus Ethylacetat (50 ml) und $H_2O$ (50 ml) gegeben. Nach Phasentrennung wurde die wäßrige Phase zweimal mit Ethylacetat (je 30 ml) extrahiert. Die kombinierten organischen Phasen wurden über $Na_2SO_4$ getrocknet und im Vakuum eingeengt. Der Rückstand wurde über Kieselgel mit Ethylacetat filtriert. Das nach Entfernen des Lösungsmittels erhaltene Rohprodukt wurde durch eine säulenchromatische Reinigung an Kieselgel (Eluent: Ethylacetat/Cyclohexan: 1 : 2) aufgereinigt.

Ausbeute: 78 %

3,6-Dimethyl-1,1-[3-(dimethylamino-(4-fluorphenyl)-methyl)-pentamethylen]-1,3,4,9-tetrahydropyrano[3,4-b]indol ($R^3$ = 4-Fluorphenyl) **40**

Keton **11** (249 mg, 1,0 mmol) und das Tryptophol-Derivat **39** (189 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (2:1) gereinigt.

Ausbeute: 209 mg (50 %), poröser Feststoff

$^{13}$C-NMR ($CDCl_3$): 22,13; 22,35; 23,50; 24,87; 25,77; 26,00; 26,21; 26,75; 27,34; 30,30; 30,61; 33,68; 34,28; 35,42; 36,08; 38,29; 38,45; 42,04; 42,07; 42,52; 42,62; 65,65; 65,75; 72,92; 74,68; 77,44; 107,61; 110,62; 114,55; 114,58; 114,78; 115,07; 118,06; 118,17; 123,12; 123,30; 127,49; 128,88; 130,73; 130,81; 130,97; 131,04; 132,80; 132,86; 132,89; 134,14; 134,30; 139,65; 160,75; 163,17.

3,6-Dimethyl-1,1-[3-(dimethylam ino-(3-fluorphenyl)-methyl)-pentamethylen]-1,3,4,9-tetrahydropyrano[3,4-b]indol ($R^3$ = 3-Fluorphenyl) **41**

Keton **12** (249 mg, 1 mmol) und das Tryptophol-Derivat **39** (189 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 277 mg (65 %), poröser Feststoff $^{13}$C-NMR ($CDCl_3$): 21,55; 21,80; 22,02; 23,35; 25,25; 30,05; 33,21; 33,87; 34,90; 35,49; 38,01; 41,71; 42,10; 42,18; 65,43; 71,24; 72,57; 73,33; 74,70; 107,32; 110,32; 113,84; 114,04; 115,87; 116,07; 117,76; 122,81; 122,93; 125,00; 126,69; 128,60; 129,07; 129,16; 133,87; 138,97; 140,04; 140,09; 161,27; 163,70.

3,6-diethyl-1,1-[3-(dimethylamino-(phenyl)-methyl)-pentamethylen]-1,3,4,9-tetrahydropyrano[3,4-b]indol Citrat ($R^3$ = Phenyl) **42**

Keton **13** (231 mg, 1 mmol) und das Tryptophol-Derivat **39** (189 mg, 1 mmol) wurden unter Argon in Methylenchlorid (10 ml) gelöst und mit Trifluormethansulfonsäure (0,09 ml, 1,014 mmol) versetzt. Der Ansatz wurde über Nacht bei RT gerührt, 2N NaOH (2 ml) zugegeben und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Das verbleibende Öl wurde in Ethanol (3 ml) gelöst und mit Citronensäure (1,2 mmol) versetzt. Da kein Feststoff auskristallisierte, wurde die Lösung eingeengt, mit Acetonitril (20 ml) aufgekocht und die entstandenen Kristalle abgesaugt.

Ausbeute: 160 mg (27 %), 2 Diastereomere

Schmelzpunkt: 245-250 °C

$^{13}$C-NMR (DMSO-$d_6$): 20,77; 21,22; 21,77; 22,22; 22,63; 27,88; 29,66; 31,01; 31,98; 32,22; 36,69; 40,94; 43,55; 64,65; 65,49; 71,66; 72,34; 104,72; 110,39; 117,13; 121,80; 126,36; 126,46; 127,24; 127,75; 129,29; 133,85; 139,89; 170,90; 175,48.

3,6-dimethyl-1,1-[3-(dimethy)amino-(phenylethyl)-methyl)-pentamethylen]-1,3,4,9-tetrahydropyrano[3,4-b]indol ($R^3$ = Phenethyl) **43**

Keton **14** (259 mg, 1,0 mmol) und das Tryptophol-Derivat **39** (189 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde

die wässrige Phase mit $CH_2Cl_2$ extrahiert, die organischen Phasen vereinigt, getrocknet ($Na_2SO_4$) und i. Vak. eingeengt. Das Produkt wurde durch Flashchromatographie mit Essigester/Cyclohexan (2:1) gereinigt.

Ausbeute: 159 mg (37 %), poröser Feststoff $^{13}$C-NMR ($CDCl_3$): 21,93; 24,95; 26,41; 29,42; 30,06; 34,13; 35,38; 35,58; 38,09; 39,29; 41,21; 65,42; 68,11; 68,26; 72,51; 107,13; 110,32; 117,84; 122,81; 125,56; 127,22; 128,18; 128,21; 128,56; 134,03; 139,40; 142,85; 142,89.

3,6-Dimethyl-1,1-[3-(dimethylamino-(4-chlorphenyl)-methyl)-pentamethylen]-1,3,4,9-tetrahydropyrano[3,4-b]indol ($R^3$ = 4-Chlorphenyl) **44**

Keton **16** (265 mg, 1 mmol) und das Tryptophol-Derivat **39** (189 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 300 mg (68 %), poröser Feststoff

$^{13}$C-NMR ($CDCl_3$): 21,55; 22,01; 23,39; 24,59; 25,43; 25,82; 27,03; 29,99; 30,04; 30,29; 33,39; 33,86; 33,95; 34,84; 35,00; 35,80; 37,76; 37,98; 41,65; 41,71; 42,22; 42,33; 65,36; 65,43; 71,25; 72,57; 73,39; 74,43; 107,14; 107,33; 110,29; 117,72; 122,81; 123,01; 126,61; 127,20; 127,69; 128,56; 130,37; 132,40; 132,80; 133,86; 134,02; 135,27; 135,38; 135,81; 138,82; 139,29.

**Synthese der 6-Fluor-3-methyl-1,1-[3-(dimethylamino-(aryl)-methyl)-pentamethylen]-1,3,4,9-tetrahydropyrano [3,4-b]indol-Derivate**

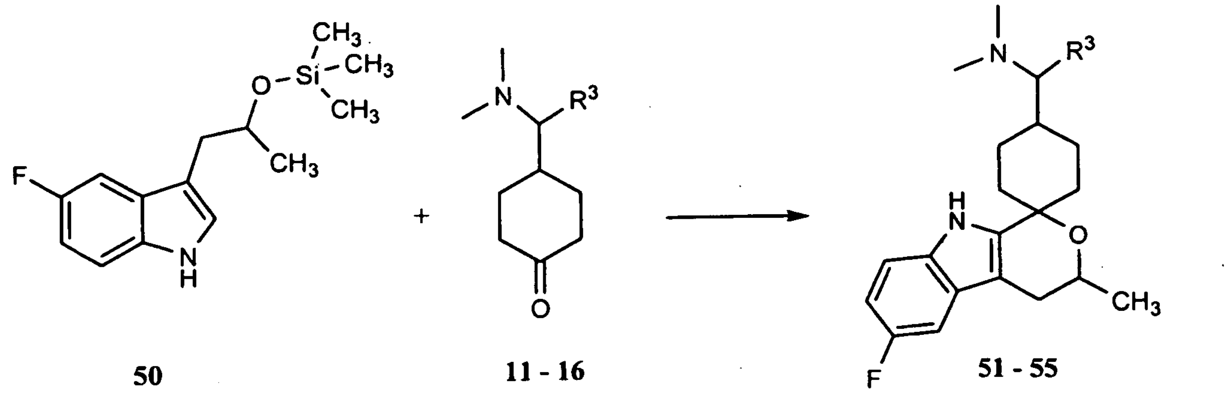

50 11 - 16 51 - 55

5-Fluor-3-hydroxy-3-(2-oxo-propyl)-1,3-dihydroindol-2-on **48** 5-Fluorisatin **32** (10 mmol, Lancaster, Bestell-Nr. 14553) wurde in Aceton **46** (50 ml) gelöst, mit $K_2CO_3$ (138 mg, 1 mmol) versetzt und 5 h bei Raumtemperatur gerührt. Der Verlauf der Reaktion wurde mittels DC (Eluent: Ethylacetat) kontrolliert.

Zur Aufarbeitung wurde das Aceton vollständig im Vakuum abdestilliert. Der Rückstand wurde in Ethylacetat gelöst und über Kieselgel mit Ethylacetat filtriert. Das Filtrat wurde auf 20 ml eingeengt und bis zur einsetzenden Fällung mit Hexan versetzt. Zur Vervollständigung der Fällung wurde die Lösung über Nacht im Kühlschrank auf ca. 10 °C gekühlt. Das ausgefallene Produkt wurde abgesaugt und im Vakuum getrocknet. Das so erhaltene Rohprodukt wurde aus Ethylacetat/Hexan (1 : 4) umkristallisiert.

Ausbeute: 90 %

Schmelzpunkt: 153-155°C

Die $^{1}$H-NMR-Daten entsprachen der Literatur: G. K. Jnaneshwara, V. H. Deshpande, Synthetic Commun. 1999, 29, 20, 3627 - 3633.

1-(5-Fluor-1*H*-indol-3-yl)propan-2-ol **49**

Das Aldolprodukt **48** (10 mmol) wurde unter Ar-Atmosphäre in absolutem THF (20 ml) gelöst. Der Ansatz wurde anschließend unter Wasserbadkühlung mit $BH_3 \times THF$ (30 ml, 1 M Lösung, 30 mmol) versetzt und 4 h bei Raumtemperatur gerührt. Der Reaktionsverlauf wurde durch DC überwacht. Nach Beendigung der Reaktion wurde die Reaktionslösung

zu einer Mischung aus Ethylacetat (50 ml) und $H_2O$ (50 ml) gegeben. Nach Phasentrennung wurde die wäßrige Phase zweimal mit Ethylacetat (je 30 ml) extrahiert. Die kombinierten organischen Phasen wurden über $Na_2SO_4$ getrocknet und im Vakuum eingeengt. Der Rückstand wurde über Kieselgel mit Ethylacetat filtriert. Das nach Entfernen des Lösungsmittels erhaltene Produkt lag als ausreichend reines Öl vor und kristallisiert spontan.

Ausbeute: 92 %

Schmelzpunkt: 74 - 76°C

Für die anschließende Synthese der Spiroverbindungen ist es eventuell notwendig, die erhaltenen 3-(2-Hydroxypropyl)-1*H*-indole für die anschließende Synthese der Spiroverbindungen zu aktivieren. Dieses geschieht durch Silylierung der Alkoholfunktion.

5-Fluor-3-(2-trimethylsilanyloxy-propyl)-1*H*-indol **50**

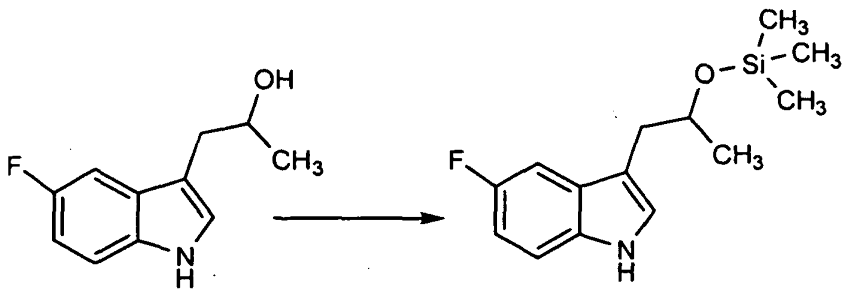

**49** **50**

Der Alkohol **49** (10 mmol) wurde unter Feuchtigkeitsausschluß in absolutem THF (15 ml) gelöst, mit Trimethylchlorsilan (2,1 ml, 15,3 mmol) und Hexamethyldisilazan (4,9 ml, 23,3 mmol) versetzt und 24 h bei Raumtemperatur gerührt. Der Reaktionsverlauf wurde dünnschichtchromatisch überwacht. Nach Beendigung der Reaktion wurde das Lösungsmittel im Vakuum entfernt. Anschließend wurde der als zähflüssiges Öl verliebende Rückstand in Diethylether (30 ml) aufgenommen und mit gesättigter $NaHCO_3$ (30 ml) versetzt. Die Phasen wurden getrennt, die wäßrige Phase wurde zweimal mit $Et_2O$ (je 15 ml) extrahiert. Die kombinierten organischen Phasen wurden über $Na_2SO_4$ getrocknet, und im Vakuum eingeengt. Das erhaltene Produkt lag zunächst als Öl vor und kristallisiert spontan. Zur weiteren Reinigung wurde das erhaltende Rohprodukt aus Hexan umkristallisiert.

Ausbeute: 88 %

Schmelzpunkt: 56 - 58°C

6-Fluor-3-methyl-1,1-[3-(dimethylamino-(4-fluorphenyl)-methyl)-pentamethylen]-1,3,4,9-tetrahydropyrano[3,4-b]indol ($R^3$ = 4-Fluorphenyl) **51**

Keton **11** (249 mg, 1,0 mmol) und das Tryptophol-Derivat **50** (265 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Das Produkt wurde durch Flashchromatographie mit Essigester/Cyclohexan (2:1) gereinigt.

Ausbeute: 348 mg (82%), poröser Feststoff

$^{13}C$-NMR ($CDCl_3$): 21,74; 21,94; 24,31; 24,52; 25,61; 25,83; 27,01; 29,85; 30,28; 33,36; 33,83; 37,70; 37,91; 38,09; 41,68; 41,71; 42,33; 65,27; 72,57; 74,34; 103,06; 103,29; 107,84; 107,87; 109,20; 109,46; 111,07; 111,16; 114,23; 114,43; 127,30; 127,40; 130,39; 130,46; 130,65; 132,17; 132,50; 141,21; 156,50; 158,82; 160,42; 162,85.

6-Fluor-3-methyl-1,1-[3-(dimethylamino-(3-fluorphenyl)-methyl)-pentamethylen]-1,3,4,9-tetrahydropyrano[3,4-b]indol ($R^3$ = 3-Fluorphenyl) **52**

Keton **12** (249 mg, 1 mmol) und das Tryptophol-Derivat 50 (265 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde

die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 80 mg (19 %), poröser Feststoff

$^{13}C$-NMR ($CDCl_3$): 21,74; 21,94; 24,31; 24,52; 25,61; 25,83; 27,01; 29,85; 30,28; 33,36; 33,83; 37,70; 37,91; 38,09; 41,68; 41,71; 42,33; 65,27; 72,57; 74,34; 103,06; 103,29; 107,84; 107,87; 109,20; 109,46; 111,07; 111,16; 114,23; 114,43; 127,30; 127,40; 130,39; 130,46; 130,65; 132,17; 132,50; 141,21; 156,50; 158,82; 160,42; 162,85.

6-Fluor-3-methyl-1,1-[3-(dimethylamino-(phenyl)-methyl)-pentamethylen]-1,3,4,9-tetrahydropyrano[3,4-b]indol ($R^3$ = Phenyl) **53**

Keton **13** (231 mg, 1 mmol) und das Tryptophol-Derivat **50** (265 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 128 mg (31 %), poröser Feststoff

$^{13}C$-NMR ($CDCl_3$): 21,74; 21,96; 22,81; 24,61; 25,61; 25,94; 27,01; 29,87; 30,29; 33,99; 35,30; 35,90; 37,77; 37,90; 41,65; 42,75; 42,87; 65,27; 65,36; 72,60; 72,97; 75,02; 75,28; 102,89; 103,31; 107,84; 108,00; 109,28; 109,54; 111,06; 111,26; 126,74; 127,33; 127,49; 127,78; 129,20; 129,49; 131,92; 132,15; 136,56; 137,79; 140,61; 141,31; 156,50; 158,83.

6-Fluor-3-methyl-1,1-[3-(dimethylamino-(phenylethyl)-methyl)-pentamethylen]-1,3,4,9-tetrahydropyrano[3,4-b]indol ($R^3$ = Phenethyl) **54**

Keton **14** (259 mg, 1,0 mmol) und das Tryptophol-Derivat **50** (265 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (2:1) gereinigt.

Ausbeute: 149 mg (34 %), poröser Feststoff

$^{13}C$-NMR ($CDCl_3$): 21,74; 21,94; 24,31; 24,52; 25,61; 25,83; 27,01; 29,85; 30,28; 33,36; 33,83; 37,70; 37,91; 38,09; 41,68; 41,71; 42,33; 65,27; 72,57; 74,34; 103,06; 103,29; 107,84; 107,87; 109,20; 109,46; 111,07; 111,16; 114,23; 114,43; 127,30; 127,40; 130,39; 130,46; 130,65; 132,17; 132,50; 141,21; 156,50; 158,82; 160,42; 162,85.

6-Fluor-3-methyl-1,1-[3-(dimethylarnino-(4-chlorphenyl)-methyl)-pentamethylen]-1,3,4,9-tetrahydropyrano[3,4-b]indol ($R^3$ = 4-Chlorphenyl) **55**

Keton **16** (265 mg, 1 mmol) und das Tryptophol-Derivat **50** (265 mg, 1 mmol) wurden unter Argon in Methylenchlorid (5 ml) gelöst und mit Methansulfonsäure (106 mg, 1,1 mmol) versetzt. Der Ansatz wurde über Nacht bei RT gerührt, 1 N NaOH (10 ml) und $CH_2Cl_2$ (10 ml) addiert und 30 min nachgerührt. Nach dem Trennen der Phasen wurde die wässrige Phase mit $CH_2Cl_2$ extrahiert, die organischen Phasen vereinigt, getrocknet ($Na_2SO_4$) und i. Vak. eingeengt. Der Rückstand wurde durch Flashchromatographie mit Essigester/Cyclohexan (1:2) gereinigt.

Ausbeute: 93 mg (21 %), poröser Feststoff

$^{13}C$-NMR ($CDCl_3$): 21,61; 24,27; 25,41; 25,61; 26,88; 29,72; 30,15; 33,81; 37,58; 37,78; 41,52; 42,34; 43,44; 65,28; 75,52; 74,34; 103,17; 103,40; 107,95; 108,00; 109,35; 109,61; 111,14; 111,24; 127,39; 127,49; 127,84; 128,08; 130,49; 130,75; 132,23; 132,56; 135,23; 141,26; 156,69; 159,01.

**Automatisierte Synthese**

Es wurden 1 ml einer Lösung des entsprechenden 1,1-[3-(Dimethylamino-phenylmethyl)-pentamethylen]-3,4-dihydro-1H-2,9-diazafluoren-Derivats (0,1 M in DCM, 100 μmol) in einem Mikrowellen-Reaktionsglas vorgelegt und mit 1 ml der entsprechenden Säurechlorid-Lösung (0,2 M in DCM, 200 μmol) und 0,5 ml N-Ethyldiisopropylamin-Lösung (0,4 M, 200 μmol) versetzt. Anschließend wurde das Mikrowellen-Reaktionsglas versiegelt und für 10 min. bei 120°C in der Mikrowelle (Biotage) bestrahlt (Prestirring 10s, Absorptions Level Normal).

Nach erfolgter Reaktion wurde die Reaktionslösung in ein tariertes Gefäß überführt und in der GeneVac bis zur Trockne eingeengt. Die Aufreinigung erfolgte mittels RP-HPLC.

Nach dieser Methode wurden die folgenden Verbindungen synthetisiert. Die Analytik erfolgte über Massenspektrometrie.

| Nr. | Name | m/z |
|---|---|---|
| **56** | 1-{1,1-[3-(Dimethylamino-phenylmethyl)-pentamethylen]-3,4-dihydro-1H-2,9-diazafluoren-2-yl}-3,3-dimethylbutan-1-on | 472,4 [$M^+$ + 1] |
| **57** | 2-[(3,4-Dimethoxyphenyl)acetyl]-{1,1-[3-(dimethylamino-phenylmethyl)-pentamethylen]-3,4-dihydro-1H-2,9-diazafluoren} | 552,4 [$M^+$ + 1] |
| **58** | 1-{1,1-[3-(Dimethylamino-phenylmethyl)-pentamethylen]-3,4-dihydro-1H-2,9-diazafluoren-2-yl}-2-methyl-3-phenylprop-2-en-1-on | 329,4 [$M^+$ + 1] |
| **59** | 2-(Cyclohexylcarbonyl)-{1,1-[3-(dimethylamino-phenylmethyl)-pentamethylen]-3,4-dihydro-1H-2,9-diazafluoren} | 518,4 [$M^+$ + 1] |
| **60** | 2-[(4-Chlorophenoxy)acetyl]-{1,1-[3-(dimethylamino-phenylmethyl)-pentamethylen]-3,4-dihydro-1H-2,9-diazafluoren} | 542,4; 544,4 [$M^+$ + 1] |
| **61** | 1-{1,1-[3-(Dimethylamino-phenylmethyl)-pentamethylen]-3,4-dihydro-1H-2,9-diazafluoren-2-yl}-3-(2-chlorphenyl)prop-2-en-1-on | 531,3; 533,3 [$M^+$ + 1], 266,4, 268,4 |
| **62** | 1-{1,1-[3-(Dimethylamino-(4-fluorphenyl)methyl)-pentamethylen]-3,4-dihydro-1H-2,9-diazafluoren-2-yl}-3,3-dimethylbutan-1-on | 490,4 [$M^+$ + 1] |
| **63** | 2-[(3,4-Dimethoxyphenyl)acetyl]-{1,1-[3-(dimethylamino-(4-fluorophenyl)methyl)-pentamethylen]-3,4-dihydro-1H-2,9-diazafluoren} | 570,4 [$M^+$ + 1] |
| **64** | 1-{1,1-[3-(Dimethylamino-(4-fluorophenyl)methyl)-pentamethylen]-3,4-dihydro-1H-2,9-diazafluoren-2-yl}-2-methyl-3-phenylprop-2-en-1-on | 536,4[$M^+$ + 1] |
| **65** | 2-[(4-Chlorophenoxy)acetyl]-{1,1-[3-(dimethylamino-(4-fluorophenyl) methyl)-pentamethylen]-3,4-dihydro-1H-2,9-diazafluoren} | 560,3; 562,3 [$M^+$ + 1] |
| **66** | 1-{1,1-[3-(Dimethylamino-(3-fluorphenylmethyl)-pentamethylen}-3,4-dihydro-1H-2,9-diazafluoren-2-yl}-3,3-dimethylbutan-1-on | 490,4[$M^+$ + 1] |
| **67** | 2-[(3,4-Dimethoxyphenyl)acetyl]-{1,1-[3-(dimethylamino-(3-fluorophenyl)methyl)-pentamethylenj-3,4-dihydro-1H-2,9-diazafluoren} | 570,4 [$M^+$ + 1] |
| **68** | 1-{1,1-[3-(Dimethylamino-(3-fluorophenyl)methyl)-pentamethylen]-3,4-dihydro-1H-2,9-diazafluoren-2-yl}-2-methyl-3-phenylprop-2-en-1-on | 536,4 [$M^+$ + 1] |
| **69** | 2-(Cyclohexylcarbonyl)-{1,1-[3-(dimethylamino-(3-fluorophenyl) methyl)-pentamethylen]-3,4-dihydro-1H-2,9-diazafluoren} | 502,4 [$M^+$ + 1] |
| **70** | 2-[(4-Chlorophenoxy)acetyl]-{1,1-[3-(dimethylamino-(3-fluorophenyl) methyl)-pentamethylen]-3,4-dihydro-1H-2,9-diazafluoren} | 560,3; 562,3 [$M^+$ + 1] |
| **71** | 2-(1-Benzothien-2-ylcarbonyl)-{1,1-[3-(dimethylamino-(3-fluorophenyl) methyl)-pentamethylen]-3,4-dihydro-1H-2,9-diazafluoren} | 552,3 [$M^+$ + 1] |
| **72** | 1-{1,1-[3-(Dimethylamino-(3-fluorphenyl)methyl)-pentamethylen]-3,4-dihydro-1H-2,9-diazafluoren-2-yl}-3-(2-chlorphenyl)prop-2-en-1-on | 556,3, 558,3 [$M^+$ + 1] |
| **73** | 1-{1,1-[3-(Dimethylamino-(4-chlorphenyl)methyl)-pentamethylen]-3,4-dihydro-1H-2,9-diazafluoren-2-yl}-3,3-dimethylbutan-1-on | 506,4, 508,3 [$M^+$ + 1] |
| **74** | 2-[(3,4-Dimethoxyphenyl)acetyl]-{1,1-[3-(dimethylamino-(4-chlorophenyl)methyl)-pentamethylen]-3,4-dihydro-1H-2,9-diazafluoren} | [586,4, 588,3 $M^+$ + 1] |
| **75** | 1-{1,1-[3-(Dimethylamino-(4-chlorphenyl)methyl)-pentamethylen]-3,4-dihydro-1H-2,9-diazafluoren-2-yl}-2-methyl-3-phenylprop-2-en-1-on | 552,4, 554,2 [$M^+$ + 1] |
| **76** | 2-(Cyclohexylcarbonyl)-{1,1-[3-(dimethylamino-(4-chlorophenyl) methyl)-pentamethylen]-3,4-dihydro-1H-2,9-diazafluoren} | 518,4, 520,4 [$M^+$ + 1] |
| **77** | 2-[(4-Chlorophenoxy)acetyl]-{1,1-[3-(dimethylamino-(4-chlorophenyl) methyl)-pentamethylen]-3,4-dihydro-1H-2,9-diazafluoren} | 576,3, 578,3 [$M^+$ + 1] |

(fortgesetzt)

| Nr. | Name | m/z |
|---|---|---|
| **78** | 2-(1-Benzothien-2-ylcarbonyl)-{1,1-[3-(dimethylamino-(4-chlorophenyl) methyl)-pentamethylen]-3,4-dihydro-1H-2,9-diazafluoren} | 568,3, 570,3[$M^+$ + 1] |
| **79** | 2-Acetyl-{1,1-[3-(dimethylamino-phenylmethyl)-pentamethylen]-3,4-dihydro-1H-2,9-diazafluoren} | 416,3 [$M^+$ + 1] |
| **80** | 1-{1,1-[3-(Dimethylamino-phenylmethyl)-pentamethylen]-3,4-dihydro-1H-2,9-diazafluoren-2-yl}-3-phenylprop-2-en-1-on | 504,3 [$M^+$ + 1] |
| **81** | 2-(Methoxyacetyl)-{1,1-[3-(dimethylamino-phenylmethyl)-pentamethylen]-3,4-dihydro-1H-2,9-diazatluoren} | 446, 3 [$M^+$ + 1] |
| **82** | 2-(2-Thienylacetyl)-{1,1-[3-(dimethylamino-phenylmethyl)-pentamethylen]-3,4-dihydro-1H-2,9-diazafluoren} | 498,3 [$M^+$ + 1] |
| **83** | 2-(Benzyloxyacetyl)-{1,1-[3-(dimethylamino-phenylmethyl)-pentamethylen]-3,4-dihydro-1H-2,9-diazafluoren} | 522,3 [$M^+$ + 1] |
| **84** | Acetyl-{1,1-[3-(dimethylamino-(4-fluorophenyl) methyl)-pentamethylen]-3,4-dihydro-1H-2,9-diazafluoren}, | 434,3 [$M^+$ + 1] |
| **85** | 2-(3-Cyclopentylpropanoyl)-{1,1-[3-(dimethylamino-(4-fluorophenyl) methyl)-pentamethylen]-3,4-dihydro-1H-2,9-diazafluoren} | 516,4 [$M^+$ + 1] |
| **86** | 1-{1,1-[3-(Dimethylamino-(4-fluorphenyl)methyl)-pentamethylen]-3,4-dihydro-1H-2,9-diazafluoren-2-yl}-3-phenylprop-2-en-1-on | 522,4 [$M^+$ + 1] |
| **87** | 2-(2-Thienylacetyl)-{1,1-[3-(dimethylamino-(4-fluorophenyl) methyl)-pentamethylen]-3,4-dihydro-1H-2,9-diazafluoren} | 516,4 [$M^+$ + 1] |
| **88** | 2-(Benzyloxyacetyl)-{1,1-[3-(dimethylamino-(4-fluorophenyl) methyl)-pentamethylen]-3,4-dihydro-1H-2,9-diazafluoren} | 540,3 [$M^+$ + 1] |
| **89** | Acetyl-{1,1-[3-(dimethylamino-(3-fluorophenyl) methyl)-pentamethylen]-3,4-dihydro-1H-2,9-diazafluoren} | 434,3 [$M^+$ + 1] |
| **90** | 2-(3-Cyclopentylpropanoyl)-{1,1-[3-(dimethylamino-(3-fluorophenyl) methyl)-pentamethylen]-3,4-dihydro-1H-2,9-diazafluoren} | 516,3 [$M^+$ + 1] |
| **91** | 2-(Cyclopropylcarbonyl)-{1,1-{3-(dimethylamino-(3-fluorophenyl) methyl)-pentamethylen]-3,4-dihydro-1H-2,9-diazafluoren} | 460,3 [$M^+$ + 1] |
| **92** | 2-(Methoxyacetyl)-{1,1-[3-(dimethylamino-(3-fluorophenyl) methyl)-pentamethylen]-3,4-dihydro-1H-2,9-diazafluoren} | 464,3 [$M^+$ + 1] |
| **93** | 2-(2-Thienylacetyl)-{1,1-[3-(dimethylamino-(3-fluorophenyl) methyl)-pentamethylen]-3,4-dihydro-1H-2,9-diazafluoren} | 516,3 [$M^+$ + 1] |
| **94** | 2-(Benzyloxyacetyl)-{1,1-[3-(dimethylamino-(3-fluorophenyl) methyl)-pentamethylen]-3,4-dihydro-1H-2,9-diazafluoren} | 540,3 [$M^+$ + 1] |
| **95** | 2-(3-Cyclopentylpropanoyl)-{1,1-[3-(dimethylamino-(4-chlorophenyl) methyl)-pentamethylen]-3,4-dihydro-1H-2,9-diazafluoren} | 532,3, 534,4 [$M^+$ +1] |
| **96** | 2-(Cyclopropylcarbonyl)-{1,1-[3-(dimethylamino-(4-chlorophenyl) methyl)-pentamethylen]-3,4-dihydro-1H-2,9-diazafluoren} | 476,3, 478,3 [$M^+$ + 1] |
| **97** | 2-(Methoxyacetyl)-{1,1-[3-(dimethylamino-(4-chlorophenyl) methyl)-pentamethyten]-3,4-dihydro-1H-2,9-diazafluoren} | 480,3, 482,3 [$M^+$ + 1] |
| **98** | 2-(2-Thienylacetyl)-{1,1-[3-(dimethylamino-(4-chlorophenyl) methyl)-pentamethylen]-3,4-dihydro-1H-2,9-diazafluoren} | 532,2, 534,2 [$M^+$ + 1] |
| **99** | 2-(Benzyloxyacetyl)-{1,1-[3-(dimethylamino-(4-chlorophenyl) methyl)-pentamethylen]-3,4-dihydro-1H-2,9-diazafluoren} | 556,3, 558,3 [$M^+$ + 1] |

(fortgesetzt)

| Nr. | Name | m/z |
|---|---|---|
| **100** | 2-(2-Ethylhexanoyl)-{1,1-[3-(dimethylamino-phenylmethyl)-pentamethylen]-3,4-dihydro-1H-2,9-diazafluoren} | 499,7 [$M^+$ + 1] |
| **101** | 2-Isobutyryl-{1,1-[3-(dimethylamino-phenylmethyl)-pentamethylen]-3,4-dihydro-1H-2,9-diazafluoren} | 443,6 [$M^+$ + 1] |
| **102** | 2-Propionyl-{1,1-[3-(dimethylamino-phenylmethyl)-pentamethylen]-3,4-dihydro-1H-2,9-diazafluoren} | 429,6 [$M^+$ + 1] |
| **103** | 2-(1-Benzothien-3-ylcarbonyl)-{1,1-[3-(dimethylamino-phenylmethyl)-pentamethylen]-3,4-dihydro-1H-2,9-diazafluoren} | 533,7 [$M^+$ + 1] |
| **104** | 2-[(4-Fluorophenyl)acetyl]-{1,1-[3-(dimethylamino-phenylmethyl)-pentamethylen]-3,4-dihydro-1H-2,9-diazafluoren} | 509,7 [$M^+$ + 1] |
| **105** | 2-(3-Chloro-1-benzothien-2-ylcarbonyl)-{1,1-[3-(dimethylamino-phenylmethyl)-pentamethylen]-3,4-dihydro-1H-2,9-diazafluoren} | 568,2 [$M^+$ + 1] |
| **106** | 2-(2-Ethylhexanoyl)-{1,1-[3-(dimethylamino-(4-fluorophenyl)methyl) pentamethylen]-3,4-dihydro-1H-2,9-diazafluoren} | 517,7 [$M^+$ + 1] |
| **107** | 2-Isobutyryl-{1,1-[3-(dimethylamino-(4-fluorophenyl) methyl)-pentamethylen]-3,4-dihydro-1H-2,9-diazafluoren} | 461,6 [$M^+$ + 1] |
| **108** | 2-Propionyl-{1,1-[3-(dimethylamino-(4-fluorophenyl) methyl)-pentamethylen]-3,4-dihydro-1H-2,9-diazafluoren} | 447,6 [$M^+$ + 1] |
| **109** | 2-Cyclopentylcarbonyl-{1,1-[3-(dimethylamino-(4-fluorophenyl) methyl)-pentamethylen]-3,4-dihydro-1H-2,9-diazafluoren} | 487,7 [$M^+$ + 1] |
| **110** | 2-[(4-Fluorophenyl)acetyl]-{1,1-[3-(dimethylamino-(4-fluorophenyl) methyl)-pentamethylen]-3,4-dihydro-1H-2,9-diazafluoren} | 527,7 [$M^+$ + 1] |
| **111** | 2-(2-Ethylhexanoyl)-{1,1-[3-(dimethylamino-(3-fluorophenyl) methyl)-pentamethylen]-3,4-dihydro-1H-2,9-diazafluoren} | 517,7 [$M^+$ + 1] |
| **112** | 2-Isobutyryl-{1,1-[3-(dimethylamino-(3-fluorophenyl) methyl)-pentamethylen]-3,4-dihydro-1H-2,9-diazafluoren} | 461,6 [$M^+$ +1] |
| **113** | 2-Propionyl-{1,1-[3-(dimethylamino-(3-fluorophenyl) methyl)-pentamethylen]-3,4-dihydro-1H-2,9-diazafluoren} | 447,6 [$M^+$ + 1] |
| **114** | 2-(1-Benzothien-3-ylcarbonyl)-{1,1-[3-(dimethylamino-(3-fluorophenyl) methyl)-pentamethylen]-3,4-dihydro-1H-2,9-diazafluoren} | 551,7 [$M^+$ + 1] |
| **115** | 2-Cyclopentylcarbonyl-{1,1-[3-(dimethylamino-(3-fluorophenyl) methyl)-pentamethylen]-3,4-dihydro-1H-2,9-diazafluoren} | 487,7 [$M^+$ + 1] |
| **116** | 2-[(4-Fluorophenyl)acetyl]-{1,1-[3-(dimethylamino-(3-fluorophenyl) methyl)-pentamethylen]-3,4-dihydro-1H-2,9-diazafluoren} | 527,7 [$M^+$ + 1] |
| **117** | 2-(3-Chloro-1-benzothien-2-ylcarbonyl)-{1,1-[3-(dimethylamino-(3-fluorophenyl)methyl)-pentamethylen]-3,4-dihydro-1H-2,9-diazafluoren} | 586,2 [$M^+$ + 1], 588,2 |
| **118** | 2-(2-Ethylhexanoyl)-{1,1-[3-(dimethylamino-(4-chlorophenyl) methyl)-pentamethylen]-3,4-dihydro-1H-2,9-diazafluoren} | 534,2 [$M^+$ + 1], 536,2 |
| **119** | 2-Isobutyryl-{1,1-[3-(dimethylamino-(4-chlorophenyl) methyl)-pentamethylen]-3,4-dihydro-1H-2,9-diazafluoren} | 478,1 [$M^+$ + 1], 480,1 |
| **120** | 2-Propionyl-{1,1-[3-(dimethylamino-(4-chlorophenyl) methyl)-pentamethylen]-3,4-dihydro-1H-2,9-diazafluoren} | 464,1 [$M^+$ + 1], 466,1 |
| **121** | 2-(1-Benzothien-3-ylcarbonyl)-{1,1-[3-(dimethylamino-(4-chlorophenyl) methyl)-pentamethylen]-3,4-dihydro-1H-2,9-diazafluoren} | 568,2 [$M^+$ + 1], 570,1 |

(fortgesetzt)

| Nr. | Name | m/z |
|---|---|---|
| **122** | 2-Cyclopentylcarbonyl-{1,1-[3-(dimethylamino-(4-chlorophenyl) methyl)-pentamethylen]-3,4-dihydro-1H-2,9-diazafluoren} | 504,1 [$M^+$ + 1], 506,1 |
| **123** | 2-[(4-Fluorophenyl)acetyl]-{1,1-[3-(dimethylamino-(4-chlorophenyl) methyl)-pentamethylen]-3,4-dihydro-1H-2,9-diazafluoren} | 544,1 [$M^+$ + 1], 546,1 |
| **124** | 2-(3-Chloro-1-benzothien-2-ylcarbonyl)-{1,1-[3-(dimethylamino-(4-chlorophenyl)methyl)-pentamethylen]-3,4-dihydro-1H-2,9-diazafluoren} | 602,6 [$M^+$ + 1], 604,6, 606,6 |
| **125** | 2-Cyclopentylcarbonyl-{1,1-[3-(dimethylamino-phenylmethyl)-pentamethylen]-3,4-dihydro-1H-2,9-diazafluoren} | 469,7 [$M^+$ + 1] |
| **126** | 2-[(3-Methoxyphenyl)acetyl]-{1,1-[3-(dimethylamino-(3-fluorophenyl) methyl)-pentamethylen]-3,4-dihydro-1H-2,9-diazafluoren} | 540,3 [$M^+$ + 1] |
| **127** | 2-(2-Phenoxypropanoyl)-{1,1-[3-(dimethylamino-(3-fluorophenyl) methyl)-pentamethylen]-3,4-dihydro-1H-2,9-diazafluoren} | 540,3 [$M^+$ + 1] |
| **128** | 2-[5-Methylbenzothien-2-ylcarbonyl]-{1,1-[3-(dimethylamino-(3-fluorophenyl)methyl)-pentamethylen]-3,4-dihydro-1H-2,9-diazafluoren} | 566,3 [$M^+$ + 1] |
| **129** | 1-{1,1-[3-(Dimethylamino-(3-fluorphenyl)methyl)-pentamethylen]-3,4-dihydro-1H-2,9-diazafluoren-2-yl}-3-methylbutan-1-on | 476,3 [$M^+$ + 1] |
| **130** | 1-{1,1-[3-(Dimethylamino-(3-fluorphenyl)methyl)-pentamethylen]-3,4-dihydro-1H-2,9-diazafluoren-2-yl}-3-phenylpropan-1-on | 524,3 [$M^+$ + 1] |
| **131** | 2-(3-phenylpropanoyl)-{1,1-[3-(dimethylamino-(4-chlorophenyl) methyl)-pentamethylen]-3,4-dihydro-1H-2,9-diazafluoren} | 506,3 [$M^+$ + 1], 508,3 |
| **132** | 2-[(3-Bromphenyl)acetyl]-{1,1-[3-(dimethylamino-(4-chlorophenyl) methyl)-pentamethylen]-3,4-dihydro-1H-2,9-diazafluoren} | 556,3 [$M^+$ + 1], 558,3 |
| **133** | 2-(2-Phenoxypropanoyl)-{1,1-[3-(dimethylamino-(4-chlorophenyl) methyl)-pentamethylen]-3,4-dihydro-1H-2,9-diazafluoren} | 556,3 [$M^+$ + 1], 558,3 |
| **134** | 1-{1,1-[3-(Dimethylamino-(4-chlorophenyl)methyl)-pentamethylen]-3,4-dihydro-1H-2,9-diazafluoren-2-yl}-3-methylbutan-1-on | 492,3 [$M^+$ + 1], 494,3 |
| **135** | (3-Phenylpropanoyl)-1-{1,1-[3-(dimethylamino-(4-chlorophenyl) methyl)-pentamethylen]-3,4-dihydro-1H-2,9-diazafluoren} | 540,3 [$M^+$ + 1], 542,3 |
| **136** | 2-[(3-Methoxyphenyl)acetyl]-{1,1-[3-(dimethylamino-phenylmethyl)-pentamethylen]-3,4-dihydro-1H-2,9-diazafluoren} | 522,3 [$M^+$ + 1] |
| **137** | 2-(2-Phenoxypropanoyl)-{1,1-[3-(dimethylamino-phenylmethyl)-pentamethylen]-3,4-dihydro-1H-2,9-diazafluoren} | 522,3 [$M^+$ + 1] |
| **138** | 1-{1,1-[3-(Dimethylamino-phenylmethyl)-pentamethylen]-3,4-dihydro-1H-2,9-diazafluoren-2-yl}-3-methylbutan-1-on | 458,3 [$M^+$ + 1] |
| **139** | 1-{1,1-[3-(Dimethylamino-(4-fluorophenyl)methyl)-pentamethylen]-3,4-dihydro-1H-2,9-diazafluoren-2-yl}-2-ethylbutan-1-on | 490,3 [$M^+$ + 1] |
| **140** | 2-[(3-Methoxyphenyl)acetyl]-{1,1-[3-(dimethylamino-(4-fluorophenyl) methyl)-pentamethylen]-3,4-dihydro-1H-2,9-diazafluoren} | 540,3 [$M^+$ + 1] |
| **141** | 2-(2-Phenoxypropanoyl)-{1,1-[3-(dimethylamino-(4-fluorophenyl) methyl)-pentamethylen]-3,4-dihydro-1H-2,9-diazafluoren} | 540,3 [$M^+$ + 1] |
| **142** | 1-{1,1-[3-(Dimethylamino-(4-fluorphenyl)methyl)-pentamethylen]-3,4-dihydro-1H-2,9-diazafluoren-2-yl}-3-phenylpropan-1-on | 524,3 [$M^+$ + 1] |

**Untersuchungen zur Wirksamkeit der erfindungsgemäßen Verbindungen**

**Methode zur Bestimmung der Affinität zum humanen μ-Opiatrezeptor**

Die Rezeptoraffinität zum humanen μ-Opiatrezeptor wird in einem homogenen Ansatz in Mikrotiterplatten bestimmt. Hierzu werden Verdünnungsreihen der zu prüfenden Substanzen mit einer Rezeptormembranpräparation (15 - 40 μg Protein / 250 μl Inkubationsansatz) von CHO-K1-Zellen, welche den humanen μ-Opiatrezeptor exprimieren (RB-HOM-Rezeptormembran-Präparation von Fa PerkinElmer Life Sciences, Zaventem, Belgien) in Gegenwart von 1 nmol/l des radioaktiven Liganden [$^3$H]-Naloxon (NET719, Fa. PerkinElmer Life Sciences, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 μl für 90 Minuten bei Raumtemperatur inkubiert. Als Inkubationspuffer wird 50 mmol/l Tris-HCl supplementiert mit 0,06 % bovinem Serumalbumin verwendet. Zur Bestimmung der unspezifischen Bindung wird zusätzlich 100 μmol/l Naloxon zugegeben. Nach Beendigung der neunzigminütigen Inkubationszeit werden die Mikrotiterplatten für 20 Minuten bei 1000 g abzentrifugiert und die Radioaktivität in einem ß-Counter (Microbeta-Trilux, Fa. PerkinElmer Wallac, Freiburg, Deutschland) vermessen. Es wird die prozentuale Verdrängung des radioaktiven Liganden aus seiner Bindung zum humanen μ-Opiatrezeptor bei einer Konzentration der Prüfsubstanzen von 1 μmol/l bestimmt und als Prozent Hemmung der spezifischen Bindung angegeben. Ausgehend von der prozentualen Verdrängung durch unterschiedliche Konzentrationen der Prüfsubstanzen werden $IC_{50}$ Hemmkonzentrationen berechnet, die eine 50-prozentige Verdrängung des radioaktiven Liganden bewirken. Durch Umrechnung mittels der Cheng-Prusoff-Beziehung werden $K_i$-Werte für die Prüfsubstanzen erhalten.

**Noradrenalin (NA)- und Serotonin(5HT)-Wiederaufnahme-Inhibierung**

Um diese in vitro Studien durchführen zu können, werden Synaptosomen aus Rattenhirnarealen frisch isoliert. Es findet jeweils eine sogenannte "$P_2$"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

**Phenylchinon-Writhing**

Die Untersuchung auf analgetische Wirksamkeit wurde im Phenylchinon-induzierten Writhing an der Maus (modifiziert nach I.C. Hendershot und J. Forsaith (1959) J. Pharmacol. Exp. Ther. 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur 5%ige Cremophor-Lösung erhalten. Alle Substanzen wurden in der Standarddosierung von 10 mg/kg getestet. Die prozentuale Hemmung (%Hemmung) der Writhingreaktion durch eine Substanz wurde nach folgender Formel berechnet:

$$\%\ \text{Hemmung} = 100 - \frac{\text{Writhingreaktionen der behandelten Tiere} * 100}{\text{Writhingreaktionen}}$$

der Kontrolltiere

Alle untersuchten erfindungsgemäßen Verbindungen zeigten eine ausgeprägte analgetische Wirkung.

**Tabelle 1:** Noradrenalin-Wiederaufnahme-Hemmung der Verbindungen **18 - 21; 26 - 28; 35; 38; 53**

| | X | $R^3$ | $R^5$ | $R^8$ | Noradrenalin-Wiederaufnahme, % Hemmung [10 µM] |
|---|---|---|---|---|---|
| **18** | NH | 3-Fluorphenyl | H | H | 58 |
| **19** | NH | Phenyl | H | H | 65 |
| **20** | NH | Phenethyl | H | H | 91 |
| **21** | NH | 2-Thiophen | H | H | 50 |
| **26** | O | 3-Fluorphenyl | H | H | 49 |
| **27** | O | Phenyl | H | H | 64 |
| **28** | O | Phenethyl | H | H | 52 |
| **35** | O | 4-Fluorphenyl | F | H | 50 |
| **38** | O | 4-Chlorphenyl | F | H | 59 |
| **53** | O | Phenyl | F | $CH_3$ | 47 |

**Tabelle 2:** 5HT-Uptake-Inhibierung der Verbindungen **17 - 22; 25 - 30; 35; 37; 38; 40; 42; 51 - 54**

| | X | $R^3$ | $R^5$ | $R^8$ | Serotonin-Wiederaufnahme, % Hemmung [10 µM] |
|---|---|---|---|---|---|
| **17** | NH | 4-Fluorphenyl | H | H | 83 |
| **18** | NH | 3-Fluorphenyl | H | H | 64 |
| **19** | NH | Phenyl | H | H | 91 |
| **20** | NH | Phenethyl | H | H | 84 |
| **21** | NH | 2-Thiophen | H | H | 72 |
| **22** | NH | 4-Chlorphenyl | H | H | 76 |
| **25** | O | 4-Fluorphenyl | H | H | 68 |
| **26** | O | 3-Fluorphenyl | H | H | 59 |
| **27** | O | Phenyl | H | H | 73 |
| **28** | O | Phenethyl | H | H | 57 |
| **29** | O | 2-Thiophen | H | H | 45 |
| **30** | O | 4-Chlorphenyl | H | H | 56 |
| **35** | O | 4-Fluorphenyl | F | H | 55 |
| **37** | O | Phenyl | F | H | 68 |
| **38** | O | 4-Chlorphenyl | F | H | 57 |
| **40** | O | 4-Fluorphenyl | $CH_3$ | $CH_3$ | 47 |
| **42** | O | Phenyl | $CH_3$ | $CH_3$ | 64 |
| **51** | O | 4-Fluorphenyl | F | $CH_3$ | 39 |
| **52** | O | 3-Fluorphenyl | F | $CH_3$ | 68 |
| **53** | O | Phenyl | F | $CH_3$ | 60 |
| **54** | O | Phenethyl | F | $CH_3$ | 55 |

**Tabelle 3:** **μ-Affinität** der Verbindungen **17 - 22; 25 - 30; 35 - 38; 40 - 44; 51 - 55**

| | X | $R^3$ | $R^5$ | $R^8$ | μ-Opioid-Rezeptor [1 μM], %Hemmung | μ-Opioid-Rezeptor, Ki [μM] |
|---|---|---|---|---|---|---|
| **17** | NH | 4-Fluorphenyl | H | H | 46 | n.b. |
| **18** | NH | 3-Fluorphenyl | H | H | 74 | 0,260 |
| **19** | NH | Phenyl | H | H | 81 | 0,180 |
| **20** | NH | Phenethyl | H | H | 97 | 0,034 |
| **21** | NH | 2-Thiophen | H | H | 62 | n.b. |
| **22** | NH | 4-Chlorphenyl | H | H | 33 | n.b. |
| **25** | O | 4-Fluorphenyl | H | H | 86 | 0,350 |
| **26** | O | 3-Fluorphenyl | H | H | 94 | 0,033 |
| **27** | O | Phenyl | H | H | 99 | 0,026 |
| **28** | O | Phenethyl | H | H | 92 | 0,110 |
| **29** | O | 2-Thiophen | H | H | 99 | 0,015 |
| **30** | O | 4-Chlorphenyl | H | H | 84 | 0,170 |
| **35** | O | 4-Fluorphenyl | F | H | 98 | 0,067 |
| **36** | O | 3-Fluorphenyl | F | H | 102 | 0,046 |
| **37** | O | Phenyl | F | H | 74 | 0,037 |
| **38** | O | 4-Chlorphenyl | F | H | 84 | 0,260 |
| **40** | O | 4-Fluorphenyl | $CH_3$ | $CH_3$ | 68 | n.b. |
| **41** | O | 3-Fluorphenyl | $CH_3$ | $CH_3$ | 88 | 0,300 |
| **42** | O | Phenyl | $CH_3$ | $CH_3$ | 101 | 0,029 |
| **43** | O | Phenethyl | $CH_3$ | $CH_3$ | 42 | n.b. |
| **44** | O | 4-Chlorphenyl | $CH_3$ | $CH_3$ | 72 | 0,490 |
| **51** | O | 4-Fluorphenyl | F | $CH_3$ | 46 | n.b. |
| **52** | O | 3-Fluorphenyl | F | $CH_3$ | 53 | n.b. |
| **53** | O | Phenyl | F | $CH_3$ | 97 | 0,082 |
| **54** | O | Phenethyl | F | $CH_3$ | 21 | n.b. |
| **55** | O | 4-Chlorphenyl | F | $CH_3$ | 58 | n.b. |

**Tabelle 4: Phenylchinon-Writhing**

| Beispiels-Nr. | %Hemmung der Writhingreaktion (Dosierung in mg/kg intravenös) |
|---|---|
| **17** | 52 (10) |
| **20** | 45 (10) |
| **27** | 53 (10) |
| **29** | 73 (10) |
| **30** | 45 (10) |
| **41** | 40 (10) |

(fortgesetzt)

| Beispiels-Nr. | %Hemmung der Writhingreaktion (Dosierung in mg/kg intravenös) |
|---|---|
| **44** | 48 (10) |

Affinität zum μ-Opioid-Rezeptor der über automatisierte Synthese hergestellten Verbindungen (Einmalmessungen bei einer Testkonzentration von 1 μM)

| | **μ-Opioid-Rezeptor, [1μmol], % Hemmung** |
|---|---|
| **56** | 36 |
| **57** | 55 |
| **58** | 50 |
| **59** | 29 |
| **60** | 26 |
| **61** | 6 |
| **62** | 12 |
| **63** | 29 |
| **64** | 15 |
| **65** | 17 |
| **66** | 26 |
| **67** | 25 |
| **68** | 19 |
| **69** | 26 |
| **70** | 31 |
| **71** | - |
| **72** | 24 |
| **73** | 24 |
| **74** | 37 |
| **75** | 13 |
| **76** | 15 |
| **77** | 17 |
| **78** | 11 |
| **79** | 4 |
| **80** | 41 |
| **81** | 0 |
| **82** | 31 |
| **83** | 50 |
| **84** | 41 |
| **85** | 50 |
| **86** | 12 |

(fortgesetzt)

| | μ-Opioid-Rezeptor, [1μmol], % Hemmung |
|---|---|
| **87** | - |
| **88** | 11 |
| **89** | 10 |
| **90** | - |
| **91** | 25 |
| **92** | 22 |
| **93** | 19 |
| **94** | 32 |
| **95** | 18 |
| **96** | 18 |
| **97** | 20 |
| **98** | 21 |
| **99** | 35 |
| **100** | 65 |
| **101** | 74 |
| **102** | 55 |
| **103** | 13 |
| **104** | 39 |
| **105** | - |
| **106** | - |
| **107** | - |
| **108** | - |
| **109** | 21 |
| **110** | 14 |
| **111** | 13 |
| **112** | - |
| **113** | 13 |
| **114** | - |
| **115** | - |
| **116** | 20 |
| **117** | - |
| **118** | 35 |
| **119** | 40 |
| **120** | 29 |
| **121** | - |
| **122** | - |
| **123** | 20 |
| **124** | - |

(fortgesetzt)

| | μ-Opioid-Rezeptor, [1μmol], % Hemmung |
|---|---|
| **125** | 29 |
| **126** | 50 |
| **127** | 55 |
| **128** | 16 |
| **129** | 74 |
| **130** | 49 |
| **131** | 51 |
| **132** | 65 |
| **133** | 71 |
| **134** | 47 |
| **135** | 38 |
| **136** | 75 |
| **137** | 68 |
| **138** | 58 |
| **139** | 25 |
| **140** | 47 |
| **141** | 47 |
| **142** | 30 |

**Patentansprüche**

**1.** Spirocyclische Cyclohexan-Derivate der allgemeinen Formel I,

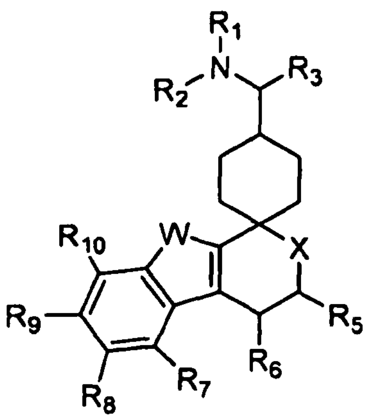

I

worin
$R^1$ und $R^2$ unabhängig voneinander H; $C_{1-5}$-Alkyl, jeweils gesättigt oder ungesättigt, verzweigt oder unverzweigt, bedeutet, wobei $R^1$ und $R^2$ nicht gleichzeitig H bedeuten,
oder die Reste $R^1$ und $R^2$ zusammen $CH_2CH_2OCH_2CH_2$' $CH_2CH_2NR^{11}CH_2CH_2$ oder $(CH_2)_{3-6}$ bedeuten,
wobei $R^{11}$ H; $C_{1-5}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, oder Heteroaryl, jeweils einfach oder mehrfach substituiert oder

unsubstituiert; oder über $C_{1-3}$-Alkyl-Gruppe gebundenes Aryl oder $C_{3-8}$-Cycloalkyl oder Heteroaryl, jeweils unsubstituiert oder einfach oder mehrfach substituiert; bedeutet;
$R^3$ Phenyl oder
Thienyl, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl; einen über eine $C_{1-3}$-Alkylkette gebundenen Phenylrest, jeweils unsubstituiert oder einfach oder mehrfach substituiert mit F, Cl, CN, $NO_2$, SH, S-$C_{1-6}$-Alkyl, OH, O-$C_{1-6}$-Alkyl, $CO_2H$, $CO_2$-$C_{1-6}$-Alkyl, $CF_3$, $C_{1-6}$-Alkyl, bedeutet ;
W für $NR^4$, O oder S steht
und
$R^4$ für H; $C_{1-5}$-Alkyl, gesättigt oder ungesättigt, verzweigt oder unverzweigt, unsubstituiert oder einfach oder mehrfach substituiert; Aryl, oder Heteroaryl, jeweils substituiert oder unsubstituiert; über eine $C_{1-3}$-Alkyl-Gruppe gebundenes Aryl, Heteroaryl oder Cycloalkyl, jeweils einfach oder mehrfach substituiert oder unsubstituiert; $COR^{12}$; $CSR^{12}$; $SO_2R^{12}$ steht,
wobei $R^{12}$ H; $C_{1-8}$-Alkyl, jeweils gesättigt oder ungesättigt, verzweigt oder unverzweigt, einfach oder mehrfach substituiert oder unsubstituiert; $C_{1-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; $(CHR^a)_4OR^{13}$, q = 0, 1 oder 2 und $R^a$ = H, Methyl oder Ethyl; $NR^{14}R^{15}$ bedeutet;
$R^5$ für H; $C_{1-5}$-Alkyl, gesättigt oder ungesättigt, verzweigt oder unverzweigt, unsubstituiert oder einfach oder mehrfach substituiert; $COOR^{13}$, $CONR^{13}$, $OR^{13}$; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;
$R^6$ für H; $OR^{13}$, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Phenyl; oder über $C_{1-3}$-Alkyl gebundenes Aryl, oder Heteroaryl, steht;
$R^7$, $R^8$, $R^9$ und $R^{10}$ unabhängig voneinander für H, F, Cl, Br, $NO_2$, $CF_3$, $OR^{13}$, CN, $COOR^{13}$, $NR^{14}R^{15}$; $C_{1-5}$-Alkyl, Phenyl oder Benzyl, stehen;
wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeutet;
$R^{14}$ und $R^{15}$ unabhängig voneinander H oder $C_{1-5}$-Alkyl bedeuten oder $R^{14}$ und $R^{15}$ zusammen $CH_2CH_2OCH_2CH_2$, $CH_2CH_2NR_{16}CH_2CH_2$ oder $(CH_2)_{3-6}$ bilden,
wobei $R^{16}$ H; $C_{1-5}$-Alkyl, gesättigt oder ungesättigt, bedeutet;
X für O, S, SO, $SO_2$ oder $NR^{17}$ steht;
wobei $R^{17}$ für H; $C_{1-5}$-Alkyl, gesättigt oder ungesättigt, verzweigt oder unverzweigt, jeweils einfach oder mehrfach substituiert oder unsubstituiert; $COR^{12}$ oder $SO_2R^{12}$ bedeutet,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

**2.** Substituierte Cyclohexanderivate gemäß Anspruch 1, worin $R^1$ und $R^2$ unabhängig voneinander H oder $C_{1-6}$-Alkyl bedeuten, wobei $R^1$ und $R^2$ nicht gleichzeitig H bedeuten, oder $R^1$ und $R^2$ zusammen $CH_2CH_2OCH_2CH_2$, oder $(CH_2)_{3-5}$ bedeuten.

**3.** Substituierte Cyclohexanderivate gemäß Anspruch 2, worin $R^1$ und $R^2$ $CH_3$ bedeuten.

**4.** Substituierte Cyclohexanderivate gemäß Anspruch worin $R^3$ Phenyl, unsubstituiert oder einfach substituiert mit Cl oder F, Phenethyl oder Thienyl bedeutet.

**5.** Substituierte Cyclohexanderivate gemäß Anspruch 1, worin $R^5$ H oder $CH_3$ bedeutet.

**6.** Substituierte Cyclohexanderivate gemäß Anspruch 1, worin $R^6$ für H steht.

**7.** Substituierte Cyclohexanderivate gemäß Anspruch 1, worin die Reste $R^8$ und $R^9$ unabhängig voneinander für H; $C_{1-5}$-Alkyl, F, Cl, Br, I, OH, $OCH_3$, COOH, $COOCH_3$, $NH_2$, $NHCH_3$, $N(CH_3)_2$ oder $NO_2$ stehen, während die Reste $R^{10}$ und $R^7$ H sind.

**8.** Substituierte Cyclohexanderivate gemäß Anspruch 1, worin $R^8$ für H, F oder $CH_3$ steht, während die Reste $R^7$, $R^9$ und $R^{10}$ H bedeuten.

**9.** Substituierte Cyclohexanderivate gemäß Anspruch 1, worin $R^{17}$ für H oder $COR^{12}$ steht.

**10.** Substituierte Cyclohexanderivate gemäß Anspruch 1 worin $R^{12}$ für für Phenyl, Phenethyl, Phenethenyl, 1-Methylphenethenyl, oder Benzyl, unsubstituiert oder einfach oder mehrfach substituiert mit F, Cl, Br, I, CN, $NH_2$, NH-$C_{1-6}$-Alkyl, NH- $C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alky), O-$C_{1-6}$Alkyl-OH, C(=O)$C_{1-6}$-Alkyl, $CO_2H$, $CO_2$-$C_{1-6}$-Alkyl, $CF_3$, $C_{1-6}$-Alkyl; Thienyl oder Benzothienyl, gegebenenfalls verbrückt über eine $CH_2$- oder eine $CH_2CH_2$-Kette, unsubstituiert oder einfach oder mehrfach substituiert mit F, Cl, Br, I, CN, $NH_2$, NH-$C_{1-6}$-Alkyl, NH- $C_{1-6}$-Alkyl-OH, N($C_{1-6}$Alkyl)$_2$, N($C_{1-6}$-Alkyl-OH)$_2$, $NO_2$, SH, S-$C_{1-6}$-Alkyl, OH, O-$C_{1-6}$-Alkyl, O-$C_{1-6}$Alkyl-OH, C(=O)$C_{1-6}$-Alkyl, $CO_2H$, $CO_2$-$C_{1-6}$-Alkyl, $CF_3$, $C_{1-6}$-Alkyl; oder Cyclopropyl, Cyclobutyl, Cyclopentyl oder Cyclohexyl, gegebenenfalls verbrückt über eine $CH_2$- oder eine $CH_2CH_2$-Kette, jeweils unsubstituiert oder einfach oder mehrfach substituiert mit F, Cl, Br, -CN, $NH_2$, NH-$C_{1-6}$-Alkyl, NH-$C_{1-8}$-Alkyl-OH, $C_{1-6}$-Alkyl, N($C_{1-6}$-Alkyl)$_2$, N($C_{1-6}$-Alkyl-OH)$_2$, $NO_2$, SH, S-$C_{1-6}$-Alky), S-Benzyl, O-$C_{1-6}$-Alkyl, OH, O-$C_{1-6}$-Alkyl-OH, =O, O-Benzyl, C(=O)$C_{1-6}$-Alkyl, $CO_2H$, $CO_2$-$C_{1-6}$-Alkyl oder Benzyl; $(CHR^a)_qOR^{13}$, q = 1 oder 2 und $R^a$ = H oder Methyl; steht.

**11.** Spirocyclische Cyclohexan-Derivate gemäß Anspruch 1 aus der Gruppe (17) 1,1-[3-(Dimethylamino-(4-fluorophenyl)methyl)-pentamethylen]-3,4-dihydro-1H-2,9-diazafluoren

(18) 1,1-[3-(Dimethylamino-(3-fluorophenyl)methyl)-pentamethylen]-3,4-dihydro-1H-2,9-diazafluoren
(19) 1,1-[3-(Dimethylamino-phenylmethyl)-pentamethylen]-3,4-dihydro-1H-2,9-diazafluoren
(20) 1,1-[3-(Dimethylamino-(phenylethyl)-methyl)-pentamethylen]-3,4-dihydro-1H-2,9-diazafluoren Citrat
(21) 1,1-[3-(Dimethylamino-(thiophen-2-yl)-methyl)-pentamethylen]-3,4-dihydro-1H-2,9-diazafluoren
(22) 1,1-[3-(4-Chlorphenyl-(dimethylamino)-methyl)-pentamethylen]-3,4-dihydro-1H-2,9-diazafluoren
(23) Cyclohexyl{1,1-[3-(Dimethylamino-(4-fluorophenyl)-methyl)-pentamethylen]-3,4-dihydro-1H-2,9-diazafluoren-2-yl}methanon
(24) Phenyl{1,1-[3-(Dimethylamino-(4-fluorophenyl)-methyl)-pentamethylen]-3,4-dihydro-1H-2,9-diazafluoren-2-yl}methanon
(25) 1,1-[3-(Dimethylamino-(4-fluorphenyl)-methyl)-pentamethylen]-1,3,4,9-tetrahydro-pyrano[3,4-b]indol
(26) 1,1-[3-(Dimethylamino-(3-fluorphenyl)-methyl)-pentamethylen]-1,3,4,9-tetrahydro-pyrano[3,4-b]indol
(27) 1,1-[3-(Dimethylamino-(phenyl)-methyl)-pentamethylen]-1,3,4,9-tetrahydropyrano[3,4-b]indol Citrat
(28) 1,1-[3-(Dimethylamino-(phenylethyl)-methyl)-pentamethylen]-1,3,4,9-tetrahydropyrano[3,4-b]indol
(29) 1,1-[3-(Dimethylamino-(thiophen-2-yl)-methyl)-pentamethylen]-1,3,4,9-tetrahydro-pyrano[3,4-b]indol
(30) 1,1-[3-(Dimethylamino-(4-chlorphenyl)-methyl)-pentamethylen]-1,3,4,9-tetrahydro-pyrano[3,4-b]indol
(35) 6-Fluor-1,1-[3-(dimethylamino-(4-fluorphenyl)-methyl)-pentamethylen]-1,3,4,9-tetrahydropyrano[3,4-b]indol
(36) 6-Fluor-1,1-[3-(dimethylamino-(3-fluorphenyl)-methyl)-pentamethylen]-1,3,4,9-tetrahydropyrano[3,4-b]indol
(37) 6-Fluor-1,1-[3-(dimethylamino-(phenyl)-methyl)-pentamethylen]-1,3,4,9-tetrahydro-pyrano[3,4-b]indol
(38) 6-Fluor-1,1-[3-(dimethylamino-(4-chlorphenyl)-methyl)-pentamethylen]-1,3,4,9-tetrahydropyrano[3,4-b]indol
(40) 3,6-Dimethyl-1,1-[3-(dimethylamino-(4-fluorphenyl)-methyl)-pentamethylen]-1,3,4,9-tetrahydropyrano [3,4-b]indol
(41) 3,6-Dimethyl-1,1-[3-(dimethylamino-(3-fluorphenyl)-methyl)-pentamethylen]-1,3,4,9-tetrahydropyrano [3,4-b]indol
(42) 3,6-Dimethyl-1,1-[3-(dimethylamino-(phenyl)-methyl)-pentamethylen]-1,3,4,9-tetrahydropyrano[3,4-b]indol Citrat
(43) 3,6-Dimethyl-1,1-[3-(dimethylamino-(phenylethyl)-methyl)-pentamethylen]-1,3,4,9-tetrahydropyrano[3,4-b]indol
(44) 3,6-Dimethyl-1,1-[3-(dimethylamino-(4-chlorphenyl)-methyl)-pentamethylen]-1,3,4,9-tetrahydropyrano [3,4-b]indol
(51) 6-Fluor-3-methyl-1,1-[3-(dimethylamino-(4-fluorphenyl)-methyl)-pentamethylen]-1,3,4,9-tetrahydropyrano [3,4-b]indol
(52) 6-Fluor-3-methyl-1,1-[3-(dimethylamino-(3-fluorphenyl)-methyl)-pentamethylen]-1,3,4,9-tetrahydropyrano [3,4-b]indol
(53) 6-Fluor-3-methyl-1,1-[3-(dimethylamino-(phenyl)-methyl)-pentamethylen]-1,3,4,9-tetrahydropyrano[3,4-b]indol

(54) 6-Fluor-3-methyl-1,1-[3-(dimethylamino-(phenylethyl)-methyl)-pentamethylen]-1,3,4,9-tetrahydropyrano [3,4-b]indol

(55) 6-Fluor-3-methyl-1,1-[3-(dimethylamino-(4-chlorphenyl)-methyl)-pentamethylen]-1,3,4,9-tetrahydropyrano[3,4-b]indol

(56) 1-{1,1-[3-(Dimethylamino-phenylmethyl)-pentamethylen]-3,4-dihydro-1H-2,9-diazafluoren-2-yl}-3,3-dimethylbutan-1-on

(57) 2-[(3,4-Dimethoxyphenyl)acetyl]-{1,1-[3-(dimethylamino-phenylmethyl)-pentamethylen]-3,4-dihydro-1H-2,9-diazafluoren}

(58) 1-{1,1-[3-(Dimethylamino-phenylmethyl)-pentamethylen]-3,4-dihydro-1H-2,9-diazafluoren-2-yl}-2-methyl-3-phenylprop-2-en-1-on

(59) 2-(Cyclohexylcarbonyl)-{1,1-[3-(dimethylamino-phenylmethyl)-pentamethylen]-3,4-dihydro-1H-2,9-diazafluoren}

(60) 2-[(4-Chlorophenoxy)acetyl]-{1,1-[3-(dimethylamino-phenylmethyl)-pentamethylen]-3,4-dihydro-1H-2,9-diazafluoren}

(61) 1-{1,1-[3-(Dimethylamino-phenylmethyl)-pentamethylen]-3,4-dihydro-1H-2,9-diazafluoren-2-yl}-3-(2-chlorphenyl)prop-Z-en-1-on

(62) 1-{1,1-[3-(Dimethylamino-(4-fluorphenyl)methyl)-pentamethylen]-3,4-dihydro-1H-2,9-diazafluoren-2-yl}-3,3-dimethylbutan-1-on

(63) 2-[(3,4-Dimethoxyphenyl)acetyl]-{1,1-[3-(dimethylamino-(4-fluorophenyl)methyl)-pentamethylen]-3,4-dihydro-1H-2,9-diazafluoren}

(64) 1-{1,1-[3-(Dimethylamino-(4-fluorophenyl)methyl)-pentamethylen]-3,4-dihydro-1H-2,9-diazafluoren-2-yl}-2-methyl-3-phenylprop-2-en-1-on

(65) 2-[(4-Chlorophenoxy)acetyl]-{1,1-[3-(dimethylamino-(4-fluorophenyl)methyl)-pentamethylen]-3,4-dihydro-1H-2,9-diazafluoren}

(66) 1-{1,1-[3-(Dimethylamino-(3-fluorphenyl)methyl)-pentamethylen]-3,4-dihydro-1H-2,9-diazafluoren-2-yl}-3,3-dimethylbutan-1-on

(67) 2-[(3,4-Dimethoxyphenyl)acetyl]-{1,1-[3-(dimethylamino-(3-fluorophenyl)methyl)-pentamethylen]-3,4-dihydro-1H-2,9-diazafluoren}

(68) 1-{1,1-[3-(Dimethylamino-(3-fluorophenyl)methyl)-pentamethylen]-3,4-dihydro-1H-2,9-diazafluoren-2-yl}-2-methyl-3-phenylprop-2-en-1-on

(69) 2-(Cyclohexylcarbonyl)-{1,1-[3-(dimethylamino-(3-fluorophenyl)methyl)-pentamethylen]-3,4-dihydro-1H-2,9-diazafluoren}

(70) 2-[(4-Chlorophenoxy)acetyl]-{1,1-[3-(dimethylamino-(3-fluorophenyl)methyl)-pentamethylen]-3,4-dihydro-1H-2,9-diazafluoren}

(71) 2-(1-Benzothien-2-ylcarbonyl)-{1,1-[3-(dimethylamino-(3-fluorophenyl)methyl)-pentamethylen]-3,4-dihydro-1H-2,9-diazafluoren}

(72) 1-{1,1-[3-(Dimethylamino-(3-fluorphenyl)methyl)-pentamethylen]-3,4-dihydro-1H-2,9-diazafluoren-2-yl}-3-(2-chlorphenyl)prop-2-en-1-on

(73) 1-{1,1-[3-(Dimethylamino-(4-chlorphenyl)methyl)-pentamethylen]-3,4-dihydro-1H-2.9-diazafluoren-2-yl}-3,3-dimethylbutan-1-on

(74) 2-[(3,4-Dimethoxyphenyl)acetyl]-{1,1-[3-(dimethylamino-(4-chlorophenyl)methyl)-pentamethylen]-3,4-dihydro-1H-2,9-diazafluoren}

(75) 1-{1,1-[3-(Dimethylamino-(4-chlorphenyl)methyl)-pentamethylen]-3,4-dihydro-1H-2,9-diazafluoren-2-yl}-2-methyl-3-phenylprop-2-en-1-on

(76) 2-(Cyclohexylcarbonyl)-{1,1-[3-(dimethylamino-(4-chlorophenyl)methyl)-pentamethylen]-3,4-dihydro-1H-2,9-diazafluoren}

(77) 2-[(4-Chlorophenoxy)acetyl]-{1,1-[3-(dimethylamino-(4-chlorophenyl)methyl)-pentamethylen]-3,4-dihydro-1H-2,9-diazafluoren}

(78) 2-(1-Benzothien-2-ylcarbonyl)-{1,1-[3-(dimethylamino-(4-chlorophenyl)methyl)-pentamethylen]-3,4-dihydro-1H-2,9-diazafluoren}

(79) 2-Acetyl-{1,1-[3-(dimethylamino-phenylmethyl)-pentamethylen]-3,4-dihydro-1H-2,9-diazafluoren}

(80) 1-{1,1-[3-(Dimethylamino-phenylmethyl)-pentamethylen]-3,4-dihydro-1H-2,9-diazafluoren-2-yl}-3-phenylprop-2-en-1-on

(81) 2-(Methoxyacetyl)-{1,1-[3-(dimethylamino-phenylmethyl)-pentamethylen]-3,4-dihydro-1H-2,9-diazafluoren}

(82) 2-(2-Thienylacetyl)-{1,1-[3-(dimethylamino-phenylmethyl)-pentamethylen]-3,4-dihydro-1H-2,9-diazafluoren}

(83) 2-(Benzyloxyacetyl)-{1,1-[3-(dimethylamino-phenylmethyl)-pentamethylen]-3,4-dihydro-1H-2,9-diazafluoren}

(84) Acetyl-{1,1-[3-(dimethylamino-(4-fluorophenyl)methyl)-pentamethylen]-3,4-dihydro-1H-2,9-diazafluoren}

(85) 2-(3-Cyclopentylpropanoyl)-{1,1-[3-(dimethylamino-(4-fluorophenyl)methyl)-pentamethylen]-3,4-dihydro-1H-2,9-diazafluoren}

(86) 1-{1,1-[3-(Dimethylamino-(4-fluorphenyl)methyl)-pentamethylen]-3,4-dihydro-1H-2,9-diazafluoren-2-yl}-3-phenylprop-2-en-1-on

(87) 2-(2-Thienylacetyl)-{1,1-[3-(dimethylamino-(4-fluorophenyl)methyl)-pentamethylen]-3,4-dihydro-1H-2,9-diazafluoren}

(88) 2-(Benzyloxyacetyl)-{1,1-[3-(dimethylamino-(4-fluorophenyl)methyl)-pentamethylen]-3,4-dihydro-1H-2,9-diazafluoren}

(89) Acetyl-{1,1-[3-(dimethylamino-(3-fluorophenyl)methyl)-pentamethylen]-3,4-dihydro-1H-2,9-diazafluoren}

(90) 2-(3-Cyclopentylpropanoyl)-{1,1-[3-(dimethylamino-(3-fluorophenyl)methyl)-pentamethylen]-3,4-dihydro-1H-2,9-diazafluoren}

(91) 2-(Cyclopropylcarbonyl)-{1,1-[3-(dimethylamino-(3-fluorophenyl)methyl)-pentamethylen]-3,4-dihydro-1H-2,9-diazafluoren}

(92) 2-(Methoxyacetyl)-{1,1-[3-(dimethylamino-(3-fluorophenyl)methyl)-pentamethylen]-3,4-dihydro-1H-2,9-diazafluoren}

(93) 2-(2-Thienylacetyl)-{1,1-[3-(dimethylamino-(3-fluorophenyl)methyl)-pentamethylen]-3,4-dihydro-1H-2,9-diazafluoren}

(94) 2-(Benzyloxyacetyl)-{1,1-[3-(dimethylamino-(3-fluorophenyl)methyl)-pentamethylen]-3,4-dihydro-1H-2,9-diazafluoren}

(95) 2-(3-Cyclopentylpropanoyl)-{1,1-[3-(dimethylamino-(4-chlorophenyl)methyl)-pentamethylen]-3,4-dihydro-1H-2,9-diazafluoren}

(96) 2-(Cyclopropylcarbonyl)-{1,1-[3-(dimethylamino-(4-chlorophenyl)methyl)-pentamethylen]-3,4-dihydro-1H-2,9-diazafluoren}

(97) 2-(Methoxyacetyl)-{1,1-[3-(dimethylamino-(4-chlorophenyl)methyl)-pentamethylen]-3,4-dihydro-1H-2,9-diazafluoren}

(98) 2-(2-Thienylacetyl)-{1,1-[3-(dimethylamino-(4-chlorophenyl)methyl)-pentamethylen]-3,4-dihydro-1H-2,9-diazafluoren}

(99) 2-(Benzyloxyacetyl)-{1,1-[3-(dimethylamino-(4-chlorophenyl)methyl)-pentamethylen]-3,4-dihydro-1H-2,9-diazafluoren}

(100) 2-(2-Ethylhexanoyl)-{1,1-[3-(dimethylamino-phenylmethyl)-pentamethylen]-3,4-dihydro-1H-2,9-diazafluoren}

(101) 2-Isobutyryl-{1,1-[3-(dimethylamino-phenylmethyl)-pentamethylen]-3,4-dihydro-1H-2,9-diazafluoren}

(102) 2-Propionyl-{1,1-[3-(dimethylamino-phenylmethyl)-pentamethylen]-3,4-dihydro-1H-2,9-diazafluoren}

(103) 2-(1-Benzothien-3-ylcarbonyl)-{1,1-[3-(dimethylamino-phenylmethyl)-pentamethylen]-3,4-dihydro-1H-2,9-diazafluoren}

(104) 2-[(4-Fluorophenyl)acetyl]-{1,1-[3-(dimethylamino-phenylmethyl)-pentamethylen]-3,4-dihydro-1H-2,9-diazafluoren}

(105) 2-(3-Chloro-1-benzothien-2-ylcarbonyl)-{1,1-[3-(dimethylamino-phenylmethyl)-pentamethylen]-3,4-dihydro-1H-2,9-diazafluoren}

(106) 2-(2-Ethylhexanoyl)-{1,1-[3-(dimethylamino-(4-fluorophenyl)methyl)-pentamethylen]-3,4-dihydro-1H-2,9-diazafluoren}

(107) 2-Isobutyryl-{1,1-[3-(dimethylamino-(4-fluorophenyl)methyl)-pentamethylen]-3,4-dihydro-1H-2,9-diazafluoren}

(108) 2-Propionyl-{1,1-[3-(dimethylamino-(4-fluorophenyl)methyl)-pentamethylen]-3,4-dihydro-1H-2,9-diazafluoren}

(109) 2-Cyclopentylcarbonyl-{1,1-[3-(dimethylamino-(4-fluorophenyl)methyl)-pentamethylen]-3,4-dihydro-1H-2,9-diazafluoren}

(110) 2-[(4-Fluorophenyl)acetyl]-{1,1-[3-(dimethylamino-(4-fluorophenyl)methyl)-pentamethylen]-3,4-dihydro-1H-2,9-diazafluoren}

(111) 2-(2-Ethylhexanoyl)-{1,1-[3-(dimethylamino-(3-fluorophenyl)methyl)-pentamethylen]-3,4-dihydro-1H-2,9-diazafluoren}

(112) 2-Isobutyryl-{1,1-[3-(dimethylamino-(3-fluorophenyl)methyl)-pentamethylen]-3,4-dihydro-1H-2,9-diazafluoren}

(113) 2-Propionyl-{1,1-[3-(dimethylamino-(3-fluorophenyl)methyl)-pentamethylen]-3,4-dihydro-1H-2,9-diazafluoren}

(114) 2-(1-Benzothien-3-ylcarbonyl)-{1,1-[3-(dimethylamino-(3-fluorophenyl)methyl)-pentamethylen]-3,4-dihydro-1H-2,9-diazafluoren}

(115) 2-Cyclopentylcarbonyl-{1,1-[3-(dimethylamino-(3-fluorophenyl)methyl)-pentamethylen]-3,4-dihydro-1H-2,9-diazafluoren}

(116) 2-[(4-Fluorophenyl)acetyl]-{1,1-[3-(dimethylamino-(3-fluorophenyl)methyl)-pentamethylen]-3,4-dihydro-1H-2,9-diazafluoren}

(117) 2-(3-Chloro-l-benzothien-2-ylcarbonyl)-{1,1-[3-(dimethylamino-(3-fluorophenyl)methyl)-pentamethylen]-3,4-dihydro- H-2,9-diazafluoren}

(118) 2-(2-Ethylhexanoyl)-{1,1-[3-(dimethylamino-(4-chlorophenyl)methyl)-pentamethylen]-3,4-dihydro-1H-2,9-diazafluoren}

(119) 2-Isobutyryl-{1,1-[3-(dimethylamino-(4-chlorophenyl)methyl)-pentamethylen]-3,4-dihydro-1H-2,9-diazafluoren}

(120) 2-Propionyl-{1,1-[3-(dimethylamino-(4-chlorophenyl)methyl)-pentamethylen]-3,4. dihydro-1H-2,9-diazafluoren}

(121) 2-(1-Benzothien-3-ylcarbonyl)-{1,1-[3-(dimethylamino-(4-chlorophenyl)methyl)-pentamethylen]-3,4-dihydro-1H-2,9-diazafluoren}

(122) 2-Cyclopentylcarbonyl-{1,1-[3-(dimethylamino-(4-chlorophenyl)methyl)-pentamethylen]-3,4-dihydro-1H-2,9-diazafluoren}

(123) 2-[(4-Fluorophenyl)acetyl]-{1,1-[3-(dimethylamino-(4-chlorophenyl)methyl)-pentamethylen]-3,4-dihydro-1H-2,9-diazafluoren}

(124) 2-(3-Chloro-1-benzothien-2-ylcarbonyl)-{1,1-[3-(dimethylamino-(4-chlorophenyl)methyl)-pentamethylen]-3,4-dihydro-1H-2,9-diazafluoren}

(125) 2-Cyclopentylcarbonyl-{1,1-[3-(dimethylamino-phenylmethyl)-pentamethylen]-3,4-dihydro-1H-2,9-diazafluoren}

(126) 2-[(3-Methoxyphenyl)acetyl]-{1,1-[3-(dimethylamino-(3-fluorophenyl)methyl)-pentamethylen]-3,4-dihydro-1H-2,9-diazafluoren}

(127) 2-(2-Phenoxypropanoyl)-{1,1-[3-(dimethylamino-(3-fluorophenyl)methyl)-pentamethylen]-3,4-dihydro-1H-2,9-diazafluoren}

(128) 2-[5-Methylbenzothien-2-ylcarbonyl]-{1,1-[3-(dimethylamino-(3-fluorophenyl)methyl)-pentamethylen]-3,4-dihydro-1H-2,9-diazafluoren}

(129) 1-{1,1-[3-(Dimethylamino-(3-fluorphenyl)methyl)-pentamethylen]-3,4-dihydro-1H-2,9-diazafluoren-2-yl}-3-methylbutan-1-on

(130) 1-{1,1-[3-(Dimethylamino-(3-fluorphenyl)methyl)-pentamethylen]-3,4-dihydro-1H-2,9-diazafluoren-2-yl}-3-phenylpropan-1-on

(131) 2-(3-phenylpropanoyl)-{1,1-[3-(dimethylamino-(4-chlorophenyl)methyl)-pentamethylen]-3,4-dihydro-1H-2,9-diazafluoren}

(132) 2-[(3-Bromphenyl)acetyl]-{1,1-[3-(dimethylamino-(4-chlorophenyl)methyl)-pentamethylen]-3,4-dihydro-1H-2,9-diazafluoren}

(133) 2-(2-Phenoxypropanoyl)-{1,1-[3-(dimethylamino-(4-chlorophenyl)methyl)-pentamethylen]-3,4-dihydro-1H-2,9-diazafluoren}

(134) 1-{1,1-[3-(Dimethylamino-(4-chlorophenyl)methyl)-pentamethylen]-3,4-dihydro-1H-2,9-diazafluoren-2-yl}-3-methylbutan-1-on

(135) (3-Phenylpropanoyl)-1-{1,1-[3-(dimethylamino-(4-chlorophenyl)methyl)-pentamethylen]-3,4-dihydro-1H-2,9-diazafluoren}

(136) 2-[(3-Methoxyphenyl)acetyl]-{1,1-[3-(dimethylamino-phenylmethyl)-pentamethylen]-3,4-dihydro-1H-2,9-diazafluoren}

(137) 2-(2-Phenoxypropanoyl)-{1,1-[3-(dimethylamino-phenylmethyl)-pentamethylen]-3,4-dihydro-1H-2,9-diazafluoren}

(138) 1-{1,1-[3-(Dimethylamino-phenylmethyl)-pentamethylen]-3,4-dihydro-1H-2,9-diazafluoren-2-yl}-3-methylbutan-1-on

(139) 1-{1,1-[3-(Dimethylamino-(4-fluorophenyl)methyl)-pentamethylen]-3,4-dihydro-1H-2,9-diazafluoren-2-yl}-2-ethylbutan-1-on

(140) 2-[(3-Methoxyphenyl)acetyl]-{1,1-[3-(dimethylamino-(4-fluorophenyl)methyl)-pentamethylen]-3,4-dihydro-1H-2,9-diazafluoren}

(141) 2-(2-Phenoxypropanoyl)-{1,1-[3-(dimethylamino-(4-fluorophenyl)methyl)-pentamethylen]-3,4-dihydro-1H-2,9-diazafluoren}

(142) 1-{1,1-[3-(Dimethylamino-(4-fluorphenyl)methyl)-pentamethylen]-3,4-dihydro-1H-2,9-diazafluoren-2-yl}-3-phenylpropan-1-on

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

**12.** Verfahren zur Herstellung eines spirocyclischen Cyclohexan-Derivats gemäß Formel I

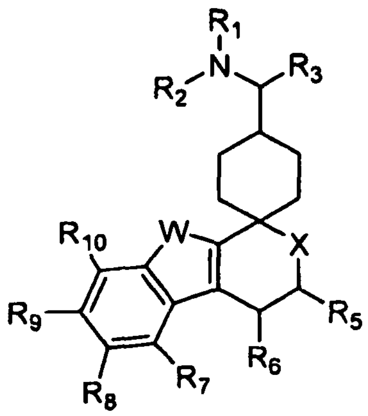

worin die Reste $R^1$-$R^{10}$ sowie W und X die in Anspruch 1 angegebene Bedeutung haben, wobei ein Edukt der allgemeinen Formel A

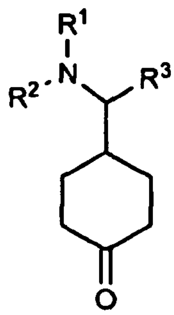

**A**

unter Zugabe von Säure, oder deren Trimethylsilylester, beispielsweise Trifluormethansulfonsäuretrimethylsilylester, Trifluormethansulfonsäure, Essigsäure, Phosphorsäure, Methansulfonsäure oder Trifluoressigsäure, in einem geeigneten Lösungsmittel, beispielsweise Dichlorethan, Dichlormethan, Chloroform, Acetonitril, Diethylether oder Nitromethan, mit einem Edukt der allgemeinen Formel B

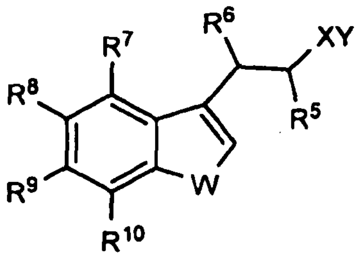

**B**

umgesetzt wird, wobei die Reste $R^1$-$R^{10}$ sowie W die in Anspruch 1 angegebenen Bedeutungen haben und Y H oder Trimethylsilyl bedeutet.

**13.** Verfahren zur Herstellung von spirocyclischen Cyclohexanderivaten gemäß Formel I

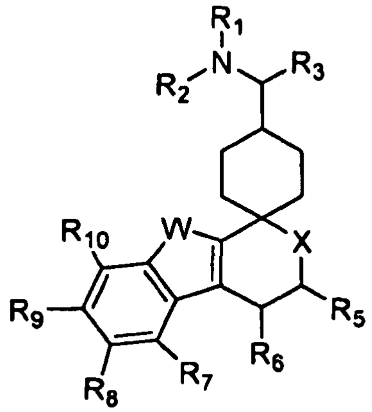

worin die Reste $R^1$-$R^{10}$ sowie W die in Anspruch 1 angegebene Bedeutung haben, X $NR^{17}$ und $R^{17}$ $COR^{12}$ oder $SO_2R^{12}$ bedeutet, wobei ein spirocyclisches Cyclohexanderivat, bei dem X NH bedeutet, unter Zugabe von Base, beispielsweise Triethylamin, mit einem Anhydrid oder einem Säurechlorid umgesetzt wird, vorzugsweise unter Mikrowelleneinstrahlung.

**14.** Arzneimittel enthaltend wenigstens ein spirocyclisches Cyclohexan-Derivat gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, sowie gegebenenfalls enthaltend geeignete Zusatz- und/oder Hilfsstoffe und/oder gegebenenfalls weiterer Wirkstoffe.

**15.** Verwendung eines spirocyclischen Cyclohexan-Derivats gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Schmerz, insbesondere von akutem, neuropathischem oder chronischem Schmerz.

**16.** Verwendung eines spirocyclischen Cyclohexan-Derivats gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Depressionen, Harninkontinenz, Diarrhöe, Pruritus, Alkohol- und Drogenmißbrauch, Medikamentenabhängigkeit, Antriebslosigkeit und/oder zur Anxiolyse.

## Claims

1. Spirocyclic cyclohexane derivatives of the general formula I

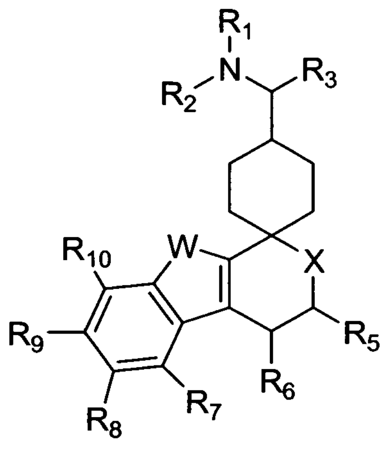

I

wherein
$R^1$ and $R^2$ independently of one another denote H; $C_{1-5}$-alkyl, in each case saturated or unsaturated, branched or unbranched, wherein $R^1$ and $R^2$ do not simultaneously denote H,
or the radicals $R^1$ and $R^2$ together denote $CH_2CH_2OCH_2CH_2$, $CH_2CH_2NR^{11}CH_2CH_2$ or $(CH_2)_{3-6}$,
wherein $R^{11}$ denotes H; $C_{1-5}$-alkyl or $C_{3-8}$-cycloalkyl, in each case saturated or unsaturated, branched or unbranched, mono- or polysubstituted or unsubstituted; aryl or heteroaryl, in each case mono- or polysubstituted or unsubstituted; or aryl or $C_{3-8}$-cycloalkyl or heteroaryl, bonded via a $C_{1-3}$-alkyl group and in each case unsubstituted or mono- or polysubstituted;
$R^3$ denotes phenyl or
thienyl, in each case unsubstituted or mono- or polysubstituted by F, Cl, CN, $NO_2$, SH, S-$C_{1-6}$-alkyl, OH, O-$C_{1-6}$-alkyl, $CO_2H$, $CO_2$-$C_{1-6}$-alkyl, $CF_3$, $C_{1-6}$-Alkyl; a phenyl radical bonded via a $C_{1-3}$-alkyl chain and in each case unsubstituted or mono- or polysubstituted by F, Cl, CN, $NO_2$, SH, S-$C_{1-6}$-alkyl, OH, O-$C_{1-6}$-alkyl, $CO_2H$, $CO_2$-$C_{1-6}$-alkyl, $CF_3$, $C_{1-6}$-alkyl;
W represents $NR^4$, O or S
and
$R^4$ represents H; $C_{1-5}$-alkyl, saturated or unsaturated, branched or unbranched, unsubstituted or mono- or polysubstituted; aryl or heteroaryl, in each case substituted or unsubstituted; or aryl, heteroaryl or cycloalkyl, bonded via a $C_{1-3}$-alkyl group and in each case mono- or polysubstituted or unsubstituted; $COR^{12}$; $CSR^{12}$; $SO_2R^{12}$,
wherein $R^{12}$ denotes H; $C_{1-8}$-alkyl, in each case saturated or unsaturated, branched or unbranched, mono- or polysubstituted or unsubstituted; $C_{3-8}$-cycloalkyl, in each case saturated or unsaturated, mono- or polysubstituted or unsubstituted; aryl or heteroaryl, in each case mono- or polysubstituted or unsubstituted; or aryl, $C_{3-8}$-cycloalkyl or heteroaryl, bonded via $C_{1-3}$-alkyl and in each case mono- or polysubstituted or unsubstituted; $(CHR^a)_qOR^{13}$, q = 0, 1 or 2 and $R^a$ = H, methyl or ethyl; $NR^{14}R^{15}$;
$R^5$ represents H; $C_{1-5}$-alkyl, saturated or unsaturated, branched or unbranched, unsubstituted or mono- or polysubstituted; $COOR^{13}$, $CONR^{13}$, $OR^{13}$; $C_{3-8}$-cycloalkyl, saturated or unsaturated, unsubstituted or mono- or polysubstituted; aryl or heteroaryl, unsubstituted or mono- or polysubstituted; or aryl, $C_{3-8}$-cycloalkyl or heteroaryl, bonded via $C_{1-3}$-alkyl and unsubstituted or mono- or polysubstituted;
$R^6$ represents H; $OR^{13}$, $COOR^{13}$, $NR^{14}R^{15}$; $C_{1-5}$-alkyl, saturated or unsaturated, branched or unbranched, unsubstituted or mono- or polysubstituted; $C_{3-8}$-cycloalkyl, saturated or unsaturated, unsubstituted or mono- or polysubstituted; phenyl; or aryl or heteroaryl, bonded via $C_{1-3}$-alkyl;
$R^7$, $R^8$, $R^9$ and $R^{10}$ independently of one another represent H, F, Cl, Br, $NO_2$, $CF_3$, $OR^{13}$, CN, $COOR^{13}$ , $NR^{14}R^{15}$; $C_{1-5}$-alkyl, phenyl or benzyl;
wherein $R^{13}$ denotes H; $C_{1-5}$-alkyl, in each case saturated or unsaturated, branched or unbranched, unsubstituted or mono- or polysubstituted; $C_{3-8}$-cycloalkyl, in each case saturated or unsaturated, unsubstituted or mono- or polysubstituted; aryl or heteroaryl, unsubstituted or mono- or polysubstituted; or aryl, $C_{3-8}$-cycloalkyl or heteroaryl, bonded via $C_{1-3}$-alkyl and unsubstituted or mono- or polysubstituted;

$R^{14}$ and $R^{15}$ independently of one another denote H or $C_{1-5}$-alkyl or $R^{14}$ and $R^{15}$ together form $CH_2CH_2OCH_2CH_2$, $CH_2CH_2NR^{16}CH_2CH_2$ or $(CH_2)_{3-6}$,
wherein $R^{16}$ denotes H; $C_{1-5}$-alkyl, saturated or unsaturated;
X represents O, S, SO, $SO_2$ or $NR^{17}$;
wherein $R^{17}$ represents H; $C_{1-5}$-alkyl, saturated or unsaturated, branched or unbranched, in each case mono- or polysubstituted or unsubstituted; $COR^{12}$ or $SO_2R^{12}$,
in the form of the racemate; of the enantiomers, diastereomers, mixtures of the enantiomers or diastereomers or of an individual enantiomer or diastereomer; of the bases and/or salts of physiologically acceptable acids.

**2.** Substituted cyclohexane derivatives according to claim 1, wherein $R^1$ and $R^2$ independently of one another denote H or $C_{1-6}$-alkyl, wherein $R^1$ and $R^2$ do not simultaneously denote H, or $R^1$ and $R^2$ together denote $CH_2CH_2OCH_2CH_2$, or $(CH_2)_{3-5}$.

**3.** Substituted cyclohexane derivatives according to claim 2, wherein $R^1$ and $R^2$ denote $CH_3$.

**4.** Substituted cyclohexane derivatives according to claim ..., wherein $R^3$ denotes phenyl, unsubstituted or monosubstituted by Cl or F, phenethyl or thienyl.

**5.** Substituted cyclohexane derivatives according to claim 1, wherein $R^5$ denotes H or $CH_3$.

**6.** Substituted cyclohexane derivatives according to claim 1, wherein $R^6$ represents H.

**7.** Substituted cyclohexane derivatives according to claim 1, wherein the radicals $R^8$ and $R^9$ independently of one another represent H; $C_{1-5}$-alkyl, F, Cl, Br, I, OH, $OCH_3$, COOH, $COOCH_3$, $NH_2$, $NHCH_3$ , $N(CH_3)_2$ or $NO_2$, while the radicals $R^{10}$ and $R^7$ are H.

**8.** Substituted cyclohexane derivatives according to claim 1, wherein $R^8$ represents H, F or $CH_3$, while the radicals $R^7$, $R^9$ and $R^{10}$ denote H.

**9.** Substituted cyclohexane derivatives according to claim 1, wherein $R^{17}$ represents H or $COR^{12}$.

**10.** Substituted cyclohexane derivatives according to claim 1, wherein $R^{12}$ represents phenyl, phenethyl, phenethenyl, 1-methyl-phenethenyl or benzyl, unsubstituted or mono- or polysubstituted by F, Cl, Br, I, CN, $NH_2$, NH-$C_{1-6}$-alkyl, NH-$C_{1-6}$-alkyl-OH, $N(C_{1-6}\text{-alkyl})_2$, $N(C_{1-6}\text{-alkyl-OH})_2$, $NO_2$, SH, S-$C_{1-6}$-alkyl, OH, O-$C_{1-6}$-alkyl, O-$C_{1-6}$-alkyl-OH, C(=O)$C_{1-6}$-alkyl, $CO_2H$, $CO_2$-$C_{1-6}$-alkyl, $CF_3$, $C_{1-6}$-alkyl; thienyl or benzothienyl, optionally bridged via a $CH_2$ or a $CH_2CH_2$ chain, unsubstituted or mono- or polysubstituted by F, Cl, Br, I, CN, $NH_2$, NH-$C_{1-6}$-alkyl, NH-$C_{1-6}$-alkyl-OH, $N(C_{1-6}\text{-alkyl})_2$, $N(C_{1-6}\text{-alkyl-OH})_2$, $NO_2$, SH, S-$C_{1-6}$-alkyl, OH, O-$C_{1-6}$-alkyl, O-$C_{1-6}$-alkyl-OH, C(=O)$C_{1-6}$-alkyl, $CO_2H$, $CO_2$-$C_{1-6}$-alkyl, $CF_3$, $C_{1-6}$-alkyl; or cyclopropyl, cyclobutyl, cyclopentyl or cyclohexyl, optionally bridged via a $CH_2$ or a $CH_2CH_2$ chain, in each case unsubstituted or mono- or polysubstituted by F, Cl, Br, - CN, $NH_2$, NH-$C_{1-6}$-alkyl, NH-$C_{1-6}$-alkyl-OH, $C_{1-6}$-alkyl, $N(C_{1-6}\text{-alkyl})_2$, $N(C_{1-6}\text{-alkyl-OH})_2$, $NO_2$, SH, S-$C_{1-6}$-alkyl, S-benzyl, O-$C_{1-6}$-alkyl, OH, O-$C_{1-6}$-alkyl-OH, =O, O-benzyl, C(=O)$C_{1-6}$-alkyl, $CO_2H$, $CO_2$-$C_{1-6}$-alkyl or benzyl; $(CHR^a)_qOR^{13}$, q = 1 or 2 and $R^a$ = H or methyl.

**11.** Spirocyclic cyclohexane derivatives according to claim 1, from the group (17) 1,1-[3-(dimethylamino-(4-fluorophenyl) methyl)-pentamethylene]-3,4-dihydro-1H-2,9-diazafluorene

(18) 1,1-[3-(dimethylamino-(3-fluorophenyl)methyl)-pentamethylene]-3,4-dihydro-1H-2,9-diazafluorene
(19) 1,1-[3-(dimethylamino-phenylmethyl)-pentamethylene]-3,4-dihydro-1H-2,9-diazafluorene
(20) 1,1-[3-(dimethylamino-(phenylethyl)-methyl)-pentamethylene]-3,4-dihydro-1H-2,9-diazafluorene citrate
(21) 1,1-[3-(dimethylamino-(thiophen-2-yl)-methyl)-pentamethylene]-3,4-dihydro-1H-2,9-diazafluorene
(22) 1,1-[3-(4-chlorophenyl-(dimethylamino)-methyl)-pentamethylene]-3,4-dihydro-1H-2,9-diazafluorene
(23) cyclohexyl{1,1-[3-(dimethylamino-(4-fluorophenyl)-methyl)-pentamethylene]-3,4-dihydro-1 H-2,9-diazafluoren-2-yl}methanone
(24) phenyl{1,1-[3-(dimethylamino-(4-fluorophenyl)-methyl)-pentamethylene]-3,4-dihydro-1 H-2,9-diazafluoren-2-yl}methanone
(25) 1,1-[3-(dimethylamino-(4-fluorophenyl)-methyl)-pentamethylene]-1,3,4,9-tetrahydro-pyrano[3,4-b]indole
(26) 1,1-[3-(dimethylamino-(3-fluorophenyl)-methyl)-pentamethylene]-1,3,4,9-tetrahydro-pyrano[3,4-b]indole
(27) 1,1-[3-(dimethylamino-(phenyl)-methyl)-pentamethylene]-1,3,4,9-tetrahydropyrano[3,4-b]indole citrate

(28) 1,1-[3-(dimethylamino-(phenylethyl)-methyl)-pentamethylene]-1,3,4,9-tetrahydropyrano[3,4-b]indole
(29) 1,1-[3-(dimethylamino-(thiophen-2-yl)-methyl)-pentamethylene]-1,3,4,9-tetrahydro-pyrano[3,4-b]indole
(30) 1,1-[3-(dimethylamino-(4-chlorophenyl)-methyl)-pentamethylene]-1,3,4,9-tetrahydro-pyrano[3,4-b]indole
(35) 6-fluoro-1,1-[3-(dimethylamino-(4-fluorophenyl)-methyl)-pentamethylene]-1,3,4,9-tetrahydropyrano[3,4-b]indole
(36) 6-fluoro-1,1-[3-(dimethylamino-(3-fluorophenyl)-methyl)-pentamethylene]-1,3,4,9-tetrahydropyrano[3,4-b]indole
(37) 6-fluoro-1,1-[3-(dimethylamino-(phenyl)-methyl)-pentamethylene]-1,3,4,9-tetrahydro-pyrano[3,4-b]indole
(38) 6-fluoro-1,1-[3-(dimethylamino-(4-chlorophenyl)-methyl)-pentamethylene]-1,3,4,9-tetrahydropyrano[3,4-b]indole
(40) 3,6-dimethyl-1,1-[3-(dimethylamino-(4-fluorophenyl)-methyl)-pentamethylene]-1,3,4,9-tetrahydropyrano[3,4-b]indole
(41) 3,6-dimethyl-1,1-[3-(dimethylamino-(3-fluorophenyl)-methyl)-pentamethylene]-1,3,4,9-tetrahydropyrano[3,4-b]indole
(42) 3,6-dimethyl-1,1-[3-(dimethylamino-(phenyl)-methyl)-pentamethylene]-1,3,4,9-tetrahydropyrano[3,4-b]indole citrate
(43) 3,6-dimethyl-1,1-[3-(dimethylamino-(phenylethyl)-methyl)-pentamethylene]-1,3,4,9-tetrahydropyrano[3,4-b]indole
(44) 3,6-dimethyl-1,1-[3-(dimethylamino-(4-chlorophenyl)-methyl)-pentamethylene]-1,3,4,9-tetrahydropyrano[3,4-b]indole
(51) 6-fluoro-3-methyl-1,1-[3-(dimethylamino-(4-fluorophenyl)-methyl)-pentamethylene]-1,3,4,9-tetrahydro-pyrano[3,4-b]indole
(52) 6-fluoro-3-methyl-1,1-[3-(dimethylamino-(3-fluorophenyl)-methyl)-pentamethylene]-1,3,4,9-tetrahydro-pyrano[3,4-b]indole
(53) 6-fluoro-3-methyl-1,1-[3-(dimethylamino-(phenyl)-methyl)-pentamethylene]-1,3,4,9-tetrahydropyrano[3,4-b]indole
(54) 6-fluoro-3-methyl-1,1-[3-(dimethylamino-(phenylethyl)-methyl)-pentamethylene]-1,3,4,9-tetrahydropyrano[3,4-b]indole
(55) 6-fluoro-3-methyl-1,1-[3-(dimethylamino-(4-chlorophenyl)-methyl)-pentamethylene]-1,3,4,9-tetrahydro-pyrano[3,4-b]indole
(56) 1-{1,1-[3-(dimethylamino-phenylmethyl)-pentamethylene]-3,4-dihydro-1H-2,9-diazafluoren-2-yl}-3,3-dimethylbutan-1-one
(57) 2-[(3,4-dimethoxyphenyl)acetyl]-{1,1-[3-(dimethylamino-phenylmethyl)-pentamethylene]-3,4-dihydro-1H-2,9-diazafluorene}
(58) 1-{1,1-[3-(dimethylamino-phenylmethyl)-pentamethylene]-3,4-dihydro-1H-2,9-diazafluoren-2-yl}-2-methyl-3-phenylprop-2-en-1-one
(59) 2-(cyclohexylcarbonyl)-{1,1-[3-(dimethylamino-phenylmethyl)-pentamethylene]-3,4-dihydro-1H-2,9-diazafluorene}
(60) 2-[(4-chlorophenoxy)acetyl]-{1,1-[3-(dimethylamino-phenylmethyl)-pentamethylene]-3,4-dihydro-1H-2,9-diazafluorene}
(61) 1-{1,1-[3-(dimethylamino-phenylmethyl)-pentamethylene]-3,4-dihydro-1H-2,9-diazafluoren-2-yl}-3-(2-chlorophenyl)prop-2-en-1-one
(62) 1-{1,1-[3-(dimethylamino-(4-fluorophenyl)methyl)-pentamethylene]-3,4-dihydro-1H-2,9-diazafluoren-2-yl}-3,3-dimethylbutan-1-one
(63) 2-[(3,4-dimethoxyphenyl)acetyl]-{1,1-[3-(dimethylamino-(4-fluorophenyl)methyl)-pentamethylene]-3,4-dihydro-1H-2,9-diazafluorene}
(64) 1-{1,1-[3-(dimethylamino-(4-fluorophenyl)methyl)-pentamethylene]-3,4-dihydro-1H-2,9-diazafluoren-2-yl}-2-methyl-3-phenylprop-2-en-1-one
(65) 2-[(4-chlorophenoxy)acetyl]-{1,1-[3-(dimethylamino-(4-fluorophenyl)methyl)-pentamethylene]-3,4-dihydro-1H-2,9-diazafluorene}
(66) 1-{1,1-[3-(dimethylamino-(3-fluorophenyl)methyl)-pentamethylene]-3,4-dihydro-1H-2,9-diazafluoren-2-yl}-3,3-dimethylbutan-1-one
(67) 2-[(3,4-dimethoxyphenyl)acetyl]-{1,1-[3-(dimethylamino-(3-fluorophenyl)methyl)-pentamethylene]-3,4-dihydro-1H-2,9-diazafluorene}
(68) 1-{1,1-[3-(dimethylamino-(3-fluorophenyl)methyl)-pentamethylene]-3,4-dihydro-1H-2,9-diazafluoren-2-yl}-2-methyl-3-phenylprop-2-en-1-one
(69) 2-(cyclohexylcarbonyl)-{1,1-[3-(dimethylamino-(3-fluorophenyl)methyl)-pentamethylene]-3,4-dihydro-1H-2,9-diazafluorene}

(70) 2-[(4-chlorophenoxy)acetyl]-{1,1-[3-(dimethylamino-(3-fluorophenyl)methyl)-pentamethylene]-3,4-dihydro-1H-2,9-diazafluorene}
(71) 2-(1-benzothien-2-ylcarbonyl)-{1,1-[3-(dimethylamino-(3-fluorophenyl)methyl)-pentamethylene]-3,4-dihydro-1H-2,9-diazafluorene}
(72) 1-{1,1-[3-(dimethylamino-(3-fluorophenyl)methyl)-pentamethylene]-3,4-dihydro-1H-2,9-diazafluoren-2-yl}-3-(2-chlorophenyl)prop-2-en-1-one
(73) 1-{1,1-[3-(dimethylamino-(4-chlorophenyl)methyl)-pentamethylene]-3,4-dihydro-1H-2,9-diazafluoren-2-yl}-3,3-dimethylbutan-1-one
(74) 2-[(3,4-dimethoxyphenyl)acetyl]-{1,1-[3-(dimethylamino-(4-chlorophenyl)methyl)-pentamethylene]-3,4-dihydro-1H-2,9-diazafluorene}
(75) 1-{1,1-[3-(dimethylamino-(4-chlorophenyl)methyl)-pentamethylene]-3,4-dihydro-1H-2,9-diazafluoren-2-yl}-2-methyl-3-phenylprop-2-en-1-one
(76) 2-(cyclohexylcarbonyl)-{1,1-[3-(dimethylamino-(4-chlorophenyl)methyl)-pentamethylene]-3,4-dihydro-1H-2,9-diazafluorene}
(77) 2-[(4-chlorophenoxy)acetyl]-{1,1-[3-(dimethylamino-(4-chlorophenyl)methyl)-pentamethylene]-3,4-dihydro-1H-2,9-diazafluorene}
(78) 2-(1-benzothien-2-ylcarbonyl)-{1,1-[3-(dimethylamino-(4-chlorophenyl)methyl)-pentamethylene]-3,4-dihydro-1H-2,9-diazafluorene}
(79) 2-acetyl-{1,1-[3-(dimethylamino-phenylmethyl)-pentamethylene]-3,4-dihydro-1H-2,9-diazafluorene}
(80) 1-{1,1-[3-(dimethylamino-phenylmethyl)-pentamethylene]-3,4-dihydro-1H-2,9-diazafluoren-2-yl}-3-phenylprop-2-en-1-one
(81) 2-(methoxyacetyl)-{1,1-[3-(dimethylamino-phenylmethyl)-pentamethylene]-3,4-dihydro-1H-2,9-diazafluorene}
(82) 2-(2-thienylacetyl)-{1,1-[3-(dimethylamino-phenylmethyl)-pentamethylene]-3,4-dihydro-1H-2,9-diazafluorene}
(83) 2-(benzyloxyacetyl)-{1,1-[3-(dimethylamino-phenylmethyl)-pentamethylene]-3,4-dihydro-1H-2,9-diazafluorene}
(84) acetyl-{1,1-[3-(dimethylamino-(4-fluorophenyl)methyl)-pentamethylene]-3,4-dihydro-1 H-2,9-diazafluorene}
(85) 2-(3-cyclopentylpropanoyl)-{1,1-[3-(dimethylamino-(4-fluorophenyl)methyl)-pentamethylene]-3,4-dihydro-1H-2,9-diazafluorene}
(86) 1-{1,1-[3-(dimethylamino-(4-fluorophenyl)methyl)-pentamethylene]-3,4-dihydro-1H-2,9-diazafluoren-2-yl}-3-phenylprop-2-en-1-one
(87) 2-(2-thienylacetyl)-{1,1-[3-(dimethylamino-(4-fluorophenyl)methyl)-pentamethylene]-3,4-dihydro-1H-2,9-diazafluorene}
(88) 2-(benzyloxyacetyl)-{1,1-[3-(dimethylamino-(4-fluorophenyl)methyl)-pentamethylene]-3,4-dihydro-1H-2,9-diazafluorene}
(89) acetyl-{1,1-[3-(dimethylamino-(3-fluorophenyl)methyl)-pentamethylene]-3,4-dihydro-1 H-2,9-diazafluorene}
(90) 2-(3-cyclopentylpropanoyl)-{1,1-[3-(dimethylamino-(3-fluorophenyl)methyl)-pentamethylene]-3,4-dihydro-1H-2,9-diazafluorene}
(91) 2-(cyclopropylcarbonyl)-{1,1-[3-(dimethylamino-(3-fluorophenyl)methyl)-pentamethylene]-3,4-dihydro-1H-2,9-diazafluorene}
(92) 2-(methoxyacetyl)-{1,1-[3-(dimethylamino-(3-fluorophenyl)methyl)-pentamethylene]-3,4-dihydro-1H-2,9-diazafluorene}
(93) 2-(2-thienylacetyl)-{1,1-[3-(dimethylamino-(3-fluorophenyl)methyl)-pentamethylene]-3,4-dihydro-1H-2,9-diazafluorene}
(94) 2-(benzyloxyacetyl)-{1,1-[3-(dimethylamino-(3-fluorophenyl)methyl)-pentamethylene]-3,4-dihydro-1H-2,9-diazafluorene}
(95) 2-(3-cyclopentylpropanoyl)-{1,1-[3-(dimethylamino-(4-chlorophenyl)methyl)-pentamethylene]-3,4-dihydro-1H-2,9-diazafluorene}
(96) 2-(cyclopropylcarbonyl)-{1,1-[3-(dimethylamino-(4-chlorophenyl)methyl)-pentamethylene]-3,4-dihydro-1H-2,9-diazafluorene}
(97) 2-(methoxyacetyl)-{1,1-[3-(dimethylamino-(4-chlorophenyl)methyl)-pentamethylene]-3,4-dihydro-1H-2,9-diazafluorene}
(98) 2-(2-thienylacetyl)-{1,1-[3-(dimethylamino-(4-chlorophenyl)methyl)-pentamethylene]-3,4-dihydro-1H-2,9-diazafluorene}
(99) 2-(benzyloxyacetyl)-{1,1-[3-(dimethylamino-(4-chlorophenyl)methyl)-pentamethylene]-3,4-dihydro-1H-2,9-diazafluorene}
(100) 2-(2-ethylhexanoyl)-{1,1-[3-(dimethylamino-phenylmethyl)-pentamethylene]-3,4-dihydro-1H-2,9-diazafluorene}
(101) 2-isobutyryl-{1,1-[3-(dimethylamino-phenylmethyl)-pentamethylene]-3,4-dihydro-1H-2,9-diazafluorene}
(102) 2-propionyl-{1,1-[3-(dimethylamino-phenylmethyl)-pentamethylene]-3,4-dihydro-1H-2,9-diazafluorene}
(103) 2-(1-benzothien-3-ylcarbonyl)-{1,1-[3-(dimethylamino-phenylmethyl)-pentamethylene]-3,4-dihydro-1H-2,9-diazafluorene}
(104) 2-[(4-fluorophenyl)acetyl]-{1,1-[3-(dimethylamino-phenylmethyl)-pentamethylene]-3,4-dihydro-1H-2,9-diazafluorene}
(105) 2-(3-chloro-1-benzothien-2-ylcarbonyl)-{1,1-[3-(dimethylamino-phenylmethyl)-pentamethylene]-3,4-dihydro-1H-2,9-diazafluorene}
(106) 2-(2-ethylhexanoyl)-{1,1-[3-(dimethylamino-(4-fluorophenyl)methyl)-pentamethylene]-3,4-dihydro-1 H-2,9-diazafluorene}
(107) 2-isobutyryl-{1,1-[3-(dimethylamino-(4-fluorophenyl)methyl)-pentamethylene]-3,4-dihydro-1H-2,9-diazafluorene}
(108) 2-propionyl-{1,1-[3-(dimethylamino-(4-fluorophenyl)methyl)-pentamethylene]-3,4-dihydro-1H-2,9-diazafluorene}
(109) 2-cyclopentylcarbonyl-{1,1-[3-(dimethylamino-(4-fluorophenyl)methyl)-pentamethylene]-3,4-dihydro-1H-2,9-diazafluorene}
(110) 2-[(4-fluorophenyl)acetyl]-{1,1-[3-(dimethylamino-(4-fluorophenyl)methyl)-pentamethylene]-3,4-dihydro-1H-2,9-diazafluorene}
(111) 2-(2-ethylhexanoyl)-{1,1-[3-(dimethylamino-(3-fluorophenyl)methyl)-pentamethylene]-3,4-dihydro-1H-2,9-diazafluorene}
(112) 2-isobutyryl-{1,1-[3-(dimethylamino-(3-fluorophenyl)methyl)-pentamethylene]-3,4-dihydro-1H-2,9-diazafluorene}
(113) 2-propionyl-{1,1-[3-(dimethylamino-(3-fluorophenyl)methyl)-pentamethylene]-3,4-dihydro-1H-2,9-diazafluorene}
(114) 2-(1-benzothien-3-ylcarbonyl)-{1,1-[3-(dimethylamino-(3-fluorophenyl)methyl)-pentamethylene]-3,4-dihydro-1H-2,9-diazafluorene}
(115) 2-cyclopentylcarbonyl-{1,1-[3-(dimethylamino-(3-fluorophenyl)methyl)-pentamethylene]-3,4-dihydro-1H-2,9-diazafluorene}
(116) 2-[(4-fluorophenyl)acetyl]-{1,1-[3-(dimethylamino-(3-fluorophenyl)methyl)-pentamethylene]-3,4-dihydro-1H-2,9-diazafluorene}
(117) 2-(3-chloro-1-benzothien-2-ylcarbonyl)-{1,1-[3-(dimethylamino-(3-fluorophenyl)methyl)-pentamethylene]-3,4-dihydro-1H-2,9-diazafluorene}
(118) 2-(2-ethylhexanoyl)-{1,1-[3-(dimethylamino-(4-chlorophenyl)methyl)-pentamethylene]-3,4-dihydro-1H-2,9-diazafluorene}
(119) 2-isobutyryl-{1,1-[3-(dimethylamino-(4-chlorophenyl)methyl)-pentamethylene]-3,4-dihydro-1H-2,9-diazafluorene}
(120) 2-propionyl-{1,1-[3-(dimethylamino-(4-chlorophenyl)methyl)-pentamethylene]-3,4-dihydro-1H-2,9-diazafluorene}
(121) 2-(1-benzothien-3-ylcarbonyl)-{1,1-[3-(dimethylamino-(4-chlorophenyl)methyl)-pentamethylene]-3,4-dihydro-1H-2,9-diazafluorene}
(122) 2-cyclopentylcarbonyl-{1,1-[3-(dimethylamino-(4-chlorophenyl)methyl)-pentamethylene]-3,4-dihydro-1H-2,9-diazafluorene}
(123) 2-[(4-fluorophenyl)acetyl]-{1,1-[3-(dimethylamino-(4-chlorophenyl)methyl)-pentamethylene]-3,4-dihydro-1H-2,9-diazafluorene}
(124) 2-(3-chloro-1-benzothien-2-ylcarbonyl)-{1,1-[3-(dimethylamino-(4-chlorophenyl)methyl)-pentamethylene]-3,4-dihydro-1H-2,9-diazafluorene}
(125) 2-cyclopentylcarbonyl-{1,1-[3-(dimethylamino-phenylmethyl)-pentamethylene]-3,4-dihydro-1H-2,9-diazafluorene}
(126) 2-[(3-methoxyphenyl)acetyl]-{1,1-[3-(dimethylamino-(3-fluorophenyl)methyl)-pentamethylene]-3,4-dihydro-1H-2,9-diazafluorene}
(127) 2-(2-phenoxypropanoyl)-{1,1-[3-(dimethylamino-(3-fluorophenyl)methyl)-pentamethylene]-3,4-dihydro-1H-2,9-diazafluorene}
(128) 2-[5-methylbenzothien-2-ylcarbonyl]-{1,1-[3-(dimethylamino-(3-fluorophenyl)methyl)-pentamethylene]-3,4-dihydro-1H-2,9-diazafluorene}
(129) 1-{1,1-[3-(dimethylamino-(3-fluorophenyl)methyl)-pentamethylene]-3,4-dihydro-1H-2,9-diazafluoren-2-yl}-3-methylbutan-1-one

(130) 1-{1,1-[3-(dimethylamino-(3-fluorophenyl)methyl)-pentamethylene]-3,4-dihydro-1H-2,9-diazafluoren-2-yl}-3-phenylbutan-1-one

(131) 2-(3-phenylpropanoyl)-{1,1-[3-(dimethylamino-(4-chlorophenyl)methyl)-pentamethylene]-3,4-dihydro-1H-2,9-diazafluorene}

(132) 2-[(3-bromophenyl)acetyl]-{1,1-[3-(dimethylamino-(4-chlorophenyl)methyl)-pentamethylene]-3,4-dihydro-1H-2,9-diazafluorene}

(133) 2-(2-phenoxypropanoyl)-{1,1-[3-(dimethylamino-(4-chlorophenyl)methyl)-pentamethylene]-3,4-dihydro-1H-2,9-diazafluorene}

(134) 1-{1,1-[3-(dimethylamino-(4-chlorophenyl)methyl)-pentamethylene]-3,4-dihydro-1H-2,9-diazafluoren-2-yl}-3-methylbutan-1-one

(135) (3-phenylpropanoyl)-1-{1,1-[3-(dimethylamino-(4-chlorophenyl)methyl)-pentamethylene]-3,4-dihydro-1H-2,9-diazafluorene}

(136) 2-[(3-methoxyphenyl)acetyl]-{1,1-[3-(dimethylamino-phenylmethyl)-pentamethylene]-3,4-dihydro-1H-2,9-diazafluorene}

(137) 2-(2-phenoxypropanoyl)-{1,1-[3-(dimethylamino-phenylmethyl)-pentamethylene]-3,4-dihydro-1H-2,9-diazafluorene}

(138) 1-{1,1-[3-(dimethylamino-phenylmethyl)-pentamethylene]-3,4-dihydro-1H-2,9-diazafluoren-2-yl}-3-methylbutan-1-one

(139) 1-{1,1-[3-(dimethylamino-(4-fluorophenyl)methyl)-pentamethylene]-3,4-dihydro-1H-2,9-diazafluoren-2-yl}-2-ethylbutan-1-one

(140) 2-[(3-methoxyphenyl)acetyl]-{1,1-[3-(dimethylamino-(4-fluorophenyl)methyl)-pentamethylene]-3,4-dihydro-1H-2,9-diazafluorene}

(141) 2-(2-phenoxypropanoyl)-{1,1-[3-(dimethylamino-(4-fluorophenyl)methyl)-pentamethylene]-3,4-dihydro-1H-2,9-diazafluorene}

(142) 1-{1,1-[3-(dimethylamino-(4-fluorophenyl)methyl)-pentamethylene]-3,4-dihydro-1H-2,9-diazafluoren-2-yl}-3-phenylbutan-1-one

in the form of the racemate; of the enantiomers, diastereomers, mixtures of the enantiomers or diastereomers or of an individual enantiomer or diastereomer; of the bases and/or salts of physiologically acceptable acids.

**12.** Process for the preparation of a spirocyclic cyclohexane derivative according to formula I

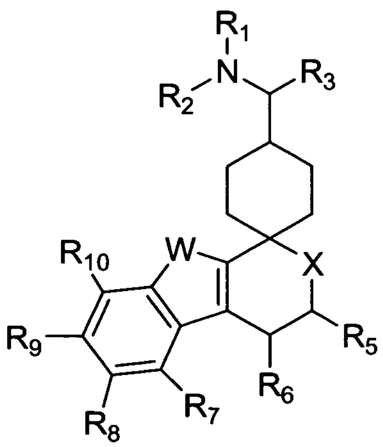

wherein the radicals $R^1$-$R^{10}$ and W and X have the meaning given in claim 1, wherein an educt of the general formula A

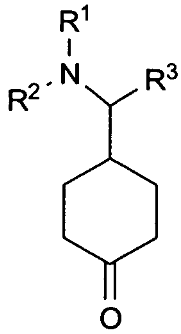

**A**

is reacted, with the addition of an acid or trimethylsilyl ester thereof, for example trifluoromethanesulfonic acid trimethylsilyl ester, trifluoromethanesulfonic acid, acetic acid, phosphoric acid, methanesulfonic acid or trifluoroacetic, in a suitable solvent, for example dichloroethane, methylene chloride, chloroform, acetonitrile, diethyl ether or nitromethane, with an educt of the general formula B

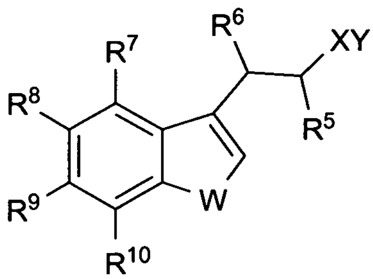

**B**

wherein the radicals $R^1$-$R^{10}$ and W have the meanings given in claim 1 and Y denotes H or trimethylsilyl.

**13.** Process for the preparation of spirocyclic cyclohexane derivatives according to formula I

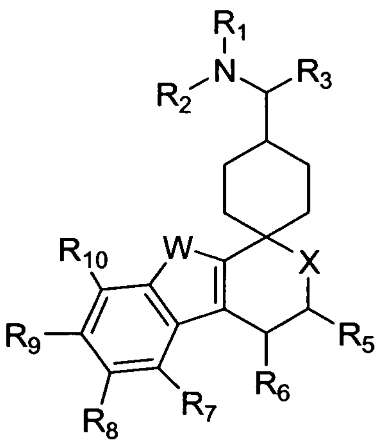

wherein the radicals $R^1$-$R^{10}$ and W have the meaning given in claim 1, X denotes $NR^{17}$ and $R^{17}$ denotes $COR^{12}$ or $SO_2R^{12}$, wherein a spirocyclic cyclohexane derivative in which X denotes NH is reacted, with the addition of base, for example triethylamine, with an anhydride or an acid chloride, preferably under microwave irradiation.

**14.** Medicament containing at least one spirocyclic cyclohexane derivative according to claim 1, optionally in the form of the racemate; of the enantiomers, diastereomers, mixtures of the enantiomers or diastereomers or of an individual enantiomer or diastereomer; of the bases and/or salts of physiologically acceptable acids, and optionally containing suitable additives and/or auxiliary substances and/or optionally further active compounds.

**15.** Use of a spirocyclic cyclohexane derivative according to claim 1, optionally in the form of the racemate; of the enantiomers, diastereomers, mixtures of the enantiomers or diastereomers or of an individual enantiomer or diastereomer; of the bases and/or salts of physiologically acceptable acids, for the preparation of a medicament for treatment of pain, in particular of acute, neuropathic or chronic pain.

**16.** Use of a spirocyclic cyclohexane derivative according to claim 1, optionally in the form of the racemate; of the enantiomers, diastereomers, mixtures of the enantiomers or diastereomers or of an individual enantiomer or diastereomer; of the bases and/or salts of physiologically acceptable acids, for the preparation of a medicament for treatment of depressions, urinary incontinence, diarrhoea, pruritus, alcohol and drug abuse, medicament dependency, lack of drive and/or for anxiolysis.

**Revendications**

**1.** Dérivés spirocycliques du cyclohexane de formule générale I,

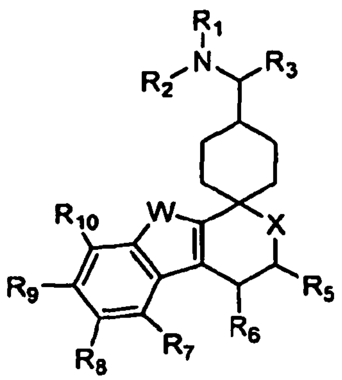

I

où
$R^1$ et $R^2$ signifient, indépendamment l'un de l'autre, H ;
$C_{1-5}$-alkyle, à chaque fois saturé ou insaturé, ramifié ou non ramifié, $R^1$ et $R^2$ ne signifiant pas simultanément H, ou les radicaux $R^1$ et $R^2$ signifient ensemble
$CH_2CH_2OCH_2CH_2$, $CH_2CH_2NR^{11}CH_2CH_2$ ou $(CH_2)_{3-6}$,
où $R^{11}$ signifie H ; $C_{1-5}$-alkyle ou $C_{3-8}$-cycloalkyle, à
chaque fois saturé ou insaturé, ramifié ou non ramifié, monosubstitué, polysubstitué ou non substitué ; aryle ou hétéroaryle, à chaque fois monosubstitué, polysubstitué ou non substitué ; ou aryle ou $C_{3-8}$-cycloalkyle ou hétéroaryle, à chaque fois non substitué, monosubstitué ou polysubstitué, lié par $C_{1-3}$-alkyle
$R^3$ signifie phényle ou
thiényle, à chaque fois non substitué, monosubstitué ou polysubstitué par F, Cl, CN, $NO_2$, SH, S-$C_{1-6}$-alkyle, OH, O-$C_{1-6}$-alkyle, $CO_2H$, $CO_2$-$C_{1-6}$-alkyle, $CF_3$, $C_{1-6}$-alkyle ; un radical phényle lié via une chaîne $C_{1-3}$-alkyle, à chaque fois non substitué, monosubstitué ou polysubstitué par F, Cl, CN, $NO_2$, SH, S-$C_{1-6}$-alkyle, OH, O-$C_{1-6}$-alkyle, $CO_2H$, $CO_2$-$C_{1-6}$-alkyle, $CF_3$, $C_{1-6}$-alkyle ;
W représente $NR^4$ O ou S et
$R^4$ signifie H ; $C_{1-5}$-alkyle, saturé ou insaturé,
ramifié ou non ramifié, non substitué, monosubstitué ou polysubstitué ; aryle ou hétéroaryle, à chaque fois substitué

ou non substitué ; aryle, hétéroaryle ou cycloalkyle, à chaque fois monosubstitué, polysubstitué ou non substitué, lié par un groupe $C_{1-3}$-alkyle ; $COR^{12}$ ; $CSR^{12}$ ; $SO_2R^{12}$,
où $R^{12}$ signifie H ; $C_{1-8}$-alkyle, à chaque fois saturé
ou insaturé, ramifié ou non ramifié, monosubstitué, polysubstitué ou non substitué ; $C_{3-8}$-cycloalkyle, à chaque fois saturé ou insaturé, monosubstitué ou polysubstitué non substitué ; aryle ou hétéroaryle, à chaque fois monosubstitué, polysubstitué ou non substitué ; ou aryle, $C_{3-8}$-cycloalkyle ou hétéroaryle, à chaque fois monosubstitué, polysubstitué ou non substitué, lié par $C_{1-3}$-alkyle ; $(CHR^a)_qOR^{13}$, q = 0, 1 ou 2 et $R^a$ = H, méthyle ou éthyle ; $NR^{14}R^{15}$ ;
$R^5$ représente H ; $C_{1-5}$-alkyle, saturé ou insaturé,
ramifié ou non ramifié, non substitué, monosubstitué ou polysubstitué ; $COOR^{13}$, $CONR^{13}$, $OR^{13}$; $C_{3-8}$-cycloalkyle, saturé ou insaturé, non substitué, monosubstitué ou polysubstitué ; aryle ou hétéroaryle, non substitué, monosubstitué ou polysubstitué ; ou aryle, $C_{3-8}$-cycloalkyle ou hétéroaryle, non substitué, monosubstitué ou polysubstitué, lié par $C_{1-3}$-alkyle ;
$R^6$ représente H ; $OR^{13}$, $COOR^{13}$, $NR^{19}R^{15}$ ; $C_{1-5}$-alkyle,
saturé ou insaturé, ramifié ou non ramifié, non substitué, monosubstitué ou polysubstitué ; $C_{3-8}$-cycloalkyle, saturé ou insaturé, non substitué, monosubstitué ou polysubstitué ; phényle ; ou aryle ou hétéroaryle, lié par $C_{1-3}$-alkyle ;
$R^7$, $R^8$, $R^9$ et $R^{10}$ représentent, indépendamment
l'un de l'autre, H, F, Cl, Br, $NO_2$, $CF_3$, $OR^{13}$, CN, $COOR^{13}$, $NR^{14}R^{15}$; $C_{1-5}$-alkyle, phényle ou benzyle ; où $R^{13}$ signifie H ; $C_{1-5}$-alkyle, à chaque fois saturé ou insaturé, ramifié ou non ramifié, non substitué, monosubstitué ou polysubstitué ; $C_{3-8}$-cycloalkyle, à chaque fois saturé ou insaturé, non substitué, monosubstitué ou polysubstitué ; aryle ou hétéroaryle, non substitué, monosubstitué ou polysubstitué ; ou aryle, $C_{3-8}$-clcloalkyle ou hétéroaryle, non substitué, monosubstitué ou polysubstitué, lié par $C_{1-3}$-alkyle ;
$R^{14}$ et $R^{15}$ signifient indépendamment l'un de l'autre, H
ou $C_{1-5}$-alkyle ou
$R^{14}$ et $R^{15}$ forment, ensemble, $CH_2CH_2OCH_2CH_2$,
$CH_2CH_2NR^{16}CH_2CH_2$ ou $(CH_2)_{3-6}$,
où $R^{16}$ signifie H ; $C_{1-5}$-alkyle, saturé ou insaturé ;
X représente O, S, SO, $SO_2$ ou $NR^{17}$ ;
où $R^{17}$ signifie H ; $C_{1-5}$-alkyle, saturé ou insaturé,
ramifié ou non ramifié, à chaque fois monosubstitué, polysubstitué ou non substitué ; $COR^{12}$ ou $SO_2R^{12}$
sous forme du racémate ; des énantiomères, diastéréo-isomères, mélanges des énantiomères ou diastéréo-isomères ou d'un seul énantiomère ou diastéréo-isomère ; des bases et/ou des sels des acides physiologiquement acceptables.

**2.** Dérivés substitués de cyclohexane selon la revendication 1, où $R^1$ et $R^2$ signifient indépendamment l'un de l'autre H ou $C_{1-6}$-alkyle, où $R^1$ et $R^2$ ne signifient pas simultanément H, ou $R^1$ et $R^2$ signifient ensemble $CH_2CH_2OCH_2CH_2$ ou $(CH_2)_{3-5}$.

**3.** Dérivés substitués de cyclohexane selon la revendication 2, où $R^1$ et $R^2$ signifient $CH_3$.

**4.** Dérivés substitués de cyclohexane selon la revendication 1, où $R^3$ signifie phényle, non substitué ou monosubstitué par Cl ou F, phénéthyle ou thiényle.

**5.** Dérivés substitués de cyclohexane selon la revendication 1, où $R^5$ signifie H ou $CH_3$.

**6.** Dérivés substitués de cyclohexane selon la revendication 1, où $R^6$ signifie H.

**7.** Dérivés substitués de cyclohexane selon la revendication 1, où les radicaux $R^8$ et $R^9$ représentent, indépendamment l'un de l'autre, H ; $C_{1-5}$-alkyle, F, Cl, Br, I, OH, $OCH_3$, COOH, $COOCH_3$, $NH_2$, $NHCH_3$, $N(CH_3)_2$ ou $NO_2$, alors que les radicaux $R^{10}$ et $R^7$ représentent H.

**8.** Dérivés substitués de cyclohexane selon la revendication 1, où $R^8$ représente H, F ou $CH_3$, alors que les radicaux $R^7$, $R^9$ et $R^{10}$ signifient H.

**9.** Dérivés substitués de cyclohexane selon la revendication 1, où $R^{17}$ signifie H ou $COR^{12}$.

**10.** Dérivés substitués de cyclohexane selon la revendication 1, où $R^{12}$ représente phényle, phénéthyle, phénéthényle, 1-méthylphénéthényle, ou benzyle, non substitué, monosubstitué ou polysubstitué par F, Cl, Br, I, CN, $NH_2$, NH-$C_{1-6}$-alkyle, NH-$C_{1-6}$-alkyl-OH, N $(C_{1-6}alkyle)_2$, $N(C_{1-6}\text{-alkyl-OH})_2$, $NO_2$, SH, S-$C_{1-6}$-alkyle, OH, O-$C_{1-6}$-alkyle, O-

$C_{1-6}$alkyl-OH, C(=O)$C_{1-6}$-alkyle, $CO_2H$, $CO_2$-$C_{1-6}$-alkyle, $CF_3$, $C_{1-6}$-alkyle ; thiényle ou benzothiényle, le cas échéant ponté via une chaîne $CH_2$ ou $CH_2CH_2$, non substitué, monosubstitué ou polysubstitué par F, Cl, Br, I, CN, $NH_2$, NH-$C_{1-6}$-alkyle, NH-$C_{1-6}$-alkyl-OH, N($C_{1-6}$ alkyle)$_2$, N($C_{1-6}$-alkyl-OH)$_2$, $NO_2$, SH, S-$C_{1-6}$-alkyle, OH, O-$C_{1-6}$-alkyle, O-$C_{1-6}$alkyl-OH, C(=O)$C_{1-6}$-alkyle, $CO_2H$, $CO_2$-$C_{1-6}$-alkyle, $CF_3$, $C_{1-6}$-alkyle ; ou cyclopropyle, cyclobutyle, cyclopentyle ou cyclohexyle, le cas échéant ponté via une chaîne $CH_2$ ou $CH_2CH_2$, à chaque fois non substitué, monosubstitué ou polysubstitué par F, Cl, Br, -CN, $NH_2$, NH-$C_{1-6}$-alkyle, NH-$C_{1-6}$-alkyl-OH, $C_{1-6}$-alkyle, N($C_{1-6}$-alkyle)$_2$, N($C_{1-6}$-alkyl-OH)2, $NO_2$, SH, S-$C_{1-6}$-alkyle, S-benzyle, O-$C_{1-6}$-alkyle, OH, O-$C_{1-6}$-alkyl-OH, =O, O-benzyle, C(=O)$C_{1-6}$-alkyle, $CO_2H$, $CO_2$-$C_{1-6}$-alkyle ou benzyle ; $(CHR^a)_qOR^{13}$, q = 1 ou 2 et $R^a$= H ou méthyle.

**11.** Dérivés spirocycliques de cyclohexane selon la revendication 1 du groupe

17. 1,1-[3-(diméthylamino-(4-fluorophényl)méthyl)-pentaméthylène]-3,4-dihydro-1H-2,9-diazafluorène
18. 1,1-[3-(diméthylamino-(3-fluorophényl)méthyl)-pentaméthylène]-3,4-dihydro-1H-2,9-diazafluorène
19. 1,1-[3-(diméthylaminophénylméthyl)-pentaméthylène]-3,4-dihydro-1H-2,9-diazafluorène
20. citrate de 1,1-[3-(diméthylamino-(phényléthyl)-méthyl)-pentaméthylène]-3,4-dihydro-1H-2,9-diazafluorène
21. 1,1-[3-(diméthylamino-(thiophén-2-y)-méthyl)-pentaméthylène]-3,4-dihydro-1H-2,9-diazafluorène
22. 1,1-[3-(4-chlorophényl-(diméthylamino)-méthyl)-pentaméthylène]-3,4-dihydro-1H-2,9-diazafluorène
23. cyclohexyl{1,1-[3-(diméthylamino-(4-fluorophényl)-méthyl)-pentaméthylène]-3,4-dihydro-1H-2,9-diazafluorén-2-yl}méthanone
24. phényl{1,1-[3-(diméthylamino-(4-fluorophényl)-méthyl)-pentaméthylène]-3,4-dihydro-1H-2,9-diazafluorén-2-yl)méthanone
25. 1,1-[3-(diméthylamino-(4-fluorophényl)-méthyl)-pentaméthylène]-1,3,4,9-tétrahydropyrano[3,4-b]indole
26. 1,1-[3-(diméthylamino-(3-fluorophényl)-méthyl)-pentaméthylène]-1,3,4,9-tétrahydropyrano[3,4-b]indole
27. citrate de 1,1-[3-(diméthylamino-(phényl)-méthyl)-pentaméthylène]-1,3,4,9-tétrahydropyrano[3,4-b]indole
28. 1,1-[3-(diméthylamino-(phényléthyl)-méthyl)-pentaméthylène]-1,3,4,9-tétrahydropyrano[3,4-b]indole
29. 1,1-[3-(diméthylamino-(thiophén-2-yl)-méthyl)-pentaméthylène]-1,3,4,9-tétrahydropyrano[3,4-b]indole
30. 1,1-[3-(diméthylamino-(4-chlorophényl)-méthyl)-pentaméthylène]-1,3,4,9-tétrahydropyrano[3,4-b]indole
35. 6-fluoro-1,1-[3-(diméthylamino-(4-fluorophényl)-méthyl)-pentaméthylène]-1,3,4,9-tétrahydropyrano[3,4-b]indole
36. 6-fluoro-1,1-[3-(diméthylamino-(3-fluorophényl)-méthyl)-pentaméthylène]-1,3,4,9-tétrahydropyrano[3,4-b]indole
37. 6-fluoro-1,1-[3-(diméthylamino-(phényl)-méthyl)-pentaméthylène]-1,3,4,9-tétrahydropyrano[3,4-b]indole
38. 6-fluoro-1,1-[3-(diméthylamino-(4-chlorophényl)-méthyl)-pentaméthylène]-1,3,4,9-tétrahydropyrano[3,4-b]indole
40. 3,6-diméthyl-1,1-[3-(diméthylamino-(4-fluorophényl)-méthyl)-pentaméthylène]-1,3,4,9-tétrahydropyrano[3,4-b]indole
41. 3,6-diméthyl-1,1-[3-(diméthylamino-(3-fluorophényl)-méthyl)-pentaméthylène]-1,3,4,9-tétrahydropyrano[3,4-b]indole
42. citrate de 3,6-diméthyl-1,1-[3-(diméthylamino-(phényl)-méthyl)-pentaméthylène]-1,3,4,9-tétrahydropyrano[3,4-b]indole
43. 3,6-diméthyl-1,1-[3-(diméthylamino-(phényléthyl)-méthyl)-pentaméthylène]-1,3,4,9-tétrahydropyrano[3,4-b]indole
44. 3,6-diméthyl-1,1-[3-(diméthylamino-(4-chlorophényl)-méthyl)-pentaméthylène]-1,3,4,9-tétrahydropyrano[3,4-b]indole
51. 6-fluoro-3-méthyl-1,1-[3-(diméthylamino-(4-fluorophényl)-méthyl)-pentaméthylène]-1,3,4,9-tétrahydropyrano[3,4-b]indole
52. 6-fluoro-3-méthyl-1,1-[3-(diméthylamino-(3-fluorophényl)-méthyl)-pentaméthylène]-1,3,4,9-tétrahydropyrano[3,4-b]indole
53. 6-fluoro-3-méthyl-1,1-[3-(diméthylamino-(phényl)-méthyl)-pentaméthylène]-1,3,4,9-tétrahydropyrano[3,4-b]indole
54. 6-fluoro-3-méthyl-1,1-[3-(diméthylamino-(phényléthyl)-méthyl)-pentaméthylène]-1,3,4,9-tétrahydropyrano[3,4-b]indole
55. 6-fluoro-3-méthyl-1,1-[3-(diméthylamino-(4-chlorophényl)-méthyl)-pentaméthylène]-1,3,4,9-tétrahydropyrano[3,4-b]indole
56. 1-{1,1-[3-(diméthylaminophénylméthyl)-pentaméthylène]-3,4-dihydro-1H-2,9-diazafluorén-2-yl}-3,3-diméthylbutan-1-one
57. 2-[(3,4-diméthoxyphényl)acétyl]-{1,1-[3-(diméthylaminophénylméthyl)-pentaméthylène]-3,4-dihydro-1H-2,9-diazafluorène}

58. 1-{1,1-[3-(diméthylaminophénylméthyl)-pentaméthylène]-3,4-dihydro-1H-2,9-diazafluorén-2-yl}-2-méthyl-3-phénylprop-2-én-1-one
59. 2-(cyclohexylcarbonyl)-{1,1-[3-(diméthylaminophénylméthyl)-pentaméthylène]-3,4-dihydro-1H-2,9-diazafluorène}
60. 2-[(4-chlorophénoxy)acétyl]-{1,1-[3-(diméthylaminophénylméthyl)-pentaméthylène]-3,4-dihydro-1H-2,9-diazafluorène}
61. 1-{1,1-[3-(diméthylaminophénylméthyl)-pentaméthylène]-3,4-dihydro-1H-2,9-diazafluorén-2-yl}-3-(2-chlorophényl)prop-2-én-1-one
62. 1-{1,1-[3-(diméthylamino-(4-fluorophényl)méthyl)-pentaméthylène]-3,4-dihydro-1H-2,9-diazafluorén-2-yl}-3,3-diméthylbutan-1-one
63. 2-[(3,4-diméthoxyphényl)acétyl]-{1,1-[3-(diméthylamino-(4-fluorophényl)méthyl)-pentaméthylène]-3,4-dihydro-1H-2,9-diazafluorène}
64. 1-{1,1-[3-(diméthylamino-(4-fluorophényl)méthyl)-pentaméthylène]-3,4-dihydro-1H-2,9-diazafluorén-2-yl}-2-méthyl-3-phénylprop-2-én-1-one
65. 2-[(4-chlorophénoxy)acétyl]-(1,1-[3-(diméthylamino-(4-fluorophényl)méthyl)-pentaméthylène]-3,4-dihydro-1H-2,9-diazafluorène}
66. 1-{1,1-[3-(diméthylamino-(3-fluorophényl)méthyl)-pentaméthylène]-3,4-dihydro-1H-2,9-diazafluorén-2-yl}-3,3-diméthylbutan-1-one
67. 2-[(3,4-diméthoxyphényl)acétyl]-{1,1-[3-(diméthylamino-(3-fluorophényl)méthyl)-pentaméthylène]-3,4-dihydro-1H-2,9-diazafluorène}
68. 1-{1,1-[3-(diméthylamino-(3-fluorophényl)méthyl)-pentaméthylène]-3,4-dihydro-1H-2,9-diazafluorén-2-yl}-2-méthyl-3-phénylprop-2-én-1-one
69. 2-(cyclohexylcarbonyl)-{1,1-[3-(diméthylamino-(3-fluorophényl)méthyl)-pentaméthylène]-3,4-dihydro-1H-2,9-diazafluorène}
70. 2-[(4-chlorophénoxy)acétyl]-{1,1-[3-(diméthylamino-(3-fluorophényl)méthyl)-pentaméthylène]-3,4-dihydro-1H-2,9-diazafluorène}
71. 2-(1-benzothién-2-ylcarbonyl)-{1,1-[3-(diméthylamino-(3-fluorophényl)méthyl)-pentaméthylène]-3,4-dihydro-1H-2,9-diazafluorène}
72. 1-{1,1-[3-(diméthylamino-(3-fluorophényl)méthyl)-pentaméthylène]-3,4-dihydro-1H-2,9-diazafluorén-2-yl}-3-(2-chlorophényl)prop-2-én-1-one
73. 1-{1,1-[3-(diméthylamino-(4-chlorophényl)méthyl)-pentaméthylène]-3,4-dihydro-1H-2,9-diazafluorén-2-yl}-3,3-diméthylbutan-1-one
74. 2-[(3,4-diméthoxyphényl)acétyl]-{1,1-[3-(diméthylamino-(4-chlorophényl)méthyl)-pentaméthylène]-3,4-dihydro-1H-2,9-diazafluorène}
75. 1-{1,1-[3-(diméthylamino-(4-chlorophényl)méthyl)-pentaméthylène]-3,4-dihydro-1H-2,9-diazafluorén-2-yl}-2-méthyl-3-phénylprop-2-én-1-one
76. 2-(cyclohexylcarbonyl)-{1,1-[3-(diméthylamino-(4-chlorophényl)méthyl)-pentaméthylène]-3,4-dihydro-1H-2,9-diazafluorène}
77. 2-[(4-chlorophénoxy)acétyl]-{1,1-[3-(diméthylamino-(4-chlorophényl)méthyl)-pentaméthylène]-3,4-dihydro-1H-2,9-diazafluorène}
78. 2-(1-benzothién-2-ylcarbonyl)-{1,1-[3-(diméthylamino-(4-chlorophényl)méthyl)-pentaméthylène]-3,4-dihydro-1H-2,9-diazafluorène}
79. 2-acétyl-{1,1-[3-(diméthylaminophénylméthyl)-pentaméthylène]-3,4-dihydro-1H-2,9-diazafluorène}
80. 1-{1,1-[3-(diméthylaminophénylméthyl)-pentaméthylène]-3,4-dihydro-1H-2,9-diazafluorène-2-yl}-3-phénylprop-2-én-1-one
81. 2-(méthoxyacétyl)-{1,1-[3-(diméthylaminophénylméthyl)-pentaméthylène]-3,4-dihydro-1H-2,9-diazafluorène}
82. 2-(2-thiénylacétyl)-{1,1-[3-(diméthylaminophénylméthyl)-pentaméthylène]-3,4-dihydro-1H-2,9-diazafluorène}
83. 2-(benzyloxyacétyl)-{1,1-[3-(diméthylaminophénylméthyl)-pentaméthylène]-3,4-dihydro-1H-2,9-diazafluorène}
84. acétyl-{1,1-[3-(diméthylamino-(4-fluorophényl)méthyl)-pentaméthylène]-3,4-dihydro-1H-2,9-diazafluorène}
85. 2-(3-cyclopentylpropanoyl)-{1,1-[3-(diméthylamino-(4-fluorophényl)méthyl)-pentaméthylène]-3,4-dihydro-1H-2,9-diazafluorène}
86. 1-{1,1-[3-(diméthylamino-(4-fluorophényl)méthyl)-pentaméthylène]-3,4-dihydro-1H-2,9-diazafluorén-2-yl}-3-phénylprop-2-én-1-one
87. 2-(2-thiénylacétyl)-{1,1-[3-(diméthylamino-(4-fluorophényl)méthyl)-pentaméthylène]-3,4-dihydro-1H-2,9-diazafluorène}

88. 2-(benzyloxyacétyl)-{1,1-[3-(diméthylamino-(4-fluorophényl)méthyl)-pentaméthylène]-3,4-dihydro-1H-2,9-diazafluorène}
89. acétyl-{1,1-[3-(diméthylamino-(3-fluorophényl)méthyl)-pentaméthylène]-3,4-dihydro-1H-2,9-diazafluorène}
90. 2-(3-cyclopentylpropanoyl)-{1,1-[3-(diméthylamino-(3-fluorophényl)méthyl)-pentaméthylène]-3,4-dihydro-1H-2,9-diazafluorène}
91. 2-(cyclopropylcarbonyl)-{1,1-[3-(diméthylamino-(3-fluorophényl)méthyl)-pentaméthylène]-3,4-dihydro-1H-2,9-diazafluorène}
92. 2-(méthoxyacétyl)-{1,1-[3-(diméthylamino-(3-fluorophényl)méthyl)-pentaméthylène]-3,4-dihydro-1H-2,9-diazafluorène}
93. 2-(2-thiénylacétyl)-{1,1-[3-(diméthylamino-(3-fluorophényl)méthyl)-pentaméthylène]-3,4-dihydro-1H-2,9-diazafluorène}
94. 2-(benzyloxyacétyl)-{1,1-[3-(diméthylamino-(3-fluorophényl)méthyl)-pentaméthylène]-3,4-dihydro-1H-2,9-diazafluorène}
95. 2-(3-cyclopentylpropanoyl)-{1,1-[3-(diméthylamino-(4-chlorophényl)méthyl)-pentaméthylène]-3,4-dihydro-1H-2,9-diazafluorène}
96. 2-(cyclopropylcarbonyl)-{1,1-[3-(diméthylamino-(4-chlorophényl)méthyl)-pentaméthylène]-3,4-dihydro-1H-2,9-diazafluorène}
97. 2-(méthoxyacétyl)-{1,1-[3-(diméthylamino-(4-chlorophényl)méthyl)-pentaméthylène]-3,4-dihydro-1H-2,9-diazafluorène}
98. 2-(2-thiénylacétyl)-{1,1-[3-(diméthylamino-(4-chlorophényl)méthyl)-pentaméthylène]-3,4-dihydro-1H-2,9-diazafluorène}
99. 2-(benzyloxyacétyl)-{1,1-[3-(diméthylamino-(4-chlorophényl)méthyl)-pentaméthylène]-3,4-dihydro-1H-2,9-diazafluorène}
100. 2-(2-éthylhexanoyl)-{1,1-[3-(diméthylaminophénylméthyl)-pentaméthylène]-3,4-dihydro-1H-2,9-diazafluorène}
101. 2-isobutyryl-{1,1-[3-(diméthylaminophénylméthyl)-pentaméthylène]-3,4-dihydro-1H-2,9-diazafluorène}
102. 2-propionyl-{1,1-[3-(diméthylaminophénylméthyl)-pentaméthylène]-3,4-dihydro-1H-2,9-diazafluorène}
103. 2-(1-benzothién-3-ylcarbonyl)-{1,1-[3-(diméthylaminophénylméthyl)-pentaméthylène]-3,4-dihydro-1H-2,9-diazafluorène}
104. 2-[(4-fluorophényl)acétyl]-{1,1-[3-(diméthylaminophénylméthyl)-pentaméthylène]-3,4-dihydro-1H-2,9-diazafluorène}
105. 2-(3-chloro-1-benzothién-2-ylcarbonyl)-{1,1-[3-(diméthylaminophénylméthyl)-pentaméthylène]-3,4-dihydro-1H-2,9-diazafluorène}
106. 2-(2-éthylhexanoyl)-{1,1-[3-(diméthylamino-(4-fluorophényl)méthyl)-pentaméthylène]-3,4-dihydro-1H-2,9-diazafluorène}
107. 2-isobutyryl-{1,1-[3-(diméthylamino-(4-fluorophényl)méthyl)-pentaméthylène]-3,4-dihydro-1H-2,9-diazafluorène}
108. 2-propionyl-{1,1-[3-(diméthylamino-(4-fluorophényl)méthyl)-pentaméthylène]-3,4-dihydro-1H-2,9-diazafluorène}
109. 2-cyclopentylcarbonyl-{1,1-[3-(diméthylamino-(4-fluorophényl)méthyl)-pentaméthylène]-3,4-dihydro-1H-2,9-diazafluorène}
110. 2-[(4-fluorophényl)acétyl)-{1,1-[3-(diméthylamino-(4-fluorophényl)méthyl)-pentaméthylène]-3,4-dihydro-1H-2,9-diazafluorène}
111. 2-(2-éthylhexanoyl)-{1,1-[3-(diméthylamino-(3-fluorophényl)méthyl)-pentaméthylène]-3,4-dihydro-1H-2,9-diazafluorène}
112. 2-isobutyryl-{1,1-[3-(diméthylamino-(3-fluorophényl)méthyl)-pentaméthylène]-3,4-dihydro-1H-2,9-diazafluorène}
113. 2-propionyl-{1,1-[3-(diméthylamino-(3-fluorophényl)méthyl)-pentaméthylène]-3,4-dihydro-1H-2,9-diazafluorène}
114. 2-(1-benzothién-3-ylcarbonyl)-{1,1-[3-(diméthylamino-(3-fluorophényl)méthyl)-pentaméthylène]-3,4-dihydro-1H-2,9-diazafluorène}
115. 2-cyclopentylcarbonyl-{1,1-[3-(diméthylamino-(3-fluorophényl)méthyl)-pentaméthylène]-3,4-dihydro-1H-2,9-diazafluorène}
116. 2-[(4-fluorophényl)acétyl]-{1,1-[3-(diméthylamino-(3-fluorophényl)méthyl)-pentaméthylène]-3,4-dihydro-1H-2,9-diazafluorène}
117. 2-(3-chloro-1-benzothién-2-ylcarbonyl)-{1,1-[3-(diméthylamino-(3-fluorophényl)méthyl)-pentaméthylène]-3,4-dihydro-1H-2,9-diazafluorène}
118. 2-(2-éthylhexanoyl)-{1,1-[3-(diméthylamino-(4-chlorophényl)méthyl)-pentaméthylène]-3,4-dihydro-1H-2,9-diazafluorène}
119. 2-isobutyryl-{1,1-[3-(diméthylamino-(4-chlorophényl)méthyl)-pentaméthylène]-3,4-dihydro-1H-2,9-diazafluorène}
120. 2-propionyl-{1,1-[3-(diméthylamino-(4-chlorophényl)méthyl)-pentaméthylène]-3,4-dihydro-1H-2,9-diazafluorène}
121. 2-(1-benzothién-3-ylcarbonyl)-{1,1-[3-(diméthylamino-(4-chlorophényl)méthyl)-pentaméthylène]-3,4-dihydro-1H-2,9-diazafluorène}
122. 2-cyclopentylcarbonyl-{1,1-[3-(diméthylamino-(4-chlorophényl)méthyl)-pentaméthylène]-3,4-dihydro-1H-2,9-diazafluorène}
123. 2-[(4-fluorophényl)acétyl)-{1,1-[3-(diméthylamino-(4-chlorophényl)méthyl)-pentaméthylène]-3,4-dihydro-1H-2,9-diazafluorène}
124. 2-(3-chloro-1-benzothién-2-ylcarbonyl)-{1,1-[3-(diméthylamino-(4-chlorophényl)méthyl)-pentaméthylène]-3,4-dihydro-1H-2,9-diazafluorène}
125. 2-cyclopentylcarbonyl-{1,1-[3-(diméthylaminophénylméthyl)-pentaméthylène]-3,4-dihydro-1H-2,9-diazafluorène}
126. 2-[(3-méthoxyphényl)acétyl]-{1,1-[3-(diméthylamino-(3-fluorophényl)méthyl)-pentaméthylène]-3,4-dihydro-1H-2,9-diazafluorène}
127. 2-(2-phénoxypropanoyl)-{1,1-[3-(diméthylamino-(3-fluorophényl)méthyl)-pentaméthylène]-3,4-dihydro-1H-2,9-diazafluorène}
128. 2-[5-méthylbenzothién-2-ylcarbonyl]-(1,1-[3-(diméthylamino-(3-fluorophényl)méthyl)-pentaméthylène]-3,4-dihydro-1H-2,9-diazafluorène}
129. 1-{1,1-[3-(diméthylamino-(3-fluorophényl)méthyl)-pentaméthylène]-3,4-dihydro-1H-2,9-diazafluorén-2-yl}-3-méthylbutan-1-one
130. 1-{1,1-[3-(diméthylamino-(3-fluorophényl)méthyl)-pentaméthylène]-3,4-dihydro-1H-2,9-diazafluorén-2-yl}-3-phénylpropan-1-one
131. 2-(3-phénylpropanoyl)-{1,1-[3-(diméthylamino-(4-chlorophényl)méthyl)-pentaméthylène]-3,4-dihydro-1H-2,9-diazafluorène}
132. 2-[(3-bromophényl)acétyl]-{1,1-[3-(diméthylamino-(4-chlorophényl)méthyl)-pentaméthylène]-3,4-dihydro-1H-2,9-diazafluorène}
133. 2-(2-phénoxypropanoyl)-{1,1-[3-(diméthylamino-(4-chlorophényl)méthyl)-pentaméthylène]-3,4-dihydro-1H-2,9-diazafluorène}
134. 1-{1,1-[3-(diméthylamino-(4-chlorophényl)méthyl)-pentaméthylène]-3,4-dihydro-1H-2,9-diazafluorén-2-yl}-3-méthylbutan-1-one
135. (3-phénylpropanoyl)-1-{1,1-[3-(diméthylamino-(4-chlorophényl)méthyl)-pentaméthylène]-3,4-dihydro-1H-2,9-diazafluorène}
136. 2-[(3-méthoxyphényl)acétyl]-{1,1-[3-(diméthylaminophénylméthyl)-pentaméthylène]-3,4-dihydro-1H-2,9-diazafluorène}
137. 2-(2-phénoxypropanoyl)-{1,1-[3-(diméthylaminophénylméthyl)-pentaméthylène]-3,4-dihydro-1H-2,9-diazafluorène}
138. 1-{1,1-[3-(diméthylaminophénylméthyl)-pentaméthylène]-3,4-dihydro-1H-2,9-diazafluorén-2-yl}-3-méthylbutan-1-one
139. 1-{1,1-[3-(diméthylamino-(4-fluorophényl)méthyl)-pentaméthylène]-3,4-dihydro-1H-2,9-diazafluorén-2-yl}-2-éthylbutan-1-one
140. 2-[(3-méthoxyphényl)acétyl]-(1,1-[3-(diméthylamino-(4-fluorophényl)méthyl)-pentaméthylène]-3,4-dihydro-1H-2,9-diazafluorène}
141. 2-(2-phénoxypropanoyl)-{1,1-[3-(diméthylamino-(4-fluorophényl)méthyl)-pentaméthylène]-3,4-dihydro-1H-2,9-diazafluorène}
142. 1-{1,1-[3-(diméthylamino-(4-fluorophényl)méthyl)-pentaméthylène]-3,4-dihydro-1H-2,9-diazafluorén-2-yl}-3-phénylpropan-1-one

sous forme du racémate ; des énantiomères, diastéréo-isomères, mélanges des énantiomères ou diastéréo-isomères ou d'un seul énantiomère ou diastéréo-isomère ; des bases et/ou des sels des acides physiologiquement acceptables.

**12.** Procédé pour la préparation d'un dérivé spirocyclique du cyclohexane selon la formule I

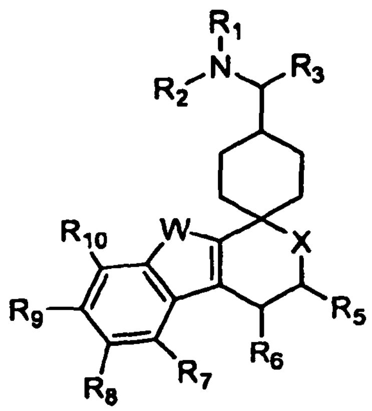

où les radicaux $R^1$-$R^{10}$ ainsi que W et X ont la signification indiquée dans la revendication 1, où on transforme un produit de départ de formule générale A

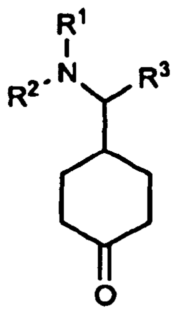

**A**

avec addition d'un acide ou son ester triméthylsilylique, par exemple l'ester triméthylsilylique de l'acide trifluorométhanesulfonique, l'acide trifluorométhanesulfonique, l'acide acétique, l'acide phosphorique, l'acide méthanesulfonique ou l'acide trifluoroacétique, dans un solvant approprié, par exemple le dichloroéthane, le dichlorométhane, le chloroforme, l'acétonitrile, le diéthyléther ou le nitrométhane, avec un produit d'addition de formule générale B

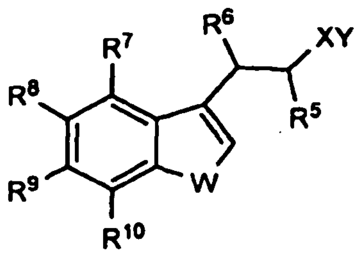

**B**

les radicaux $R^1$-$R^{10}$ ainsi que W ayant les significations indiquées dans la revendication 1 et Y représentant H ou triméthylsilyle.

**13.** Procédé pour la préparation de dérivés spirocycliques du cyclohexane selon la formule I

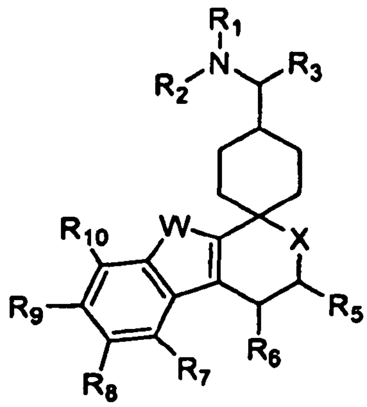

où les radicaux $R^1$-$R^{10}$ ainsi que W présentent la signification indiquée dans la revendication 1, X signifie $NR^{17}$ et $R^{17}$ signifie $COR^{12}$ ou $SO_2R^{12}$, où un dérivé spirocyclique du cyclohexane, dans lequel X signifie NH, est transformé, avec addition d'une base, par exemple de triéthylamine, avec un anhydride ou un chlorure d'acide, de préférence sous irradiation par des micro-ondes.

**14.** Médicament contenant au moins un dérivé spirocyclique du cyclohexane selon la revendication 1, le cas échéant sous forme du racémate ; des énantiomères, diastéréo-isomères, mélanges des énantiomères ou diastéréo-isomères ou d'un seul énantiomère ou diastéréo-isomère ; des bases et/ou des sels des acides physiologiquement acceptables, et contenant le cas échéant des additifs et/ou des adjuvants appropriés et/ou le cas échéant d'autres substances actives.

**15.** Utilisation d'un dérivé spirocyclique du cyclohexane selon la revendication 1, le cas échéant sous forme du racémate ; des énantiomères, diastéréo-isomères, mélanges des énantiomères ou diastéréo-isomères ou d'un seul énantiomère ou diastéréo-isomère ; des bases et/ou des sels des acides physiologiquement acceptables pour la préparation d'un médicament pour le traitement de la douleur, en particulier de la douleur aiguë, neuropathique ou chronique.

**16.** Utilisation d'un dérivé spirocyclique du cyclohexane selon la revendication 1, le cas échéant sous forme du racémate ; des énantiomères, diastéréo-isomères, mélanges des énantiomères ou diastéréo-isomères ou d'un seul énantiomère ou diastéréo-isomère ; des bases et/ou des sels des acides physiologiquement acceptables pour la préparation d'un médicament pour le traitement de dépressions, de l'incontinence urinaire, de la diarrhée, du prurit, de l'abus d'alcool et de drogues, de la dépendance aux médicaments, du manque d'entrain et/ou pour l'anxiolyse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 04043967 A
- WO 2005063769 A
- WO 2005066183 A
- WO 2006018184 A


### In der Beschreibung aufgeführte Nicht-Patentliteratur


- **Jirkovsky et al.** *J. Heterocycl. Chem.,* 1975, vol. 12, 937-940
- **Campaigne et al.** *J. Heterocycl. Chem.,* 1965, vol. 2, 231-235
- **Efange et al.** *J. Med. Chem.,* 1998, vol. 41, 4486-4491
- **Ellingboe et al.** *J. Med. Chem.,* 1992, vol. 35, 1176-1183
- **Pearson et al.** *Aust. J. Chem.,* 1991, vol. 44, 907-917
- **Yokohama et al.** *Chem. Pharm. Bull.,* 1992, vol. 40, 2391-2398
- **Beck et al.** *J. Chem. Soc. Perkin,* 1992, vol. 1, 813-822
- **Shinada et al.** *Tetrahedron Lett.,* 1996, vol. 39, 7099-7102
- **Garden et al.** *Tetrahedron,* 2002, vol. 58, 8399-8412
- Analgesics - from Chemistry and Pharmacology to Clinical Application. Wiley, 2002, 265-284
- **T. V. RajanBabu ; B. L. Chendard ; M. A. Petti.** *J. Org. Chem.,* 1986, vol. 51, 1704-1712
- **S. J. Garden ; R. B. da Silva ; A. C. Pinto.** *Tetrahedron,* 2002, vol. 58, 8399-8412
- **G. K. Jnaneshwara ; V. H. Deshpande.** *Synthetic Commun.,* 1999, vol. 29 (20), 3627-3633
- **E.G. Gray ; V.P. Whittaker.** *J. Anat.,* 1962, vol. 76, 79-88
- **M.Ch. Frink ; H.-H. Hennies ; W. Englberger ; M. Haurand ; B. Wilffert.** *Arzneim.-Forsch./Drug Res.,* 1996, vol. 11 (III), 1029-1036
- **I.C. Hendershot ; J. Forsaith.** *J. Pharmacol. Exp. Ther.,* 1959, vol. 125, 237-240